Europäisches Patentamt

(19) European Patent Office

Office européen des brevets

(11) Publication number : 0 215 126 B1

## (12) EUROPEAN PATENT SPECIFICATION

(45) Date of publication of patent specification :
31.07.91 Bulletin 91/31

(51) Int. Cl.⁵ : **C12N 15/27, C07K 13/00,**
**A61K 37/02**

(21) Application number : 86901138.7

(22) Date of filing : 07.02.86

(86) International application number :
PCT/JP86/00052

(87) International publication number :
WO 86/04605 14.08.86 Gazette 86/18

### (54) HUMAN GRANULOCYTE COLONY STIMULATING FACTOR.

(30) Priority : 08.02.85 JP 23777/85
17.09.85 JP 206066/85
20.09.85 JP 209638/85
02.12.85 JP 269455/85
02.12.85 JP 269456/85
03.12.85 JP 270838/85
03.12.85 JP 270839/85

(43) Date of publication of application :
25.03.87 Bulletin 87/13

(45) Publication of the grant of the patent :
31.07.91 Bulletin 91/31

(84) Designated Contracting States :
AT BE CH DE FR GB IT LI NL SE

(56) References cited :
EP-A- 0 169 566
WO-A-87/01132
JP-A- 5 978 122
JP-A-54 140 789
JP-A-57 114 525
NATURE, vol. 319, no. 6052, January/February 1986,
pages 415-418, London, GB; S. NAGATA et al.:
"Molecular cloning and expression of cDNA for
human granulocyte colony-stimulating factor"
Lymhokine Research, vol. 3, no. 4, 1984, Abstract no.
281
J. Cell Physiol., vol. 110, 1982, pp. 43-49
Proc.Natl.Acad.Sci. USA, Vol 78, n 11, November
1981, pp. 6613-6617

(73) Proprietor : Chugai Seiyaku Kabushiki Kaisha
5-1, 5-chome, Ukima Kita-ku
Tokyo (JP)

(72) Inventor : ONO, Masayoshi Chugai Seiyaku
Kabushiki Kaisha
41-8, Takada 3-chome
Toshima-ku Tokyo 171 (JP)
Inventor : NOMURA, Hitoshi Chugai Seiyaku
Kabushiki Kaisha
41-8, Takada 3-chome Toshima-ku
Tokyo 171 (JP)
Inventor : TAMURA, Masahiko Chugai Seiyaku
Kabushiki Kaisha
41-8, Takada 3-chome
Toshima-ku Tokyo 171 (JP)
Inventor : MATSUMOTO, Masahiko
Chugai Seiyaku Kabushiki Kaisha 41-8Takada
3chome
Toshima-ku Tokyo 171 (JP)
Inventor : YAMAZAKI, Tatsumi
Chugai Seiyaku Kabushiki Kaisha 41-8Takada
3chome
Toshima-ku Tokyo 171 (JP)
Inventor : YAMAMOTO, Osami
Chugai Seiyaku Kabushiki Kaisha 41-8Takada
3chome
Toshima-ku Tokyo 171 (JP)
Inventor : HIRATA, Yuichi
Chugai Seiyaku Kabushiki Kaisha 41-8Takada
3chome
Toshima-ku Tokyo 171 (JP)
Inventor : SEKIMORI, Yasuo
Chugai Seiyaku Kabushiki Kaisha 41-8Takada
3chome
Toshima-ku Tokyo 171 (JP)
Inventor : TSUCHIYA,Masayuki Chugai Seiyaku
Kabushiki Kaisha
41-8, Takada 3-chome Toshima-ku
Tokyo 171 (JP)
Inventor : NAGATA, Shigekazu
24-62-3-305, Tamagawa 2-chome
Ohta-ku Tokyo 146 (JP)

(74) Representative : Vossius & Partner
Siebertstrasse 4 P.O. Box 86 07 67
W-8000 München 86 (DE)

## Description

The present invention relates to a human granulocyte colony stimulating factor. More particularly, the present invention relates to a gene coding for a polypeptide having the activity of a colony stimulating factor (hereinafter abbreviated as CSF) which is a specific stimulating factor necessary for the principal purpose of forming colonies of human granulocytic cells. The present invention also relates to a recombinant vector inserted said gene, a transformant containing said vector, a polypeptide or glycoprotein having the CSF activity as produced from said transformant, and an infection protective agent which contains CSF as an effective ingredient.

When bone marrow cells as target cells and kidney cells or fetal cells were cultured by the double-layer soft agar cultivation method, with the bone marrow cells being in the upper layer and the kidney or fetal cells in the lower layer, part of the cells in the upper layer grew and differentiated to form colonies of neutrophilic granulocytes (hereunder simply referred to as granulocytes) or monocytic macrophages. This observation has led to the assumption of the presence in vivo of factors which promote the formation of colonies [Pluznik and Sach, J. Cell. Comp. Physiol., 66, 319 (1965) ; and Bradley and Metcalf, Aust. J. Exp. Biol. Med. Sci., 44, 287 (1966)].

These factors which are collectively referred to as CSF are known to be produced by cells, such as T-cells, monocytic macrophages, fibroblasts and endothelial cells, which normally are distributed extensively in vivo. Among subclasses of CSF are included : granulocyte-monocytic macrophage CSF (abbreviated as GM-CSF) which act on the stem cells of granulocytes or monocyte macrophages in such a manner that they stimulate the growth of such stem cells and induce their differentiation to form colonies of granulocytes or monocytic macrophages ; monocytic macrophage CSF (abbreviated as M-CSF) which is principally capable of forming colonies of macrocytic macrophages ; multipotent CSF (abbreviated as multi-CSF) which acts on less differentiated multipotent stem cells ; and granulocyte CSF (abbreviated as G-CSF) of the type contemplated by the present invention which is principally capable of forming granulocytic colonies. It has recently been held that the stages of differentiation of target cells differ from one subclass of CSF to another [Asano, Taisha - Metabolism and Disease, 22, 249 (1985) ; and Yunis et al., "Growth and Maturation Factors", edited by Guroff, John Wiley & Sons, NY, vol. 1, 209 (1983)].

Many reports have been published on human CSF, in particular, CSF derived from normal human tissues and CSF derived from human tumor cells [see, for example, Stanley et al., Fed. Proc., 35, 2272 (1975) ; Burgess et al., Blood, 49, 573 (1977) ; Shah et al., Blood, 50, 811 (1977) ; Fojo et al., Biochem., 17, 3109 (1978) ; Okabe et al., Cancer Res., 38, 3910 (1978) ; Asano et al., Blood, 49, 845 (1977) ; Golde et al., Blood, 57, 1321 (1981); Wu et al., J. Biol. Chem., 254, 6226 (1979) ; and Dipersio et al., Blood, 56, 717 (1980)].

However, these species of human CSF are not in a completely pure form and much is left unknown about the utility or effectiveness of human CSF as a pharmaceutical agent.

The recent advances in chemotherapeutics in the field of infectious diseases have been so remarkable that it has become possible to combat the known strong toxin producing microorganisms having specific pathogenicity. On the other hand, in the wake of advances in medicine and the growing population of aged people, the number of compromised hosts with reduced biophylactic capability is increasing and the opportunistic infection with pathogenic microorganisms that have weak pathogenicity but which are tolerant of drugs and disinfectants is causing a new problem in clinical fields. Opportunistic infectious diseases are caused by highly drug-resistant bacteria or fungi which cannot be combatted by many useful antibiotics and such diseases are desirably treated by application of the combination of conventional chemotherapeutics and drugs that will potentiate the biophylactic ability of the host. Unfortunately, no such potentiating drugs have yet been found.

Hosts have several biophylactic capabilities and, in the early periods of infections, the phagocytic and germkilling actions of leukocytes would be one of the most influencing factors, so it is suggested that drugs would prove effective in the treatment of infections if they were capable of enhancing the host's ability to protect itself against infections by promoting the growth and differentiation for maturation of neutrophiles or macrophages.

Active efforts have been made in order to achieve large-scale preparation of pure human CSF but no success has been reported. Under these circumstances, the present inventors established a cell strain, CHU-1, having an extremely high human G-CSF producing ability and good growth capability from tumor cells of patients with oral cavity cancer (this strain was deposited with C.N.C.M. under Accession Number I-315) and first succeeded in isolating, from the supernatant of a culture of this cell strain, a pure human CSF that was capable of promoting the formation of colonies of human neutrophiles (Japanese Patent Application No. 153273/1984 = EP-A-0169566).

The present inventors checked the protective effect of this human G-CSF against microbial infection using infection animal models, and found that the human G-CSF was effective as a therapeutic against infectious diseases since it had the ability to induce pronounced maturation of neutrophiles in vivo and, hence, exhibited the capability of protecting the hosts from infection.

Subsequently, the present inventors established a cell strain, CHU-2, which was also derived from human oral cavity cancer (this strain was deposited with C.N.C.M. under Accession Number I-483) and successfully isolated from the supernatant of a culture of this cell strain a pure form of G-CSF which was completely identical to the one derived from CHU-1.

However, homogeneous G-CSF has yet to be produced in large quantities by the method of cell cultivation wherein G-CSF is isolated from the supernatant of a culture of either cell strain because G-CSF can only be produced in low concentration and complex purification procedures are required to obtain a trace amount of G-CSF from a large volume of culture solution. Therefore, it has been strongly desired to achieve mass production of G-CSF by recombinant DNA technology.

The International Patent Application WO-A-87/01132 published on 26.02.87 and having the earliest priority date of 23.08.85 deals with the production of human pluripotent granulocyte colony-stimulating factor by recombinant DNA technology.

One object of the present invention is to provide a gene encoding a polypeptide having the human G-CSF activity.

Another object of the present invention is to provide a recombinant vector having inserted said gene.

Still another object of the present invention is to provide a transformant which has been produced by transforming a host with said recombinant vector, and a polypeptide or glycoprotein which is produced by said transformant.

A further object of the present invention is to provide an infection protective agent which contains human G-CSF as an effective ingredient.

Brief Description of the Drawings :

Fig. 1 shows the amino acid sequences and the nucleotide sequences of three different probes, IWQ, A and LC ;

Fig. 2 shows the nucleotide sequence of a pHCS-1 insert ;

Fig. 3(A) shows the nucleotide sequence of a cDNA insert in pBRG4 ;

Fig. 3(B) (I) shows the amino acid sequence of a human G-CSF precursor as deduced from pBRG4 cDNA;

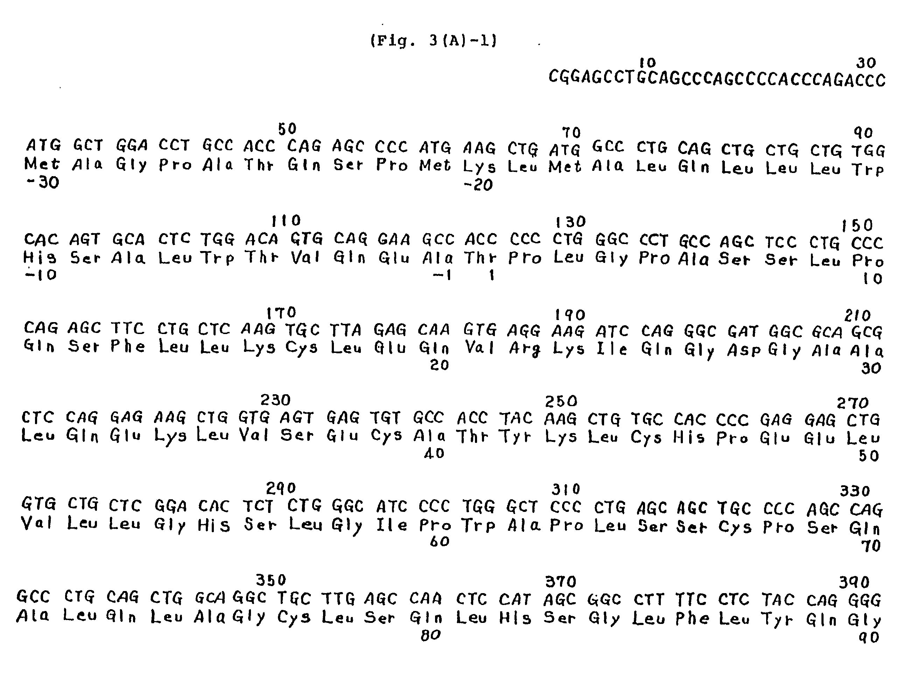

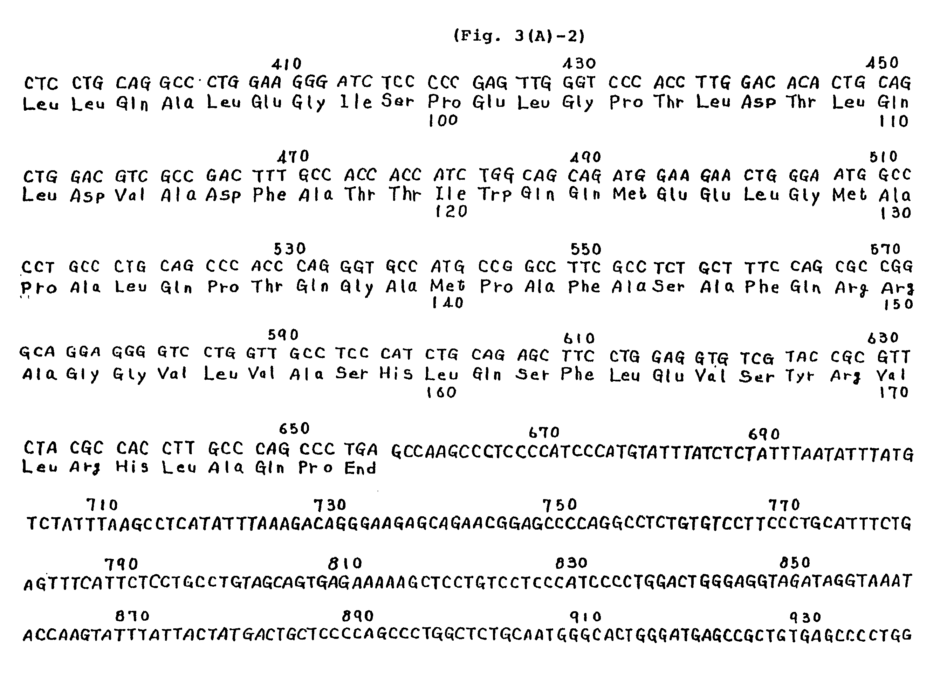

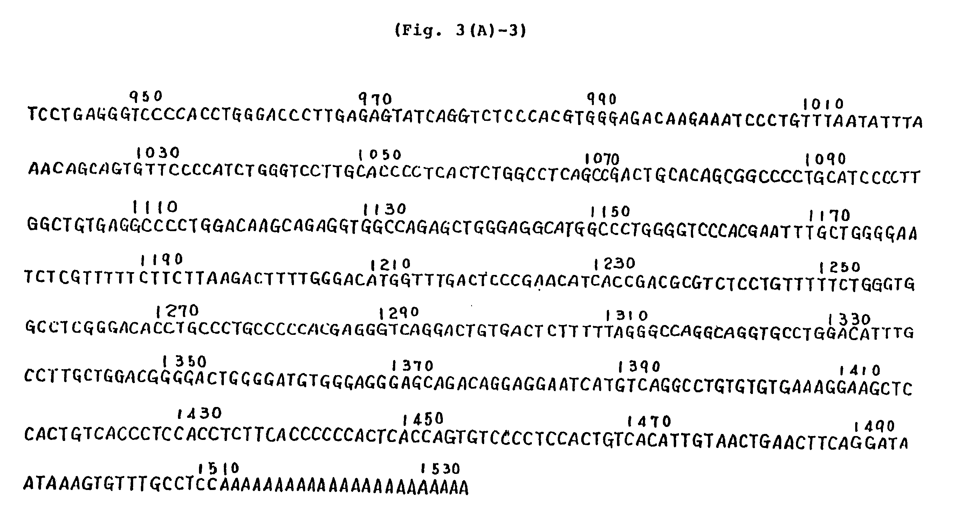

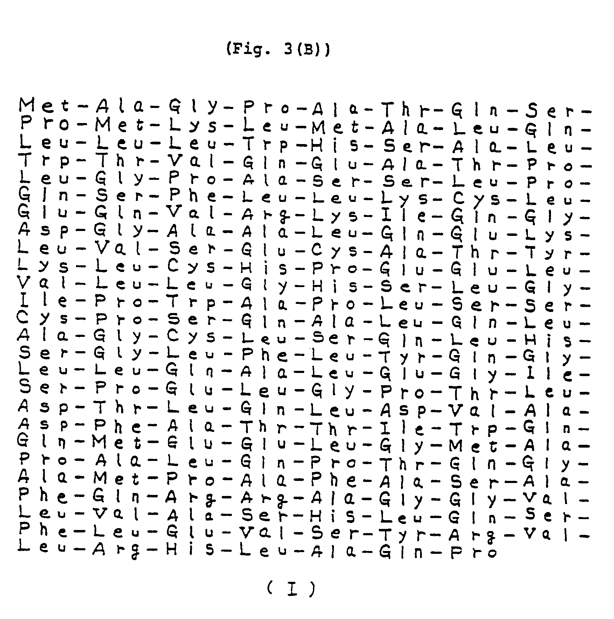

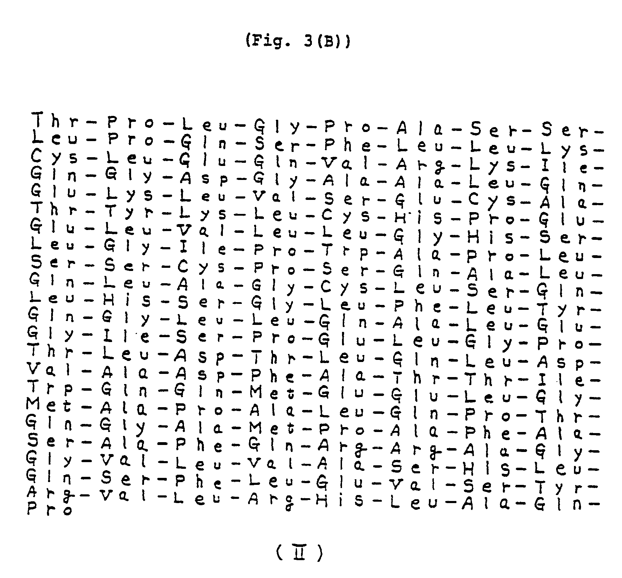

Fig. 3(B) (II) shows the amino acid sequence of human mature G-CSF as deduced from pBRG4 cDNA ;

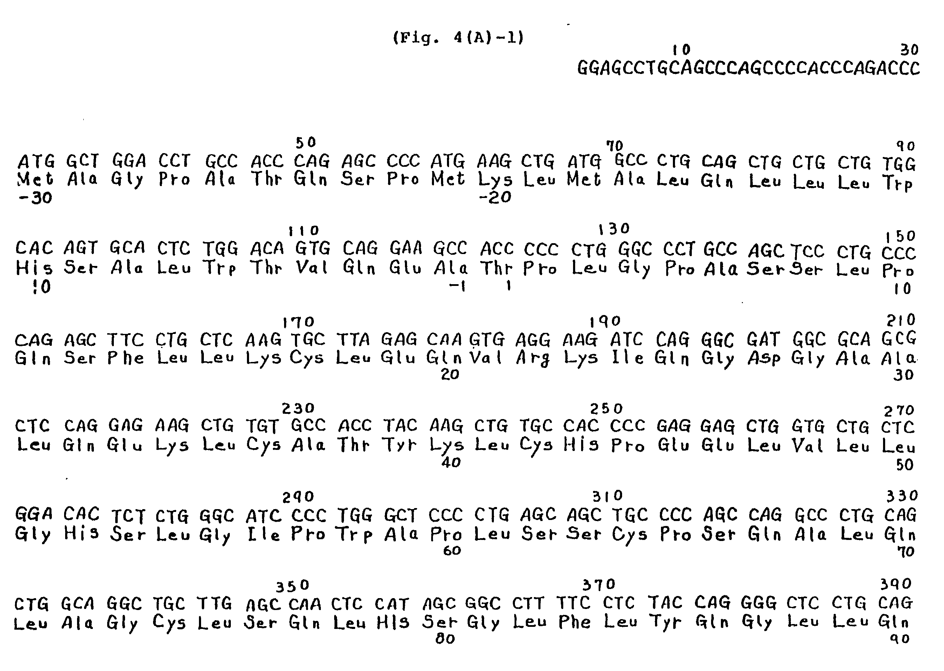

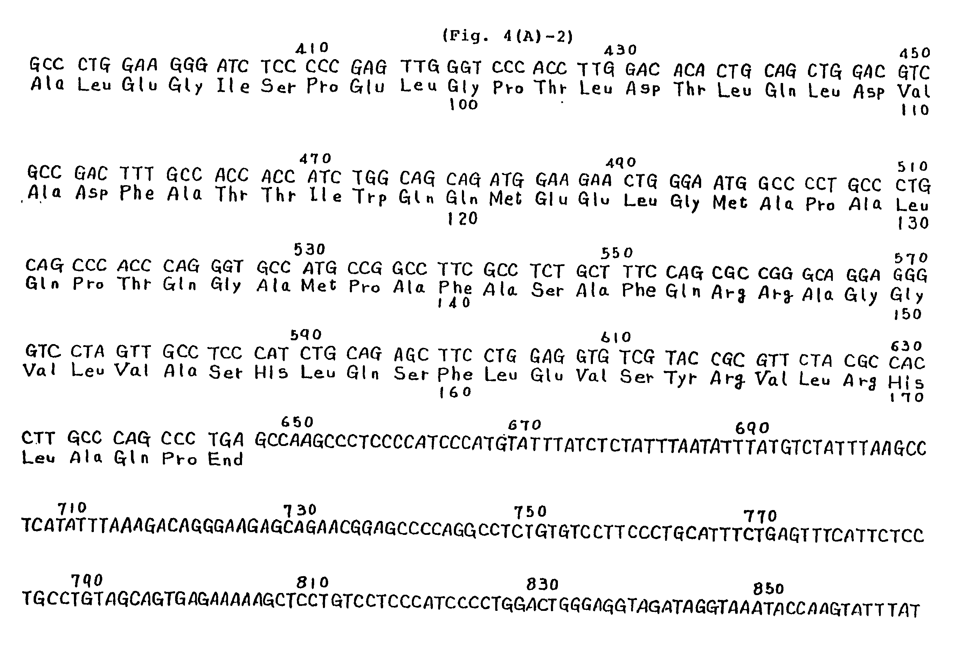

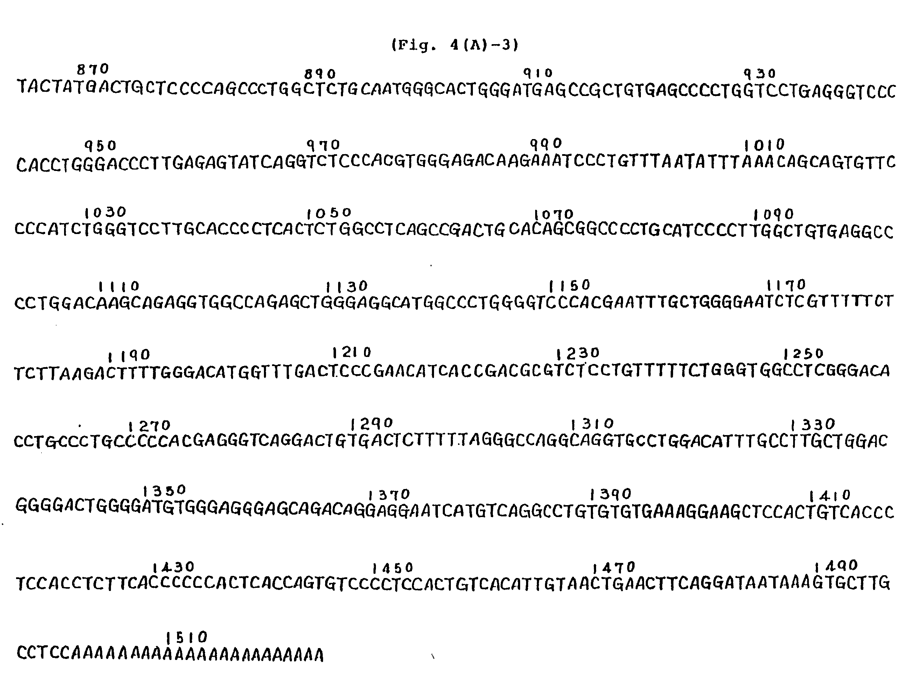

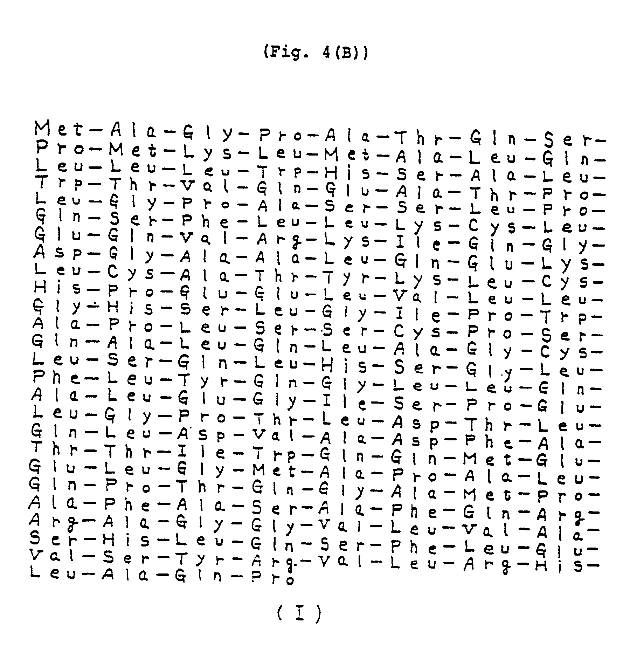

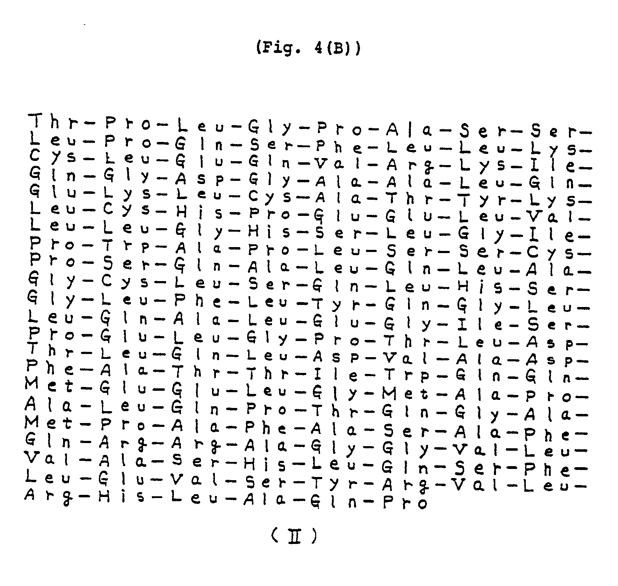

Fig. 4(A) shows the nucleotide sequence of a cDNA insert in pBRV2 ;

Fig. 4(B) (I) shows the amino acid sequence of a human G-CSF precursor as deduced from pBRV2 cDNA;

Fig. 4(B) (II) shows the amino acid sequence of human mature G-CSF as deduced from pBRV2 cDNA ;

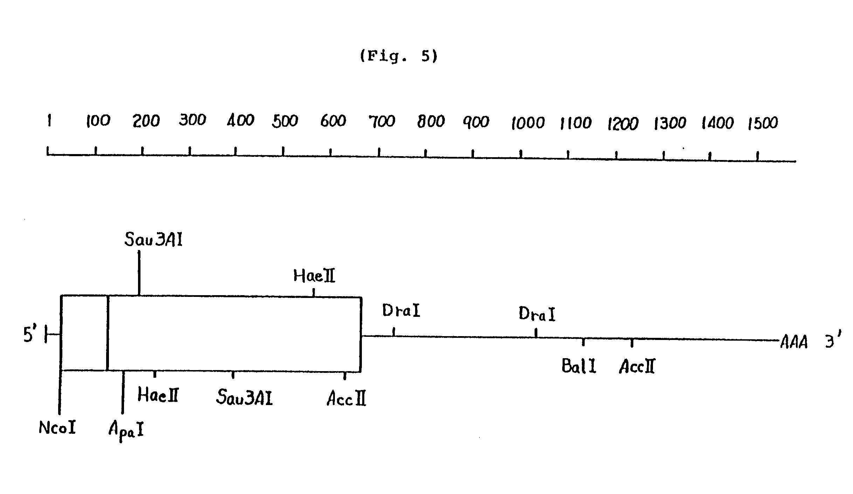

Fig. 5 shows the restriction enzyme cleavage sites of pBRG4- or pBRV2-derived human G-CSF cDNA ;

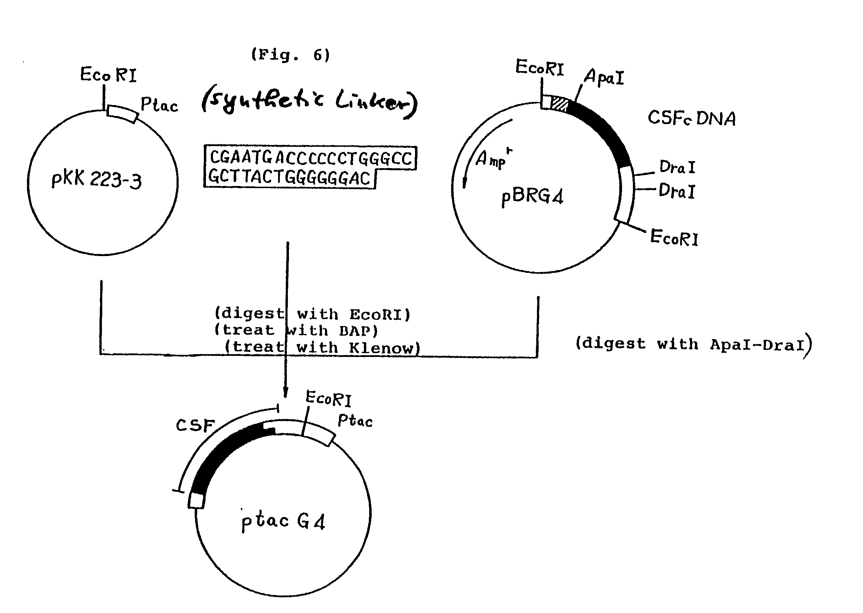

Fig. 6 is a partial presentation of the process for preparing a tac promoter-containing vector (+VSE line) ;

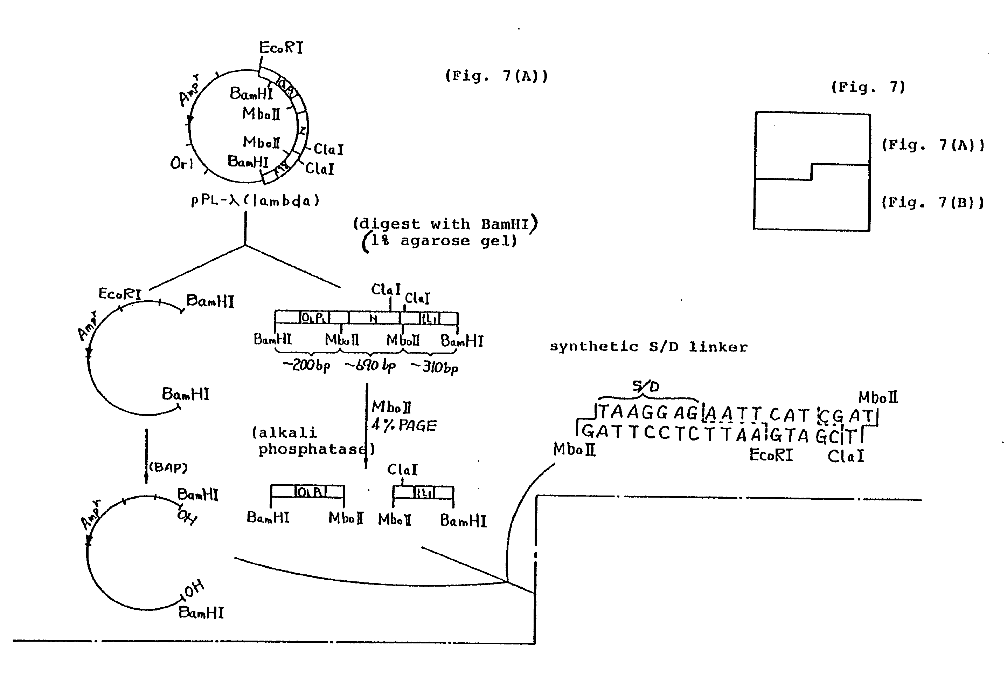

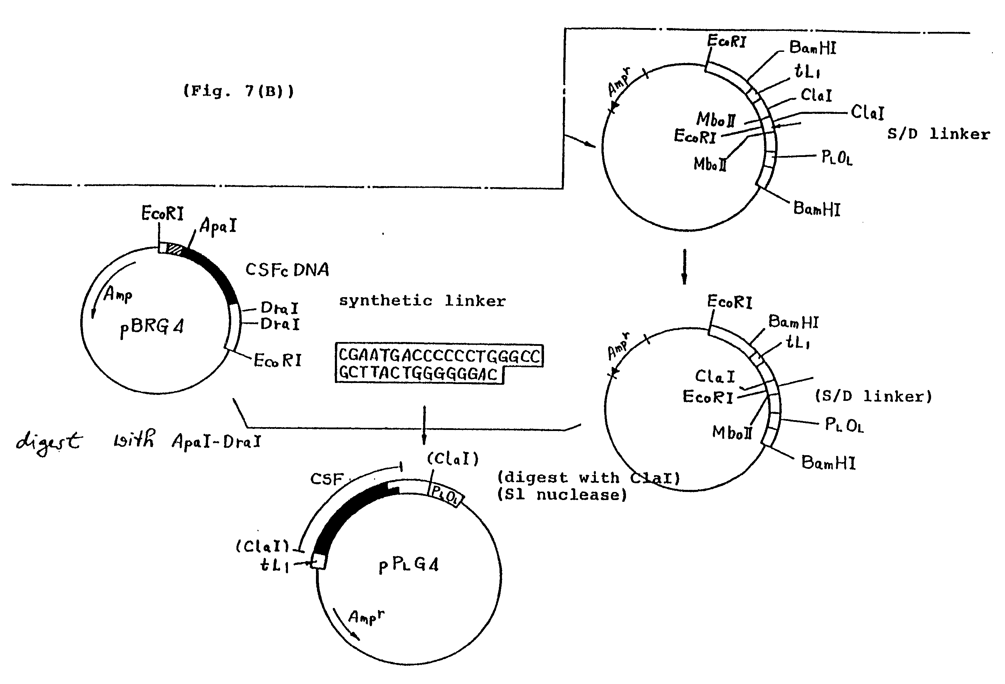

Fig. 7 is a presentation of the process for preparing a $P_L$ promoter-containing vector (+VSE line) ;

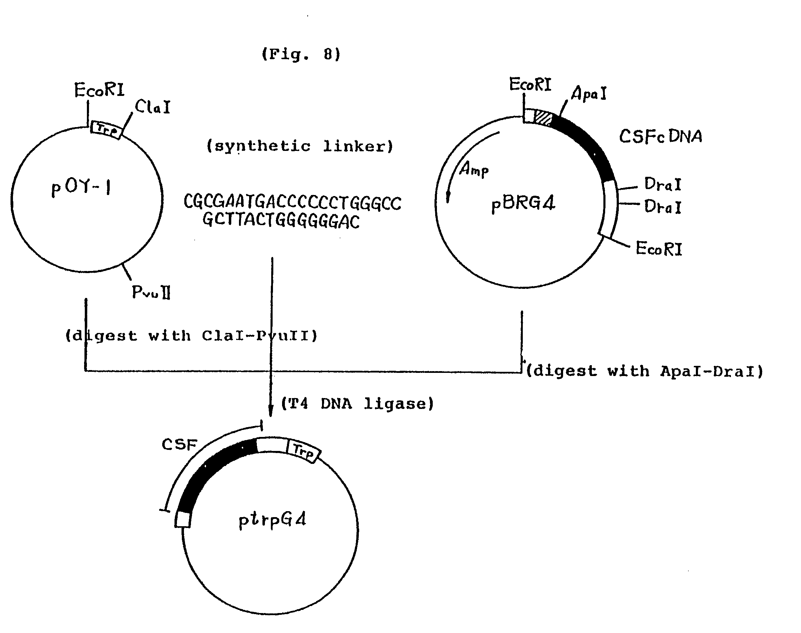

Fig. 8 is a presentation of the process for preparing a trp promoter-containing vector (+VSE line) ;

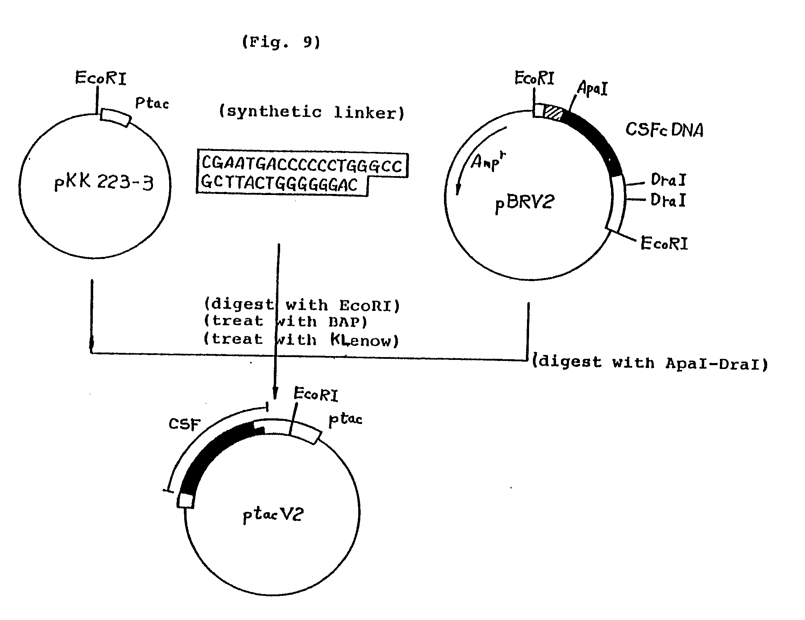

Fig. 9 is a partial presentation of the process for preparing a tac promoter-containing vector (-VSE line) ;

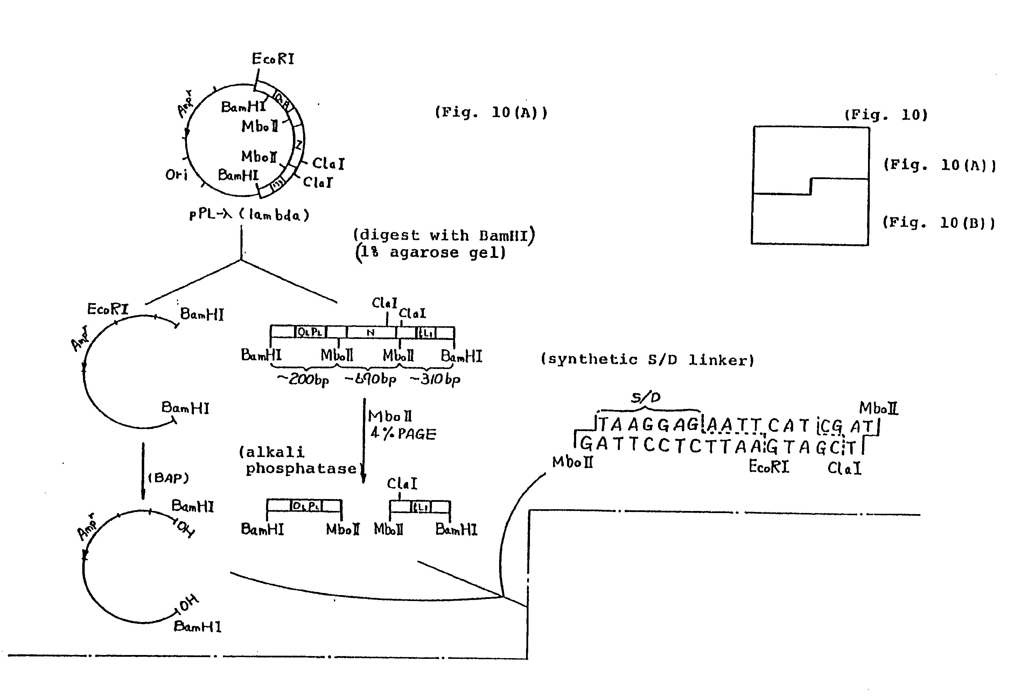

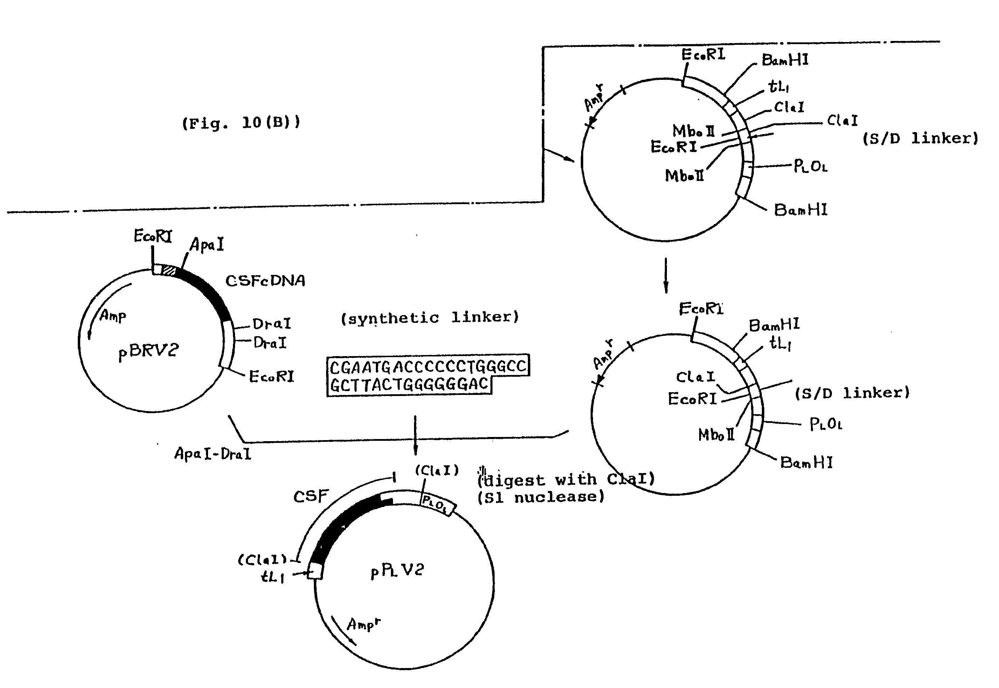

Fig. 10 is a presentation of the process for preparing a $P_L$ promoter-containing vector (-VSE line) ;

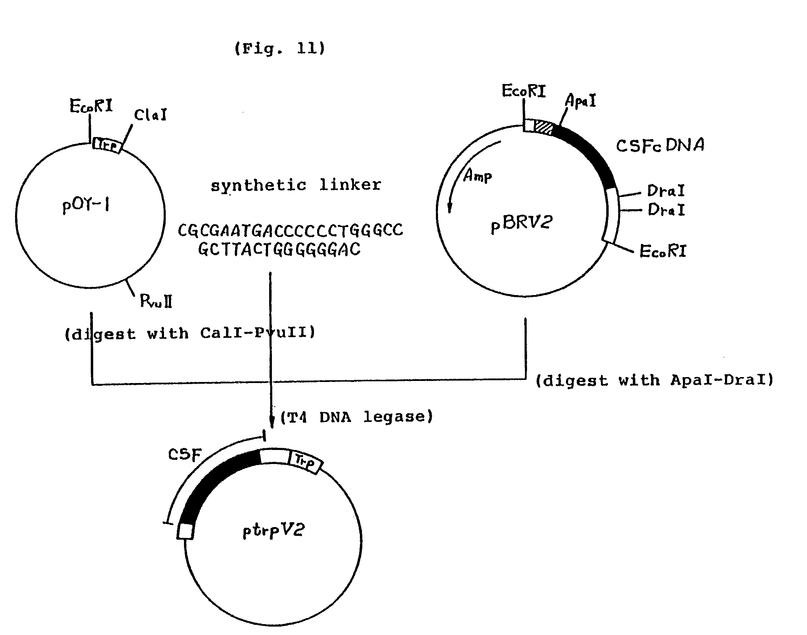

Fig. 11 is a presentation of the process for preparing a trp promoter-containing vector (-VSE line) ;

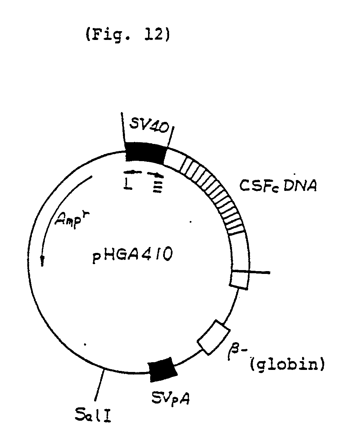

Fig. 12 shows schematically the structure of pHGA410 ;

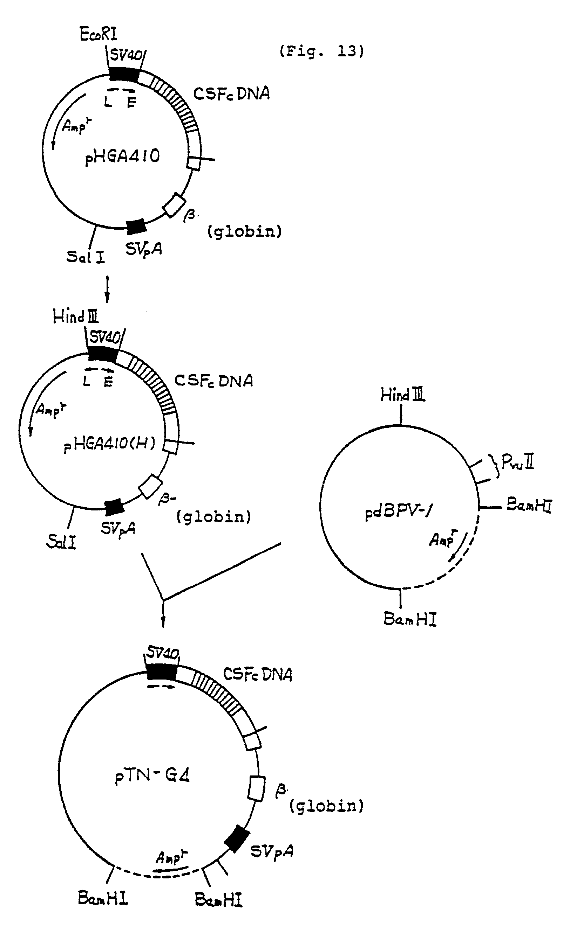

Fig. 13 is a presentation of the process for constructing an expression recombinant vector pTN-G4 ;

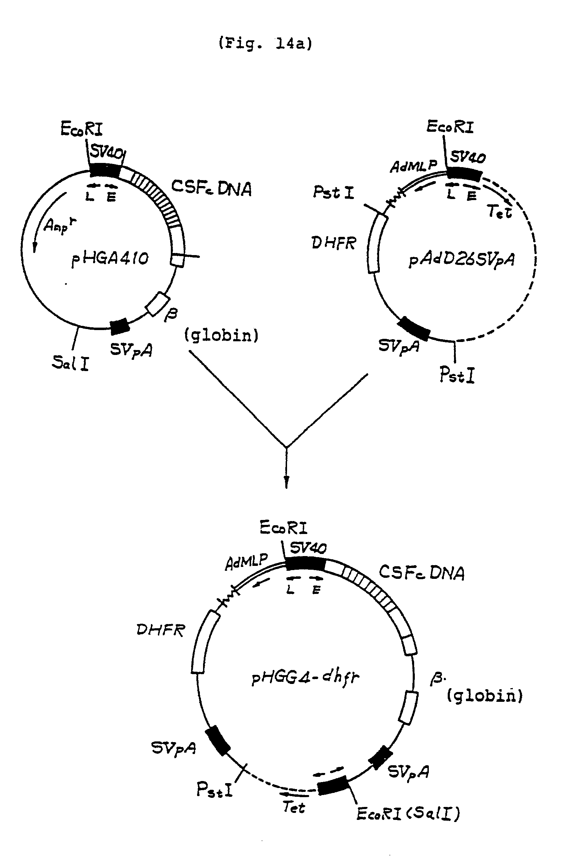

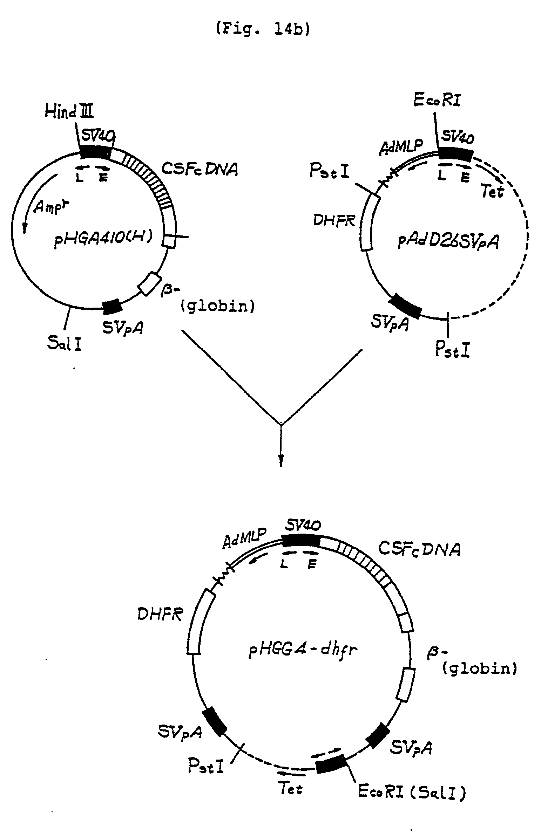

Figs. 14a and 14b show two processes for constructing pHGG4-dhfr ;

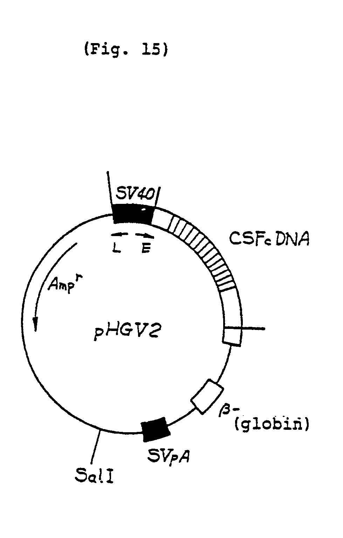

Fig. 15 shows schematically the structure of pHGV2 ;

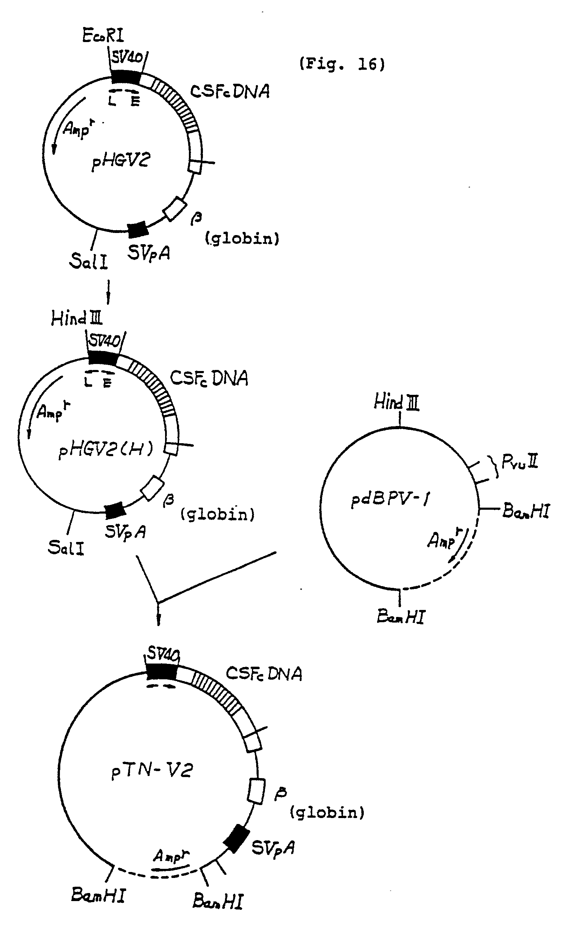

Fig. 16 is a presentation of the process for constructing an expression recombinant vector pTN-V2 ; and

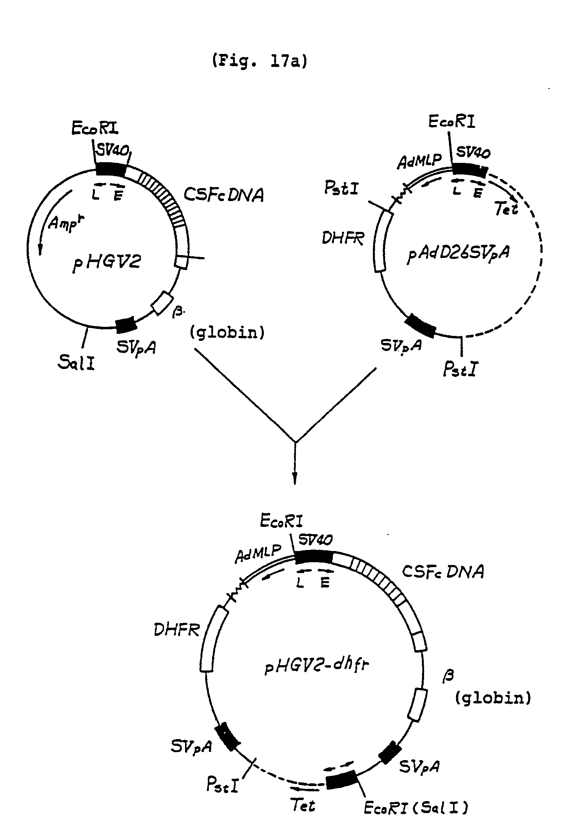

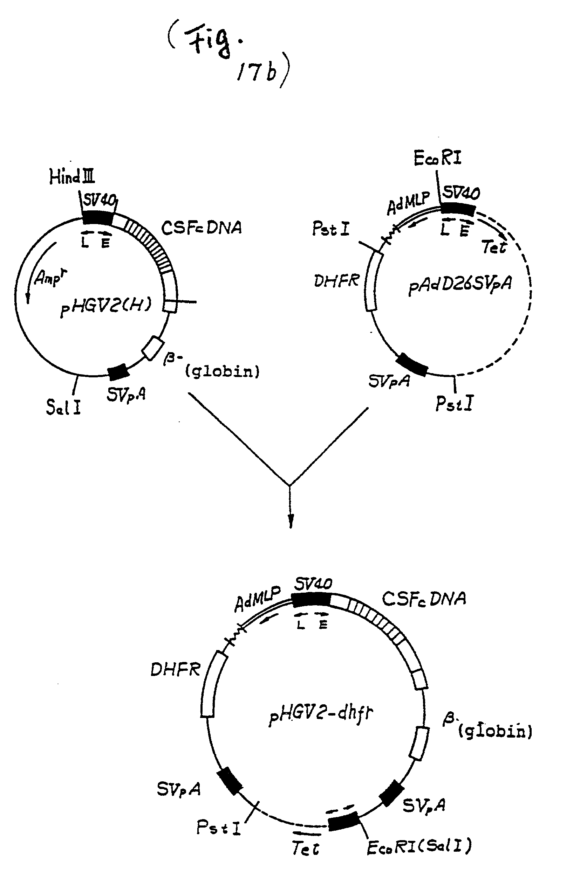

Figs. 17a and 17b show two processes for constructing an expression recombinant vector pHGV2-dhfr.

The gene coding for a polypeptide having the human G-CSF activity according to the present invention is a DNA (cDNA) which is complementary to the messenger RNA (mRNA) that is obtained as 15 - 17S factions by sucrose density gradient centrifugation and which codes for a polypeptide having the human G-CSF activity.

The present inventors obtained two lines of this cDNA.

The cDNA of one line has all or part of a gene coding for the polypeptide I or II shown in Fig. 3(B). More specifically, this cDNA has the nucleotide sequence delineated by ATG at 32 - 34 nucleotide positions from 5'-terminus [see Fig. 3(A)] and CCC at 650 - 652 nucleotide positions, or by ACC at 122 - 124 positions and CCC at 650 - 652 positions. Alternatively, the cDNA has the nucleotide sequence shown in Fig. 3(A) or a part thereof. The cDNA of this line is hereinafter referred to as cDNA (+VSE).

The cDNA of the other line has all or part of a gene coding for the polypeptide I or II shown in Fig. 4(B). More specifically, this cDNA has the nucleotide sequence delineated by ATG at 31 - 33 nucleotide positions

3

from 5'-terminus [see Fig. 4(A)] and CCC at 640 - 642 nucleotide positions, or by ACC at 121 - 123 positions and CCC at 640 - 642 positions. Alternatively, this cDNA may have the nucleotide sequence shown in Fig. 4(A) or a part thereof. The cDNA of this line is hereinafter referred to as cDNA (-VSE).

The gene of the present invention may be obtained by first preparing a mRNA coded G-CSF from mammalian animal cells or other host cells having the ability to produce a polypeptide having the G-CSF activity, then converting said mRNA to a double-stranded cDNA by any of the known methods.

The mammalian cell which may be used as a source of mRNA supply is a human oral cavity cancer-derived cell strain, CHU-2 (deposited at Collection Nationale de Cultures de Microorganismes, or C.N.C.M., under Accession Number I-483). It should however be understood that in place of such tumor cell strains, cells that can be separated from mammals or any other appropriate established cell strains may be employed. Preparation of mRNA may be achieved by one of the methods that have already been proposed for cloning the genes of several other physiologically active proteins : for example, the whole RNA is first obtained by treatments with a surfactant and phenol in the presence of a ribonuclease inhibitor such as a vanadyl-ribonucleoside complex [see Berger and Birkenmeier, Biochemistry, 18, 5143 (1979)] or by CsCl density gradient centrifugation following treatment with guanidine thiocyanate [see Chirgwin et al., Biochemistry, 18, 5294 (1979)], then poly(A⁺) RNA (mRNA) is obtained by subjecting the whole RNA to batch adsorption or affinity column chromatography on oligo(dT)-cellulose or poly-U-Sepharose with Sepharose 2B used as a carrier. The poly(A⁺) RNA may be further fractionated by an appropriate method such as sucrose density gradient centrifugation. The ability of thus obtained mRNA to code for a polypeptide having the G-CSF activity may be confirmed by several methods ; for example, the mRNA is translated into a protein and its physiological activities are checked ; alternatively, the identity of that protein is determined with the aid of an anti-G-CSF antibody. More specifically, mRNA is injected into oocytes of Xenopus laevis for effecting translation [see Gurdon et al., Nature, 233, 177 (1972)], or translational reactions may be performed with rabbit reticulocytes or wheat germs [Schleif and Wensink, "Practical Methods in Molecular Biology", Springer-Verlag, NY (1981)]. The G-CSF activity may be assayed by applying the soft agar cultivation method using bone marrow cells, and techniques for performing this method have been reviewed (Metcalf, "Hemopoietic Colonies", Springer-Verlag, Berlin, Heidelberg, NY (1977)].

A single-stranded cDNA is synthesized with the so obtained mRNA being used as a template ; a double-stranded cDNA is synthesized from this single-stranded cDNA ; and the double-stranded cDNA is inserted into an appropriate vector DNA to form a recombinant plasmid. This recombinant plasmid may be used to transform a suitable host, say Escherichia coli, so as to obtain a group of DNAs in the transformants (hereunder referred to as a cDNA library).

A double-stranded cDNA may be obtained from the mRNA by one of the following two methods : the mRNA is treated with a reverse transcriptase with oligo(dT) which is complementary to the poly(A)-chain at 3'-terminus being used as a primer ; or an oligonucleotide that corresponds to part of the amino acid sequence of G-CSF protein is synthesized, and a cDNA which is complementary to the mRNA is synthesized by treatment with a reverse transcriptase with the synthesized oligonucleotide being used as a primer. A double-stranded cDNA may also be obtained by the following methods : mRNA is decomposed and removed by treatment with an alkali and the resulting single-stranded cDNA is treated first with a reverse transcriptase or DNA polymerase I (e.g. Klenow fragment), then with S1 nuclease ; alternatively, the mRNA may be directly treated with RNase H and DNA polymerase (e.g. E. coli polymerase I). For more information, see, Maniatis et al., "Molecular Cloning", Cold Spring Harbor Laboratory (1982) ; and Gubler and Hoffman, Gene, 25, 263 (1983).

The so obtained double-stranded cDNA is inserted into an appropriate vector such as, for example, one of the EK-type plasmid vectors typified by pSCl01, pDF41, ColE1, pMB9, pBR322, pBR327 and pACYCl, or one of the phage vectors typified by λgt, λc, λgt10 and λgtWES, and thereafter, the recombinant vector is used to transform a strain of E. coli (e.g. X1776, HB101, DH1 or C600) so as to obtain a cDNA library (see, for example, "Molecular Cloning", ibid.)

The double-stranded cDNA may be joined to a vector by the following procedures : a terminus of the cDNA is provided with a joinable end by attachment of an appropriate chemically synthesized DNA fragment ; and a vector DNA which has been cleaved with a restriction enzyme is joined to said cDNA by treatment with a T4 phage DNA ligase in the presence of ATP. Alternatively, dC, dG-chains (or dT, dA-chains) are attached, respectively, to the double-stranded cDNA and a vector DNA which has been cleaved with a restriction enzyme, and a solution containing both DNAs is annealed (see "Molecular Cloning", ibid.)

A host cell may be transformed by the so obtained recombinant DNA by any of the known methods. If the host cell is E. coli, the method detailed by Hanahan [J. Mol. Biol., 166, 557 (1983)] may be employed, wherein the recombinant DNA is added to a competent cell prepared in the presence of CaCl₂, MgCl₂ or RbCl.

Screening for the cells harboring the desired gene may be performed by several methods which include : the plus-minus method employed in the cloning of interferon cDNA [Taniguchi et al., Proc. Jpn. Acad., 55, Ser. B., 464 (1979)], the hybridization-translation assay method [Nagata et al., Nature, 284, 316 (1980)], and the colony or plaque hybridization method using an oligonucleotide probe which is chemically synthesized on the basis of the amino acid sequence of the protein having the human G-CSF activity [Wallace et al., Nucleic Acids Res., 9, 879 (1981)].

The fragment harboring the thus cloned gene coding for the polypeptide having the human G-CSF activity may be re-inserted in an appropriate vector DNA for the purpose of transforming other prokaryotic or eukaryotic host cells. By introducing an appropriate promoter and an expression-associated sequence into the vector, the gene can be expressed in an individual host cell.

Illustrative prokaryotic host cells include Escherichia coli, Bacillus subtilis, and Bacillus thermophilus. The gene of interest may be expressed within these host cells by transforming them with a replicon (i.e. a plasmid vector harboring an origin and regulator sequence) which is derived from a host-compatible species. A desirable vector is one having a sequence capable of providing the transformed cell with selectivity for expressed trait (phenotype).

To take an example, E. coli may be transformed with pBR322 which is a vector capable of replication in E. coli [see Bolivar, Gene, 2, 95 (1975)]. This vector contains both ampicillin- and tetracycline-resistance genes and either one of the properties may be used to identify the transformed cell. Examples of the promoter that is necessary for genetic expression in prokaryotic hosts include the promoter of the β-lactamase gene [Chang et al., Nature, 275, 615 (1978)], the lactose promoter [see Goeddel et al., Nature, 281, 544 (1979)] and the tryptophan promoter [see Goeddel et al., Nucleic Acid Res., 8, 4057 (1980)] and so on. Any of these promoters may be employed in the production of a polypeptide having the human G-CSF activity according to the present invention.

A eukaryotic microorganism such as Saccharomyces cerevisiae may be used as a host cell and transformed by a vector such as plasmid YRp7 [see Stinchcomb et al., Nature, 282, 39 (1979)]. This plasmid has the TRPI gene as a selection marker for yeast strains lacking the ability to produce tryptophan, so the transformants can be selected by performing growth in the absence of tryptophan. Examples of the promoter that can be utilized for gene expression include an acidic phosphatase gene promoter [Miyanohara et al., Proc. Natl. Acad. Sci., USA, 80, 1 (1983)] and an alcohol dehydrogenase gene promoter [Valenzuela et al., Nature, 298, 347 (1982)].

The host cell may also be derived from mammalian cells such as COS cells, Chinese hamster ovary (CHO) cells, C-127 cells and Hela cells. An illustrative vector that may be used to transform these cells is pSV2-gpt [see Mulligan and Berg ; Proc. Natl. Acad. Sci., USA, 78, 2072 (1981)]. The vectors used to transform these cells contain origin, selection marker, a promoter preceding in position the gene to be expressed, RNA splicing site, polyadenylation signal, etc.

Illustrative promoters that may be used for gene expression in mammalian cells include the promoters of a retrovirus, polyoma virus, adenovirus, simian virus 40 (SV40), etc. If the promoter of SV40 is used, the desired gene expression may be readily achieved in accordance with the method of Mulligan et al. described in Nature, 277, 108 (1979).

Illustrative origins that can be used include those derived from SV40, polyoma virus, adenovirus, bovine papilloma virus (BPV), etc. Illustrative selection markers that can be used include the phosphotransferase APH (3') II or I (neo) gene, thymidine kinase (TK) gene, E. coli xanthine-guanine phosphoribosyltransferase (Ecogpt) gene, dihydrofolate reductase (DHFR) gene, etc.

In order to obtain polypeptides having the human G-CSF activity from the above listed host-vector systems, the following procedures may be used : the gene coding for the peptide having the human G-CSF activity is inserted at a suitable site in one of the vectors mentioned above ; the host cell is transformed with the resulting recombinant DNA ; and the obtained transformants are cultured. The desired polypeptide may be isolated and purified from the cell or culture solution by any one of the known techniques.

Eukaryotic genes are generally held to exhibit polymorphysm as is known for the case of the human interferon gene [see Nishi et al., J. Biochem., 97, 153 (1985)] and this phenomenon may cause substitution of one or more amino acids or a change in the nucleotide sequence but no change in the amino acid sequence at all.

The G-CSF activity may also be possessed by a polypeptide which is deficient of one or more of the amino acids in the amino acid sequence shown in Fig. 3(B) or which has such amino acids added thereto, or a polypeptide which has one or more of these amino acids replaced by one or more amino acids. It is also known that a polypeptide obtained by converting each of the cysteine codons in the human interleukin-2 (IL-2) gene to a serine codon has the activity of interleukin-2 [Wang et al., Science, 224, 1431 (1984)]. Therefore, so long as the polypeptides, either naturally occurring or chemically synthesized, have the human G-CSF activity, all of the genes that code for these polypeptides are included within the scope of the present invention.

Hereunder outlined are the processes for producing the gene of the present invention coding for a polypeptide having the human G-CSF activity, a recombinant vector having said gene and a transformant having this recombinant vector, and a polypeptide or glycoprotein having the human G-CSF activity expressed in this transformant. ·

(1) Probe preparation

A homogeneous human CSF protein was purified from the supernatant of a culture of a tumor cell line, CHU-2, and its amino acid sequence from the N terminus was determined. Fragments were obtained by decomposition with bromocyan and treatment with trypsin and the amino sequences of these fragments were also determined [Example 3(i), (ii) and (iii)].

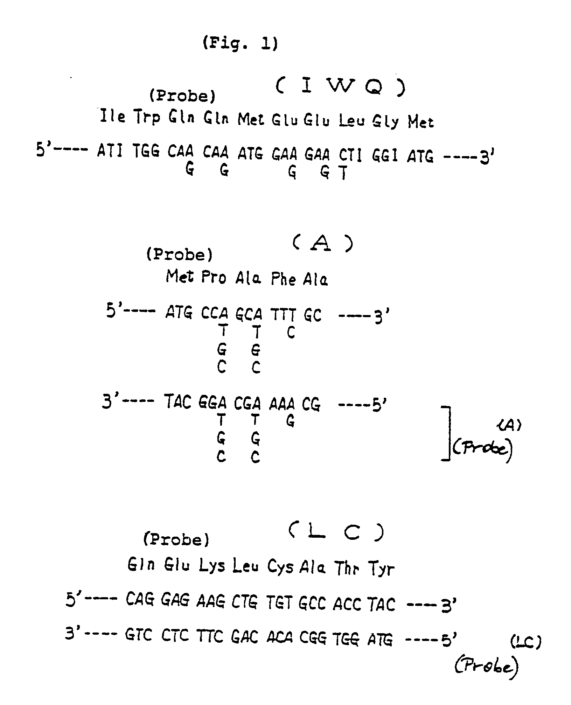

From the determined amino acid sequences, three nucleotide probes, (A), (LC) and (IWQ), having the sequences shown in Fig. 1 were synthesized (Example 4). Probe (A) was of the mixed type composed of 14 successive nucleotides. Probe (IWQ) was composed of 30 successive nucleotides with deoxyinosine and was a probe of the type used in the cloning of the human cholecystokinin gene [Takahashi et al., Proc. Natl. Acad. Sci., USA, 82, 1931 (1985)]. Probe (LC) was a 24-nucleotide probe that was synthesized from the nucleotides at 32 - 39 positions from the N terminus of the amino acid sequence shown in Example 3(i) on the basis of the nucleotide sequence shown in Fig. 3.

Chemical synthesis of nucleotides can be achieved by applying the improved phosphotriester method to the solid phase method and has been reviewed by Narang [Tetrahedron, 39, 3-22 (1983)].

Probes based on amino acid sequences at positions other than those in the above-mentioned probes may also be used.

(2) Construction of cDNA library

CHU-2 cells were homogenized after addition of a guanidine thiocyanate solution and the total RNA was obtained by CsCl density gradient centrifugation.

Poly(A+) RNA was isolated from the total RNA by column chromatography on oligo(dT)-cellulose. Thereafter, a single-stranded cDNA was synthesized with a reverse transcriptase, and RNase H and E. coli DNA polymerase I were added to obtain a double-stranded cDNA. A dC chain was attached to the obtained double-stranded cDNA, which was joined to a vector, pBR322, to which a dG chain had been attached at the Pst I cleavage site. The resulting recombinant DNA was used to transform a strain of E. coli, X1776, and a pBR322-line cDNA library was constructed (Examples 5 and 6).

In a similar manner, the double-stranded cDNA was joined to the λgt10 vector with the EcoRI linker and λ-phage line cDNA library was constructed (Example 7).

(3) Screening

Recombinants derived from the pBR322-line cDNA library were fixed on Whatmann 541 filter paper and a single clone could be selected by colony hybridization with $^{32}$P-labelled probe (IWQ). Further study with the Southern blotting method [Southern, J. Mol. Biol., 98, 503 (1975)] showed that this clone also hybridized with probe (A). The nucleotide sequence of this clone was determined by the dideoxy method [Sanger, Science, 214, 1205 (1981)].

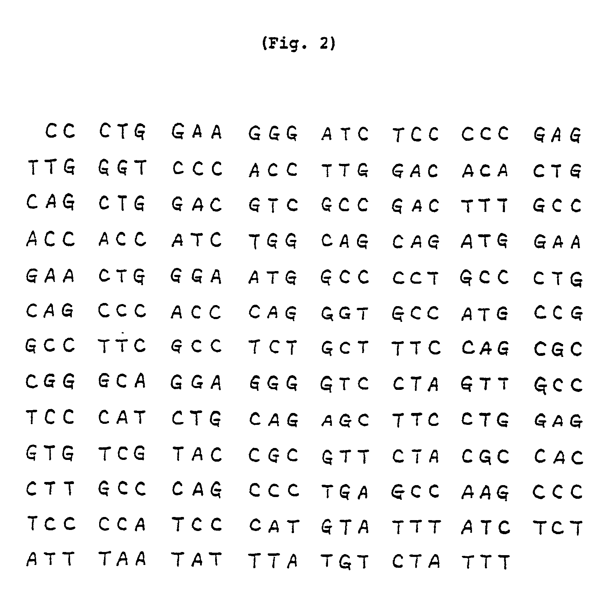

The nucleotide sequence of the obtained cDNA insert is shown in Fig. 2, from which one can see that this insert consisted of 308 base pairs including probes (IWQ) and (A), and had an open reading frame coding for 83 amino acids containing the amino acid sequence shown in Example 3(iii). The pBR322 derived plasmid containing these 308 base pairs is hereunder referred to as pHCS-1 (Example 8).

A DNA fragment containing the 308 base pairs obtained from pHCS-1 was radiolabelled by the nick translation method (see Molecular Cloning, ibid.) and, with this fragment used as a probe, the λgt10-derived cDNA library was screened by plaque hybridization [Benton and Davis, Science, 196, 180 (1977)] to obtain five clones. The nucleotide sequence of a clone which was believed to contain cDNA was determined by the same method as described above (Fig. 3(A)).

As shown in Fig. 3(A), this cDNA insert had a single large open reading frame.

The amino acid sequence encoded by this cDNA can be deduced as shown in Fig. 3(A).

Comparison with the N-terminal amino acid sequence of G-CSF protein shown in Example 3(i) revealed that this cDNA contained a nucleotide sequence which corresponded to both a signal peptide encoded by 90 base pairs starting with the ATG sequence at 32 - 34 nucleotide positions from 5'-terminus and ending with the GCC sequence at 119 - 121 positions, and a mature G-CSF polypeptide encoded by 531 base pairs starting with the ACC sequence at 122 - 124 positions and ending with the CCC sequence at 650 - 652 positions. Therefore, the polypeptide of the amino acid sequence I shown in Fig. 3(B) was composed of 207 amino acids and its molecular weight was calculated as 22292.67 daltons. The polypeptide of the amino acid sequence II was composed of 177 amino acids and its molecular weight was calculated as 18986.74 daltons (Example 9).

It should be noted that the ATG at 32 - 34 positions or at 68 - 70 positions can also be considered to be the protein initiation site. Escherichia coli strain X1776 harboring pBR322 which had this cDNA (+VSE) at the EcoRI cleavage site has been deposited with the Fermentation Research Institute, the Agency of Industrial Science and Technology (FERM BP-954).

Fig. 5 shows the restriction enzyme cleavage sites of the gene.

This cDNA was joined to pBR327 [Soberon et al., Gene, 9, 287 (1980)] at the EcoRI site and the resulting plasmid is hereunder referred to as pBRG4.

The thus obtained pBRG4 was treated with a restriction enzyme, EcoRI, to obtain a DNA fragment containing cDNA of about 1500 base pairs. This fragment was radiolabelled by the nick translation method (see Molecular Cloning, ibid.) and, with this radiolabelled DNA fragment being used as a probe, the λgt10-derived cDNA library was screened once again by plaque hybridization (see Benton and Davis, ibid.) In this plaque hybridization, two sheets of λ-Phage DNA fixed nitrocellulose filter paper were prepared ; one of these sheets was used for the above-mentioned plaque hybridization and another one was subjected to plaque hybridization with the already described probe (LC). The phages which turned positive for both probes were selected. A clone which has a "full-length" cDNA was selected and the nucleotide sequence of the cDNA insert as determined by the dideoxy method is shown in Fig. 4(A).

This cDNA had a single large open reading frame and the amino acid sequence that would be encoded by this cDNA was deduced as shown in Fig. 4(A).

Comparison with the N-terminal amino acid sequence of G-CSF protein shown in Example 3(i) revealed that this cDNA contained a nucleotide sequence which corresponded to both a signal peptide encoded by 90 base pairs starting with the ATG sequence at 31 - 33 nucleotide positions from 5′-terminus and ending with the GCC sequence at 118 - 120 positions, and a mature G-CSF polypeptide encoded by 522 base pairs starting with the ACC sequence at 121 - 123 positions and ending with the CCC sequence at 640 - 642 positions. Therefore, the polypeptide of the amino acid sequence I shown in Fig. 4(B) was composed of 204 amino acids and its molecular weight was calculated as 21977.35 daltons. The polypeptide of the amino acid sequence 11 was composed of 174 amino acids and its molecular weight was calculated as 18671.42 daltons (Example 10).

It should be noted that the ATG at 58 - 60 positions or at 67 - 69 positions can also be considered to be the protein initiation site.

Escherichia coli strain X1776 harboring pBR322 which had this cDNA (-VSE) at the EcoRI cleavage site has been deposited with the Fermentation Research Institute, the Agency of Industrial Science and Technology (FERM BP-955).

Fig. 5 shows the restriction enzyme cleavage sites of the gene. This cDNA was joined to pBR327 at the EcoRI site to form a plasmid which is hereunder referred to as pBRV2.

(4) Construction of recombinant vector for expression in E. coli

(A) +VSE line recombinant vector

From the so obtained pBRG4 plasmid (Example 9), a cDNA fragment of the G-CSF polypeptide was cut out with a restriction enzyme and a recombinant vector was constructed by one of the following methods :

(i) using an annealed synthetic linker, the fragment was ligated with a fragment prepared from a tac promoter-containing pKK223-3 (Pharmacia Fine Chemicals) (Example 11 and Fig. 6) ;

(ii) three fragments prepared from $P_L$ promoter containing pPL-lambda (Pharmacia Fine Chemicals) were ligated with an annealed synthetic linker and, the ligation product and the cDNA fragment were subjected to re-preparation procedures to construct a recombinant vector (Example 12, Fig. 7) ; or

(iii) using an annealed synthetic linker, the fragment was ligated with a fragment prepared from a trp promoter-containing pOYI plasmid (Example 13 and Fig. 8).

(B) -VSE line recombinant vector

In the same manner as described above, three recombinant vectors were constructed using the plasmid pBRV2 (Example 10) as shown in Example 18 and Figs. 9, 10 and 11.

(5) Preparation of E. coli transformants, and cultivation and expression thereof

Using three recombinant vectors of each of the +VSE and -VSE lines, E. coli strain DHI, N4830 or JM105 was transformed by the calcium chloride or rubidium chloride procedure described in Molecular Cloning, ibid.

(Examples 11, 12, 13 and 18). Each of the transformants obtained was cultivated in ampicillin-containing Luria medium, with induction being subsequently conducted as required to achieve expression (Examples 14 and 19).

(6) Recovery and purification of G-CSF polypeptide from E. coli and amino acid analysis thereof

A culture solution of the transformants was centrifuged to obtain a cell pellet. The collected cells were treated with a lysozyme and, after lysis by cyclic freezing and thawing, the supernatant was obtained. Alternatively, the cells were treated with guanidium chloride, centrifuged and the supernatant was recovered.

The supernatant was subjected to gel filtration on an Ultrogel ACA54 column (LKB) and the active fractions were concentrated with an ultrafiltration apparatus.

Subsequently, an aqueous solution of trifluoroacetic acid containing n-propanol was added to the concentrate and, after being left in ice, the mixture was centrifuged and adsorbed on a reverse-phase Cl8 column. After elution, the fractions were checked for their activity. The active fractions were collected and subjected to the same procedures of purification as described above. The purified fractions were freeze-dried and the powder was dissolved and subjected to high performance liquid chromatography based on molecular size. The obtained polypeptides were subjected to SDS-polyacrylamide gel electrophoresis and a single band for the desired G-CSF polypeptide was confirmed (Examples 15 and 19). The so obtained polypeptide showed human G-CSF polypeptide was analyzed by an amino acid analyzing method with a Hitachi 835 Automatic Amino Acid Analyzer (Hitachi, Ltd.) For analysis of the N-terminal amino acids, a gas-phase sequencer (for Edman decomposition), high performance liquid chromatographic apparatus and Ultrasphere-ODS column were used (Examples 17 and 20).

(7) Construction of recombinant vectors for animal cells

Recombinant vectors (derived from BPV) for use with Cl27 and NIH3T3 cells as host animal cells were constructed for each of the +VSE and -VSE lines. Recombinant vectors (derived from dhfr) for use with CHO cells were also constructed for each of the +VSE and -VSE lines. In the following, construction of recombinant vectors for use with Cl27 and CHO cells is outlined and, for further details, reference should be made to the relevant working examples.

(A) Construction of recombinant vectors of the +VSE line

The cDNA (+VSE) fragment obtained in (3) was inserted into a vector pdKCR to make a plasmid pHGA410 (Example 21 and Fig. 12), which was partially digested with EcoRI followed by treatment with DNA polymerase I (Klenow fragment) to create blunt ends. A linker HindIII was attached to the DNA, which was subsequently treated with HindIII and T4DNA ligase. The treated DNA was used to transform E. coli strain DHI by the rubidium chloride procedure (see Molecular Cloning, ibid.). The resulting plasmid was named pHGA410(H) (Fig. 13).

The pHGA410(H) was treated with SalI and, after blunt ends were created, it was treated with HindIII once again and a HindIII-SalI fragment was recovered. A plasmid pdBPV-1 having a transformed fragment of bovine papilloma virus was treated with HindIII and PvuII and the larger DNA fragment was separated and joined to the separately prepared HindIII-SalI fragment. The joined fragments were used to transform E. coli strain DHI to obtain a plasmid, pTN-G4, which had the pHGA410-derived CSF-cDNA (Fig. 13 and Example 22).

Either plasmid, pHGA410 or pHGA410(H), in combination with the plasmid pAdD26SVpA was used to construct pHGG4-dhfr which was a recombinant vector (+VSE) for use with CHO cells (Figs. 14a and b, and Example 24).

(B) Construction of -VSE line recombinant vectors

The cDNA (-VSE) fragment obtained in (3) was inserted into a vector pdKCR to make a plasmid pHGV2 (Example 27), which was partially digested with EcoRI followed by treatment with DNA polymerase I (Klenow fragment) to create blunt ends. A linker HindIII was attached to the DNA, which was subsequently treated with HindIII and T4DNA ligase. The treated DNA was used to transform E. coli strain DHI by the rubidium chloride procedure (see Molecular Cloning, ibid.) The resulting plasmid was named pHGV2(H) (Fig. 16).

The pHGV2(H) was treated with SalI and, after blunt ends were created, it was treated with HindIII once again and a HindIII-SalI fragment was recovered. A plasmid pdBPV-1 having a transformed fragment of bovine papilloma virus was treated with HindIII and PvuII and the larger DNA fragment was separated and joined to the separately prepared HindIII-SalI fragment. The joined fragments were used to transform E. coli strain DHI

to obtain a plasmid, pTN-V2, which had the pHGV2-derived CSF-cDNA (Fig. 16 and Example 28).

By similar procedures, either plasmid, pHGV2 or pHGV2(H), in combination with the plasmid pAdD26SVpA was used to construct pHGV2-dhfr which was a recombinant vector (-VSE) for use with CHO cells (Figs. 17a and b, and Example 30).

(8) Expression in animal cells

Expression of animal cells is hereunder described, with mouse Cl27 cells being taken as an example. For further details, see the relevant working examples.

Plasmid pTN-G4 or pTN-V2 was treated with BamHI. The treated plasmid was used to transform Cl27 cells (previously grown by cultivation) by the calcium phosphate procedure. The transformed cells were cultured and clones having high pCSF production rate were selected. Glycoproteins containing the expressed G-CSF were recovered and purified from the culture solution of the transformed cells and were found to have human G-CSF activity. The presence of the desired glycoprotein was also confirmed by amino acid and sugar content analyses of the sample.

For sugar content analysis, the CSF sample used in amino acid analysis was subjected to determination of amino sugar by the Elson-Margan method, determination of neutral sugar by the orcinol sulfate procedure, or determination of sialic acid by the thiobarbiturate procedure. The procedures of each determination are shown in "Tohshitsu no Kagaku "Chemistry of Saccharides" (Part 2 of two parts)", Chapter 13, Vol. 4 of A Course in Biochemical Experiments, published by Tokyo Kagaku Dojin. Conversion of the measured values into weight percent revealed that the sugar content of the G-CSF obtained was distributed within the range of 1 - 20 wt% depending upon the type of host cells, expression vectors and the cultivation conditions.

The present invention also provides an infection protective agent containing human G-CSF as an effective ingredient.

The human G-CSF which is an effective ingredient of the infection protective agent can be obtained by the gene recombinant techniques described in the foregoing pages. Alternatively, it may be obtained from a human CSF producing cell (CHU-1 or CHU-2) in accordance with the method described in Japanese Patent Application No. 153273/1984, or by cultivating, in the presence or absence of a mitogen, a hybridoma which is a cell-fusion product of G-CSF producing cells and self-proliferating malignant tumor cells.

All of the human G-CSFs that are obtained by the methods described above are included within the scope of the present invention.

The human G-CSF containing solution obtained may be further purified and concentrated by any of the known techniques as required, then stored frozen. Alternatively, it may be stored after being dehydrated by such means as freeze-drying or vacuum-drying. If desired, the human G-CSF may be dissolved in an appropriate buffer solution, then an injection is prepared by filtering the solution aseptically through an appropriate medium such as a Millipore filter.

The infection protective agent of the present invention may contain a pharmaceutically acceptable carrier or excipient, or any necessary stabilizer or adsorption-preventing agent to provide a pharmaceutical preparation that is suitable for administration to humans or animals.

The dosage and the frequency of administration of the human G-CSF present in the infection protective agent of the present invention may be properly determined in consideration of the severity of the disease in the patient, but typically a preparation containing 0.1 - 500 g, preferably 5 - 100 g, of human G-CSF per adult may be administered in one to seven doses during the course of one week. It should however be noted that the present invention is by no means limited by the content of human G-CSF.

The infection protective agent of the present invention containing human G-CSF as an effective ingredient may be used effectively against various infectious microorganisms which may be illustrated by, but by no means limited to, the following : Gram-positive cocci such as Staphylococcus and Streptococcus ; Gram-negative anaerobic facultative microorganisms including enterobacteria such as Escherichia, Serratia and Klebsiella, and Hemophilus ; Gram-negative aerobic microorganisms such as Pseudomonas and Alcaligenes ; Gram-negative anaerobic microorganisms such as Bacteroides ; fungi such as Candida and Aspergillus ; and intracellular parasitic microorganisms such as Listeria. The infection protective agent of the present invention will exhibit excellent effects against infections caused by a single species of these microorganisms or against mixed infections caused by more than one species of such microoragnisms.

Examples

Before the present invention is described in greater detail with reference to working examples, the following referential examples are provided for the purpose of illustrating the methods of assaying the CSF activity, the isolation of human G-CSF from CHU-1, and the physicochemical properties of the isolated human G-CSF.

Referential Example 1 : Assaying CSF Activity

The following methods were used to determine the CSF activity (hereunder abbreviated as CSA) in the present invention.

CSA assay

(a) With human bone marrow cells :

Single-layer soft agar cultivation was conducted in accordance with the method of Bradley, T.R. and Metcalf, D. (Aust. J. Exp. Biol. Med. Sci., 44, 287-300, 1966). More specifically, 0.2 ml of a bovine fetal serum, 0.1 ml of the sample, 0.1 ml of a human bone marrow nonadherent cell suspension (1 - 2 x $10^5$ nuclear cells), 0.2 ml of a modified McCoy's 5A culture solution, and 0.4 ml of a modified McCoy's 5A culture solution containing 0.75% of agar were mixed, poured into a plastic dish for tissue culture (35 mm$^\varnothing$), coagulated, and cultured at 37°C in 5% $CO_2$/95% air and at 100% humidity. Ten days later, the number of colonies formed was counted (one colony consisting of at least 50 cells) and CSA was determined with one unit being the activity required for forming one colony.

(b) With mouse bone marrow cells :

A horse serum (0.4 ml), 0.1 ml of the sample, 0.1 ml of a C3H/He (female) mouse bone marrow cell suspension (0.5 - 1 x $10^5$ nuclear cells), and 0.4 ml of a modified McCoy's 5A culture solution containing 0.75% of agar were mixed, poured into a plastic dish for tissue culture (35 mm$^\varnothing$), coagulated, and cultured for 5 days at 37°C in 5% $CO_2$/95% air and at 100% humidity. The number of colonies formed was counted (one colony consisting of at least 50 cells) and CSA was determined with one unit being the activity for forming one colony.

The modified McCoy's 5A culture solution used in each of the methods (a) and (b) and the human bone marrow nonadherent cell suspension used in (a) were prepared by the following procedures.

Modified McCoy's 5A culture solution (double concentration)

Twelve grams of MccCoy's 5A culture solution (Gibco), 2.55 g of MEM amino acid-vitamin medium (Nissui Seiyaku Co., Ltd.), 2.18 g of sodium bicarbonate and 50,000 units of potassium penicillin G were dissolved twice in 500 ml of distilled water and the solution was aseptically filtered through a Millipore filter (0.22 μm).

Human bone marrow nonadherent cell suspension

A bone marrow fluid obtained from a healthy person by sternal puncture was diluted 5-fold with an RPMI 1640 culture solution, plated over a Ficoll-Paque solution (Pharmacia Fine Chemicals) and centrifuged at 400 x g for 30 minutes at 25°C. The interfacial cell layer (specific gravity <1.077) was recovered. The cells were washed, adjusted to a concentration of 5 x $10^6$ cells/ml with an RPMI 1640 culture solution containing 20% of bovine fetal serum, poured into a 25-cm$^2$ plastic flask for tissue culture, and incubated for 30 minutes in a $CO_2$ incubator. Nonadherent cells were recovered in the supernatant, poured into a plastic flask (25 cm$^2$) and incubated for 2 hours and a half. Nonadherent cells in the supernatant were collected and used in an assay.

Referential Example 2 : Isolation of Human G-CSF

Human G-CSF was isolated and purified from the supernatant of a culture of human G-CSF producing cells, CHU-1 (C.N.C.M. Accession Number I-315) by the procedures described in Example 2 which follows.

The so obtained human G-CSF had the following physicochemical properties.

I) Molecular weight : ca. 19,000 ± 1,000 as measured by SDS-polyacrylamide gel electrophoresis.

II) Isoelectric point : Having at least one of the three isoelectric points, A, B and C, noted in the following Table I.

## Table I
## Isoelectric point (pI)

|   | in the presence of 4 M urea | in the absence of any urea |
|---|---|---|
| A | $5.7 \pm 0.1$ | $5.5 \pm 0.1$ |
| B | $6.0 \pm 0.1$ | $5.8 \pm 0.1$ |
| C | $6.3 \pm 0.1$ | $6.1 \pm 0.1$ |

III) Ultraviolet absorption : Maximum absorption at 280 nm and minimum absorption at 250 nm.

IV) The amino acid sequence of the 21 residues from N terminus was as follows :

$H_2N$ – Thr – Pro – Leu – Gly – Pro – Ala – Ser – Ser –
          (10)
Leu – Pro – Gln – (Ser) – Phe – Leu – Leu – Lys – X –
          (20)
Leu – Glu – X – Val –

V) Amino acid composition : See the following Table II.

## Table II

| Amino acid | Found (n mol) | Predicted number of amino acid residues (rounded to integral values in parentheses) |
|---|---|---|
| Asp (Asp + Asn) | 3.54 | 4.3 ( 4) |
| Thr | 4.58 | 5.5 ( 6) |
| Ser | 10.64 | 12.9 (13) |
| Glu (Glu + Gln) | 22.31 | 27.0 (27) |
| Pro | 8.30 | 10.1 (10) |
| Gly | 10.60 | 12.8 (13) |
| Ala | 14.85 | 18.0 (18) |
| 1/2 Cys | 2.59 | 3.1 ( 3) |
| Val | 6.16 | 7.5 ( 7) |
| Met | 2.26 | 2.7 ( 3) |
| Ile | 3.29 | 4.0 ( 4) |
| Leu | 27.24 | 33.0 (33) |
| Tyr | 2.60 | 3.1 ( 3) |
| Phe | 5.08 | 6.1 ( 6) |
| Lys | 3.68 | 4.5 ( 4) |
| His | 3.93 | 4.8 ( 5) |
| Trp | 1.61 | 1.9 ( 2) |
| Arg | 4.29 | 5.2 ( 5) |
| Total | | (166) |
| Calculated molecular weight (no sugar counted in 166 residues) | | 17,961 |

Example 1 : Establishment of CHU-2

A tumor of a patient with oral cavity cancer wherein pronounced increase was observed in the number of neutrophiles was transplanted into nu/nu mice. About 10 days after the transplantation, the increase in the weight of the tumor and in the number of neutrophiles was pronounced. Twelve days after the transplantation, the tumor was extracted aseptically, shredded into cubes of 1 - 2 mm³ and cultured in the following manner.

Ten to fifteen cubes of the tumor were put into a 50-ml plastic centrifugal tube. After addition of 5 ml of a trypsin solution (containing 0.25% of trypsin and 0.02% of EDTA), the tube was shaken for 10 minutes in a warm bath at 37°C and the supernatant was discarded. Another 5 ml of the same trypsin solution was added and trypsin digestion was conducted under agitation for 15 minutes at 37°C. The supernatant cell suspension was recovered and stored in ice after the trypsin had been inactivated by addition of 1 ml of a bovine fetal serum.

After repeating these procedures once again, the cell suspension was recovered, combined with the previously obtained suspension, and centrifuged at 15,000 rpm for 10 minutes to obtain a cell pellet. The pellet was washed twice with F-10 containing 10% of a bovine fetal serum and was thereafter loaded in a plastic culture flask (25 cm²) to give a cell concentration of 5 x 10⁶ cells/flask. After incubation overnight in a $CO_2$ incubator (5% $CO_2$ and 100% humidity) with an F-10 culture solution containing 10% of a bovine fetal serum, the supernatant was removed together with the nonadherent cells, and culture was continued with a fresh supply of culture solution. Six days after the start of culture, the flask became full of the cells and the culture solution was replaced by a fresh one. On the next day, the culture solution was discarded and the flask was charged with 2 ml of an anti-mouse erythrocyte antibody (Cappel) diluted 5-fold with RPMI 1640 and 2 ml of a guinea pig complement (Kyokuto Seiyaku Co., Ltd.) diluted 2.5-fold with RPMI 1640. After incubation for 20 minutes at 37°C, the culture was washed twice with F-10 containing 10% of a bovine fetal serum and the nu/nu mouse derived fibroblasts were removed. Subsequently, an F-10 culture solution containing 10% of a bovine fetal serum was added and cultivation was conducted for 2 more days. Thereafter, same of the cells were recovered and subjected to cloning by the limiting dilution method.

The resulting 11 clones were checked for their CSF activity and one clone (CHU-2) exhibited activity about 10 times as high as that of the other clones.

Example 2 : Isolation of CSF

The cells established in Example 1 were grown in a completely dense population in two culture flasks (150 cm²). The cells were recovered, suspended in 500 ml of an F-10 culture solution containing 10% of a bovine fetal serum, transferred into a glass roller bottle of 1580 cm² (Belco), and whirl-cultured at 0.5 rpm. When the cells were found to have grown in a completely dense population on the inner wall of the roller bottle, the culture solution was replaced by a serum-free RPMI 1640. After 4-day culture, the supernatant of the culture was recovered and cultivation was continued with F-10 containing 10% of a bovine fetal serum being added. After 3-day culture, the culture solution was again replaced by a serum-free RPMI 1640 and the supernatant of the culture was recovered 4 days later. By repeating these procedures, 500 ml of the serum-free supernatant of culture per bottle was obtained each week. In addition, this method enabled the supernatant of culture to be recovered, with the cells maintained over a significantly prolonged period.

A batch consisting of 5,000 ml of the supernatant of the culture obtained was mixed with 0.01% of Tween 20 and concentrated about 1000 times by ultrafiltration with Hollow Fiber DC-4 and Amicon PM-10 (Amicon). The concentrate was purified by the following steps.

(i) A portion (5 ml) of the concentrated supernatant of culture was subjected to gel filtration on an Ultrogel AcA54 column (4.6 cmØ x 90 cmᴸ ; LKB) at a flow rate of ca. 50 ml/hr with 0.01 M Tris-HCl buffer (pH 7.4) containing 0.15 M NaCl and 0.01% Tween 20 (Nakai Kagaku Co., Ltd.) The column had been calibrated with bovine serum albumin (Mw ; 67,000), ovoalbumin (Mw ; 45,000) and cytochrome C (Mw ; 12,400). After completion of the gel filtration, 0.1 ml of each of the fractions was diluted 10-fold and screened for the active fractions by the above-described method of CSA assay (b). The fractions for Ve = 400 - 700 ml were found to exhibit macrophage-dominant CSA while the fractions for Ve = 800 - 1200 ml showed granulocyte-dominant CSA. Therefore, the latter fractions were collected and concentrated to a volume of ca. 5 ml on an ultrafiltration apparatus with PM-10 (Amicon).

(ii) To the concentrated fractions was added an aqueous solution of 0.1% trifluoroacetic acid containing 30% of n-propanol (for determination of amino acid sequence ; available from Tokyo Kasei K.K.) After the mixture had been left to stand in ice for about 15 minutes, the precipitate was removed by centrifugation for 10 minutes at 15,000 rpm. The supernatant was adsorbed on a μ-Bondapak CI8 column (8 mm x 30 cm for semipreparatory use ; Waters) equilibrated with the aqueous solution containing n-propanol and trifluoroacetic acid ; the column was continuously eluted with an aqueous solution of 0.1% trifluoroacetic acid which contained n-propanol having a linear concentration gradient of 30 - 60%. A high performance liquid chromatographic apparatus, Hitachi Model 685-50 (Hitachi, Ltd.), and a detector, Hitachi Model 638-41 (Hitachi, Ltd.) were employed to determine the absorptions at 220 nm and 280 nm simultaneously. After elution, 10 μl of each of the fractions was diluted 100-fold and screened for the active fractions by the above-described method of CSA assay (b). The peaks eluted with 40% n-propanol were found to have CSA activity, so they were collected, re-chromatographed under the same conditions, and assayed for CSA by the same method. Again, CSA activity was observed in the peaks at 40% n-propanol. Therefore, these peaks were collected (4 fractions = 4 ml) and freeze-dried.

(iii) The freeze-dried powder was dissolved in 200 μl of an aqueous solution of 0.1% trifluoroacetic acid containing 40% of n-propanol, and the solution was subjected to high performance liquid chromotography on TSK-G 3000SW column (Toyo Soda Manufacturing Co., Ltd. ; 7.5 mm x 60 cm). Elution was conducted with the same aqueous solution at a flow rate of 0.4 ml/min and the fractions were taken in 0.4-ml portions with a fraction collector, FRAC-100 (Pharmacia Fine Chemicals). Each of the fractions taken was checked for its CSA by the same method as described above and activity was observed in the fractions for retention times of 37 - 38 minutes (corresponding to MW of ca. 2 x 10$^4$). The active fractions were recovered and purified on an analytical μ-Bondapak Cl8 column (4.6 mm x 30 cm). The main peaks were recovered and freeze-dried. The sample obtained was assayed by the method of CSA assay (a) ; it was found to have human G-CSF activity.

Example 3 : Determination of Amino Acid Sequence

(i) Determination of N-terminal amino acid sequence

The sample was subjected to Edman decomposition with a gas-phase sequencer (Applied Biosystems) and the resulting PTH amino acid was analyzed by routine procedures with a high performance liquid chromatographic apparatus (Beckman Instruments) and Ultrasphere-ODS column (Beckman Instruments). The column (5 μm ; 4.6 mm$^\oslash$ x 250 mm$^L$) was equilibrated with a starting buffer [aq. sol. containing 15 mM sodium acetate buffer (pH 4.5) and 40% acetonitrile] and injected with the sample (as dissolved in 20 l of the starting buffer). Separation was effected by isocratic elution with the starting buffer. The flow rate was 1.4 ml/min and the column temperature was held at 40°C. Detection of the PTH amino acid was achieved utilizing the absorptions in the UV range at 269 nm and 320 nm. Standard samples of PTH amino acid (Sigma) in 2-nmol portions were separated on the same line to determine their retention times, which were compared with those of the sample to be tested. As a result, the sample was found to have the following amino acid sequence of the 40 residues from N-terminus :

$$
\begin{aligned}
&\mathrm{H_2N} - \underset{(10)}{\mathrm{Thr}} - \mathrm{Pro} - \mathrm{Leu} - \mathrm{Gly} - \mathrm{Pro} - \mathrm{Ala} - \mathrm{Ser} - \mathrm{Ser} - \\
&\mathrm{Leu} - \mathrm{Pro} - \underset{(20)}{\mathrm{Gln}} - \mathrm{Ser} - \mathrm{Phe} - \mathrm{Leu} - \mathrm{Leu} - \mathrm{Lys} - \mathrm{Cys} - \\
&\mathrm{Leu} - \mathrm{Glu} - \mathrm{Gln} - \underset{(30)}{\mathrm{Val}} - \mathrm{Arg} - \mathrm{Lys} - \mathrm{Ile} - \mathrm{Gln} - \mathrm{Gly} - \\
&\mathrm{Asp} - \mathrm{Gly} - \mathrm{Ala} - \mathrm{Ala} - \underset{(40)}{\mathrm{Leu}} - \mathrm{Gln} - \mathrm{Glu} - \mathrm{Lys} - \mathrm{Leu} - \\
&\mathrm{Cys} - \mathrm{Ala} - \mathrm{Thr} - \mathrm{Tyr} - \mathrm{Lys} -
\end{aligned}
$$

(ii) Decomposition with bromocyan

The sample was dissolved in 70% formic acid. To the solution, 200 equivalent amounts of bromocyan that had been purified by sublimation was added. The mixture was left overnight at 37°C for reaction. The reaction product was freeze-dried and fractionated by HPLC on a TSK G3000SW column (Toyo Soda Manufacturing Co., Ltd.) to obtain four peaks. The peaks were named CN-1, CN-2, CN-3 and CN-4 in the decreasing order of the molecular weight. The first two peaks (CN-1 and CN-2) had better yields and their amino acid sequences were analyzed with an automatic gas-phase sequencer (Applied Biosystems) under the same conditions as used in (i).

As a result, CN-1 was found to be a peptide from the N-terminus of G-CSF protein, and CN-2 had the following amino acid sequence :

Pro - Ala - Phe - Ala - Ser - Ala - Phe -
Gln - Arg - Arg - Ala - Gly - Gly - Val -
Leu - Val - Ala - Ser - His - Leu - Gln -

(iii) Decomposition with trypsin

The sample was dissolved in 0.1 M Tris-HCl buffer (pH 7.4) containing 8 M urea and the solution was mixed with 0.1 M Tris-HCl buffer (pH 7.4) containing 0.1% 2-mercaptoethanol to provide a final urea concentration of 2 M. A TPCK-treated trypsin (Sigma) was added such that the sample-to-enzyme ratio was 50 :1. The mixture was held for 4 hours at 25°C and, after addition of an equal amount of TPCK-treated trypsin, the mixture was held for an additional 16 hours at 25°C. Thereafter, the reaction product was subjected to high-speed reverse-phase column chromatography on C8 column (Yamamura Kagaku K.K.), with elution conducted with 0.1% TFA containing n-propanol having a linear density gradient of 5 - 60%. While several peaks were obtained by measuring the absorption at 280 nm, the main peak was analyzed for its amino acid sequence with an automatic gas-phase sequencer (Applied Biosystems) under the same conditions as used in (i). As a result, the main peak was found to be a peptide having the following sequence which contained part of the CN-2 fragment shown in (ii) :

    Gln - Leu - Asp - Val - Ala - Asp - Phe - Ala - Thr -
    Thr - Ile - Trp - Gln - Gln - Met - Glu - Glu - Leu -
    Gly - Met - Ala - Pro - Ala - Leu - Gln - Pro - Thr -
    Gln - Gly - Ala - Met - Pro - Ala - Phe - Ala - Ser -

Example 4 : Preparation of DNA Probe

(i) Synthesis of probe (IWQ)

Thirty successive nucleotides (see Fig. 1) were prepared on the basis of the sequence of 10 amino acids (Ile-Trp-Gln-Gln-Met-Glu-Glu-Leu-Gly-Met) included within the amino acid sequence obtained in Example 3(iii). It will be necessary to make one comment about the notation of nucleotides shown in Fig. 1 ; for example, the nucleotide at 9-position from 5'-terminus is an equimolar mixture of dA and dG. The starting nucleotides were mostly dimers but mononucleotides were also used as required. A glass filter equipped column was charged with 20 mg of the starting nucleotide resin, Ap-d(G) (Yamasa Shoyu Co., Ltd.) After repeated washing with methylene chloride, the 4,4'-dimethoxytrityl group was eliminated by treatment with a solution of methylene chloride containing 3% trichloroacetic acid. Subsequently, the column was washed several times with 1 ml of methylene chloride. After the column was washed with anhydrous pyridine to displace the solvent, 20 mg of a nucleotide dimer, (DMTr)ApTp(NHR$_3$), (Nippon Zeon ; NHR$_3$ = triethylammonium ; DMTr = dimethoxytrityl) and 0.2 ml of pyridine were added, and the interior of the column was vacuum-dried with a vacuum pump. Subsequently, 20 mg of 2,4,6-trimethylbenzenesulfonyl-3-nitrotriazolide (MSNT of Wako Pure Chemical Industries, Ltd.) and 0.2 ml of anhydrous pyridine were added, and the interior of the column was displaced with a nitrogen gas. The nucleotide resin was condensed with the dimer by reaction for 45 minutes at room temperature, with occasional shaking. After completion of the reaction, the column was washed with pyridine and the unreacted OH groups were acetylated with a pyridine solution containing excess acetic anhydride and 4-dimethylaminopyridine. After washing the column with pyridine, the following dimers or monomers were condensed, in the order written, by repeating the above-described procedures : (DMTr)Ip(NHR$_3$), (DMTr)GpGp(NHR$_3$), (DMTr)Ip(NHR$_3$), an equimolar mixture of (DMTr)CpTp(NHR$_3$) and (DMTr)TpTp(NHR$_3$), an equimolar mixture of (DMTr)ApAp(NHR$_3$) and (DMTr)ApGp(NHR$_3$), an equimolar mixture of (DMTr)ApGp(NHR$_3$) and (DMTr)GpGp(NHR$_3$), (DMTr)GpAp(NHR$_3$), (DMTr)TpGp(NHR$_3$), an equimolar mixture of (DMTr)ApAp(NHR$_3$) and (DMTr)GpAp(NHR$_3$), (DMTr)CpAp(NHR$_3$), an equimolar mixture of (DMTr)ApAp(NHR$_3$) and (DMTr)ApGp(NHR$_3$), (DMTr)GpCp(NHR$_3$), (DMTr)TpGp(NHR$_3$), (DMTr)Ip(NHR$_3$) and (DMTr)ApTp(NHR$_3$), with all of these nucleotides being available from Nippon Zeon except for (DMTr)Ip(NHR$_3$) which was available from Yamasa Shoyu Co., Ltd. After completion of the reaction in the final stage, the resin was washed successively with pyridine, methylene chloride and ether without acetylation, and thereafter dried. The dried resin was suspended in 1.7 ml of a mixture of pyridine (0.5 ml), water (0.2 ml) and dioxane (1 ml) containing 1 M tetramethylguanidine and 1 M α-picolinaldoxime. The suspension was left to stand overnight at room temperature and concentrated to 100 - 200 μl under vacuum. The concentrate was mixed with a small amount (2 - 3 drops) of pyridine and 2 - 3 ml of concentrated aqueous ammonia, and the mixture was heated at 55°C for 6 hours. Following extraction with ethyl aetate, the aqueous layer was separated and concentrated under vacuum. The concentrate was dissolved in a solution of 50 mM triethyl ammonium acetate (pH 7.0) and the solution was subjected to chromatography on C-18 column (1.0 x 15 cm ; Waters), with elution conducted with acetonitrile (linear density gradient of 10 - 30%) in a solution of 50 mM triethyl ammonium acetate (pH 7.0). The peak fraction eluted at an acetonitrile concentration of about 25% was concentrated under vacuum.

To the concentrate, 80% acetic acid was added and the mixture was left to stand for 30 minutes at room temperature. Following extraction with ethyl acetate, the aqueous layer was separated and concentrated under vacuum. The resulting concentrate was further purified by high performance liquid chromatography on C-18 column (from Senshu Kagaku K.K. ; SSC-ODS-272 ; 6⌀ x 200 mm). Elution was conducted with acetonitrile (10 - 20% linear density gradient) in a solution of 50 mM triethyl ammonium acetate (pH 7.0). A synthetic DNA was obtained in a yield no lower than 10A$_{260}$ units.

Analysis by the Maxam-Gilbert sequencing method [Meth. Enzym., 65 499 (1980)] revealed that the oligonucleotide obtained had the nucleotide sequence shown in Fig. 1.

(ii) Synthesis of probe (A)

Fourteen successive nucleotide (see Fig. 1) were obtained on the basis of the sequence of 5 amino acids (Met-Pro-Ala-Phe-Ala) included within the amino acid sequence obtained in Example 3(iii).

Synthesis procedures were similar to those employed in the preparation of probe (IWQ), and the following nucleotides were condensed to a nucleotide resin, Ap-d(T) (Yamasa Shoyu Co., Ltd.) in the order written : (DMTr)CpAp(NHR$_3$), (DMTr)GpGp(NHR$_3$), an equimolar mixture of (DMTr)CpAp(NHR$_3$), (DMTr)CpTp(NHR$_3$), (DMTr)CpGp(NHR$_3$) and (DMTr)CpCp(NHR$_3$), an equimolar mixture of (DMTr)ApGp(NHR$_3$), (DMTr)TpGp(NHR$_3$), (DMTr)GpGp(NHR$_3$) and (DMTr)CpGp(NHR$_3$), (DMTr)ApAp(NHR$_3$), an equimolar mixture of (DMTr)CpAp(NHR$_3$) and (DMTr)CpGp(NHR$_3$), and (DMTr)Gp(NHR$_3$), with all nucleotides being available from Nippon Zeon. A synthetic DNA was obtained in a yield of ca. 10A$_{260}$ units. Analysis by the Maxam-Gilbert sequencing method revealed that the oligonucleotide obtained had the nucleotide sequence shown in Fig. 1.

(iii) Synthesis of probe (LC)

Automatic DNA synthesis was accomplished with a DNA synthesizer, Model 380A of Applied Biosystems. This technique, based on the principles described by Caruthers et al. [J. Am. Chem. Soc., 103, 3185 (1981)], is generally referred to as the phosphoramidite procedure.

A phosphoramidite form of (DMTr)-dT preliminarily activated with tetrazole was condensed to dG-S (S : support) wherein 5'-dimethoxytrityl group (DMTr) was deblocked.

Thereafter, the unreacted hydroxyl groups were acetylated and oxidized with iodine in the presence of water to make a phosphoryl group. After deblocking the DMTr group, condensation was repeated in the same manner until 24 nucleotides having the sequence shown in Fig. 1 were synthesized. These nucleotides were cleaved from the support, deblocked, and purified by reverse-phase high performance liquid chromatography on C-18 column (Senshu Kagaku Co., Ltd. ; SSC-ODS-272).

Example 5 : Cultivation of CHU-2 Cells and Preparation of mRNA

(1) Cultivation and recovery of CHU-2 cells

Established CHU-2 cells were grown in a completely dense population in two culture flasks (150 cm$^2$), recovered, suspended in 500 ml of an RPMI 1640 culture solution containing 10% of a bovine fetal serum, transferred into a glass roller bottle of 1580 cm$^2$ (Belco), and whirl-cultured for 4 days at 0.5 rpm. When the cells were found to have grown in a completely dense population on the inner wall of the roller bottle, the culture solution was removed from the roller bottle, which was charged with 100 ml of a preheated (37°C) physiological saline solution containing 0.02% of EDTA. After heating at 37°C for 2 minutes, the cells were separated from the inner wall of the flask by pipetting. The resulting cell suspension was centrifuged at 1500 rpm for 10 minutes to obtain a cell pellet. The cells were resuspended in 5 ml of an EDTA-free physiological saline solution. The suspension was centrifuged at 1500 rpm for 10 minutes to obtain a cell pellet (wet weight, ca. 0.8 g). The so obtained cells were stored frozen at -80°C until they were subjected to procedures for extraction of RNA.

(2) Purification of mRNA

Isolation of mRNA from the CHU-2 cells obtained in 1) was accomplished by procedures which were essentially the same as those described in "Molecular Cloning", Maniatis et al., Cold Spring Harbor, page 196, 1982. The frozen CHU-2 cells (wet weight, 3.8 g) were suspended in 20 ml of a solution of 6 M guanidine [6 M guanidinium isothiocyanate, 5 mM sodium citrate (pH 7.0), 0.1 M β-mercaptoethanol, and 0.5% sodium sarcosyl sulfate] and the suspension was well mixed by vortexing for 2 - 3 minutes. The mixture was subjected to 10 cyclic suction and ejection with a syringe (capacity, 20 ml) equipped with a 18G needle. About 6 ml of the viscous guanidinium solution containing the disrupted cells was layered onto a 6-ml cushion of 5.7 M CsCl in 0.1 M EDTA (pH 7.5) in a Beckman SW40 Ti polyallomer centrifuge tube in such a manner that the tube became full of the contents. Four centrifuge tubes were prepared by the procedures described above and centrifuged at 30,000 rpm for 15 hours at 20C. The resulting pellets were washed three times with a small amount of 70% ethanol.

The pellets obtained from the respective tubes were combined, dissolved in 550 µl of water and worked up to provide a NaCl concentration of 0.2 M. After treatment with a 1 :1 mixture of phenol and chloroform and with chloroform alone, 2.5 volumes of ethanol were added to precipitate the total RNA (ca. 10.1 mg of the total RNA was obtained from 3.8 g of wet cells).

Poly(A⁺) RNA was purified from the total RNA by the following procedures of affinity chromatography taking advantage of the attachment of a poly(A) chain at 3' terminus of the mRNA. Adsorption on oligo(dT)-cellulose (Type 7 of P-L Biochemicals) was achieved by passage through an oligo(dT)-cellulose column of the total RNA in a loading buffer [containing 10 mM Tris-HCl (pH 7.5), 0.5 M NaCl, 1 mM EDTA, and 0.1% SDS solution] after the solution had been heated at 65°C for 5 minutes. The column had been equilibrated with the same loading buffer. Elution of poly(A⁺) RNA was accomplished with a TE solution [containing 10 mM Tris-HCl (pH 7.5) and 1 mM EDTA]. The unadsorbed effluent was re-charged through the column and the eluate obtained by repeating the same procedures was mixed with the first run of eluate. As a result, 400 μg of the poly(A⁺) RNA was obtained.

The so prepared mRNA was fractionated for size by sucrose density gradient centrifugation in accordance with the procedures described in the laboratory manual of Schleif and Wensink, "Practical Methods in Molecular Biology", Springer-Verlag, New york, Heidelberg, Berlin (1981).

Stated more specifically, a 5 - 25% sucrose density gradient was created in a Backman SW40 Ti centrifuge tube. Two sucrose solutions were prepared by dissolving 5% and 25% of RNase-free sucrose (Schwarz/Mann) in a solution containing 0.1 M NaCl, 10 mM Tris-HCl (pH 7.5), 1 mM EDTA, and 0.5% SDS.

Eight hundred micrograms of the mRNA [poly(A⁺)-RNA] prepared by the method already described was dissolved in 200 - 500 μl of a TE solution. The solution was heated at 65°C for 5 minutes and, after being quenched, it was placed on the sucrose density gradient solutions, which were centrifuged at 30,000 rpm for 20 hours. Fractions each weighing 0.5 ml were collected and their absorption at 260 nm was measured. The sizes of the fractionated RNAs were determined on the basis of the positions of standard RNAs (ribosome RNAs 28S, 18S and 5S). At the same time, the G-CSF activity of each fraction was examined with oocytes of Xenopus laevis by the following procedures. First, the mRNA of each fraction was worked up into an aqueous solution having a concentration of 1 μg/μl ; oocytes were taken from Xenopus (about one year old) and the mRNA solution was injected in such a manner that a 50-ng of mRNA was injected into one oocyte ; ten such oocytes were placed in each of 96 wells in a microtiter plate ; the oocytes were cultured for 48 hours at room temperature in 100 μl of a Barth medium [88 mM NaCl ; 1 mM KCl ; 2.4 mM NaHCO₃ ; 0.82 mM MgSO₄ ; 0.33 mM Ca(NO₃)₂; 0.41 mM CaCl₂ ; 7.5 mM Tris-HCl (pH 7.6) ; penicillin, 10 mg/L ; and streptomycin sulfate, 10 mg/L] ; the supernatant of the culture was recovered, concentrated and purified to a grade suitable for assay of G-CSF activity.

The G-CSF activity was found to be present in 15 - 17S fractions.

Example 6 : Synthesis of cDNA (Construction of pBR-line cDNA Library)

From the poly(A⁺) RNA obtained in Example 6 was synthesized cDNA by the method of Land et al. [Nucleic Acids Res., 9, 2251 (1981)] as modified by the method of Gubler and Hoffman [Gene, 25, 263 (1983)].

(1) Synthesis of single-stranded cDNA

An Eppendorf tube (capacity, 1.5 ml) was charged with reagents in the following order : 80 μl of a reaction buffer (500 mM KCl, 50 mM MgCl₂, 250 mM Tris-HCl, pH 8.3) ; 20 μl of 200 mM dithiothreitol, 32 μl of 12.5 mM dNTP (containing 12.5 mM each of dATP, dGTP, dCTP and dTTP), 10 μl of α-³²P-dCTP (PB 10205 of Amerscham), 32 μl of oligo(dT)₁₂₋₁₈ (from P-L Biochemicals ; 500 μg/ml), 20 μl of poly(A⁺) RNA (2.1 μg/μl), and 206 μl of distilled water. A total of 400 μl of the reaction solution was heated at 65°C for 5 minutes, and thereafter heated at 42°C for 5 minutes. To the heated solution, 120 units of a reverse transcriptase (Takara Shuzo Co., Ltd.) was added. Following reaction for 2 more hours at 42°C, 2 μl of an RNase inhibitor (Bethesda Research Laboratories), 20 μl of a TE solution, 16 μl of 100 mM sodium pyrophosphate and 48 units (4 μl) of a reverse transcriptase were added, and reaction was carried out at 46°C for 2 hours. The reaction was quenched by addition of 0.5 M EDTA (8 μl) and 10% SDS (8 μl). By subsequent treatment with phenol/chloroform and precipitation with ethanol (twice), a single-stranded cDNA was obtained.

(2) Attachment of dC-chain to the single-stranded cDNA

The single-stranded cDNA obtained in 1) was dissolved in distilled water. To the solution was added 60 μl of a dC-chain adding buffer [400 mM potassium cacodylate, 50 mM Tris-HCl (pH 6.9), 4 mM dithiothreitol, 1 mM CoCl₂, and 1 mM dCTP], and the mixture was heated at 37°C for 5 minutes. To the reaction solution, 3 μl of a terminal transferase (27 units/μl ; P-L Biochemicals) was added and the mixture was heated at 37°C for 2.5 minutes. Following treatment with phenol/chloroform (once) and precipitation with ethanol (twice), the dC-tailed cDNA was dissolved in 40 μl of a TE solution containing 100 mM NaCl.

(3) Synthesis of double-stranded cDNA

To 40 µl of the DNA solution prepared in 2), 4 µl of oligo(dG)$_{12-18}$ (200 µg/ml ; P-L Biochemicals) was added and the mixture was heated first at 65°C for 5 minutes, then at 42°C for 30 minutes. While the reaction solution was held at 0°C, 80 µl of a buffer [100 mM Tris-HCl (pH 7.5), 20 mM MgCl$_2$, 50 mM (NH$_4$)$_2$SO$_4$, and 500 mM KCl], 4 µl of 4 mM dNTP (containing 4 mM each of dATP, dCTP, dGTP and dTTP), 60 µl of 1 mM β-NAD, 210 µl of distilled water, 20 µl of E. coli DNA polymerase I (Takara Shuzo Co., Ltd.), 15 µl of E. coli DNA ligase (Takara Shuzo Co., Ltd.) and 15 µl of E. coli RNase H (Takara Shuzo Co., Ltd.) were added, and the mixture was subjected to reaction at 12°C for 1 hour. Following addition of 4 mM dNTP (4 µl), reaction was carried out at 25°C for 1 hour. By subsequent treatment with phenolchloroform and precipitation with ethanol (once), about 8 µg of a double-stranded cDNA was obtained. This double-stranded cDNA was dissolved in a TE solution and subjected to 1.2% agarose gel electrophoresis. Fragments corresponding to the size of ca. 560 bp to 2 kb were adsorbed on Whatman DE81 and about 0.2 µg of the double-stranded cDNA could be recovered by elution.

(4) Attachment of dC-chain to the double-stranded cDNA

The double-stranded cDNA prepared in 3) was dissolved in 40 µl of a TE solution. After 8 µl of a dC-tail adding buffer of the type identified in 2) had been added, the mixture was heated at 37°C for 2 minutes. Following addition of 1 µl of a terminal transferase (27 units/µl), the mixture was subjected to reaction at 37°C for 3 minutes. Thereafter, the reaction solution was immediately cooled to 0°C, and the reaction was quenched by addition of 1 µl of 0.5 M EDTA. Following treatment with phenol/chloroform and precipitation with ethanol, the precipitate obtained was suspended in 10 µl of a TE solution.

(5) Construction of pBR-line cDNA library

Four microliters of a commercial oligo(dG)-tailed pBR322 vector (Bethesda Research Laboratories ; 10 ng/µl) and 2 µl of the dC-tailed double-stranded cDNA obtained in 4) were annealed in a TE solution containing 75 µl of 0.1 M NaCl. The annealing consisted of three stages : heating at 65°C for 5 minutes, subsequent heating at 40°C for 2 hours, followed by cooling to room temperature.

In accordance with the method described in the laboratory manual of Maniatis et al. [Molecular Cloning, Cold Spring Harbor, p 249 ff. (1982)] (other routine techniques could also be used here), competent cells were prepared from E. coli strain X1776, and transformed with the annealed plasmid to produce transformants.

Example 7 : Synthesis of cDNA (Construction of λphage Library)

(1) Synthesis of single-stranded cDNA

In accordance with the procedures described in Example 5, 3.8 g of frozen CHU-2 cells were purified twice on an oligo(dT)-cellulose column and subsequently worked up to obtain 400 µg of poly(A$^+$) RNA.

A TE solution (10 µl) having 12 µg of the poly(A$^+$) RNA dissolved therein was placed in a reaction tube containing 10 µg of actinomycin D (Sigma). Thereafter, the tube was charged with reagents in the following order : 20 µl of a reverse transcription buffer [250 mM Tris-HCl (pH 8.3) ; 40 mM MgCl$_2$ ; 250 mM KCl] ; 20 µl of 5 mM dNTP (containing 5 mM each of dATP, dGTP, dCTP and dTTP) ; 20 µl of oligo(dT)$_{12-18}$ (0.2 µg/ml ; P-L Biochemicals) ; 1 µl of 1 M dithiothreitol ; 2 µl of RNasin (30 units/µl ; Promega Biotech) ; 10 µl of a reverse transcriptase (10 units/µl ; Seikagaku Kogyo Co., Ltd.) ; 1 µl of α-$^{32}$P-dATP (10 µCi ; Amerscham) ; and 16 µl of water. The reaction solution totalling a volume of 100 µl was held at 42°C for 2 hours and the reaction was quenched by addition of 0.5 M EDTA (5 µl) and 20% SDS (1 µl). By subsequent treatment with phenol/chloroform (100 µl) and precipitation with ethanol (twice), about 4 µg of a single-stranded cDNA was obtained.

(2) Synthesis of double-stranded cDNA

The cDNA obtained in 1) was dissolved in 29 µl of a TE solution and a reaction solution was prepared by adding the following reagents in the order written : 25 µl of a polymerase buffer [400 mM Hepes (pH 7.6) ; 16 mM MgCl$_2$, 63 mM β-mercaptoethanol, and 270 mM KCl] ; 10 µl of 5 mM dNTP ; 1.0 µl of 15 mM β-NAD ; 1.0 µl of α-$^{32}$-P-dATP (10 µCi/µl) ; 0.2 µl of E. coli DNA ligase (60 units/µl ; Takara Shuzo Co., Ltd.) ; 5.0 µl of E. coli DNA polymerase I (New England Biolabs ; 10 units/µl) ; 0.1 µl of RNase H (60 units/µl ; Takara Shuzo Co., Ltd.) ; and 28.7 µl of distilled water.

The reaction solution was incubated at 14°C for 1 hour, allowed to return to room temperature, and incubated for an additional hour. Then, the reaction was quenched by addition of 0.5 M EDTA (5 µl) and 20% SDS (1 µl), and treatment with phenol/chloroform and precipitation with ethanol were performed. The DNA obtained was dissolved in 20 µl of 0.5 mM EDTA and a reaction solution was prepared by addition of 3 µl of a Klenow buffer [500 mM Tris-HCl (pH 8.0) and 50 mM MgCl$_2$], 3 µl of 5 mM dNTP, and 4 µl of water. After addition of 1 µl of a DNA polymerase (Klenow fragment ; Takara Shuzo Co., Ltd.), the reaction solution was incubated at 30°C for 15 minutes.

The incubated reaction solution was diluted with 70 µl of a TE solution and the reaction was quenched by addition of 0.5 M EDTA (5 µl) and 20% SDS (1 µl). By subsequent treatment with phenol/chloroform and precipitation with ethanol, about 8 µg of a double-stranded cDNA was obtained.

(3) Methylation of double-stranded cDNA

An aqueous solution (30 µl) of the double-stranded cDNA synthesized in 2) was mixed with 40 µl of a methylation buffer [500 mM Tris-HCl (pH 8.0) ; 50 mM EDTA], 20 µl of a SAM solution [800 µM S-adenosyl-L-methylmethionine (SAM) ; 50 mM β-mercaptoethanol], and 100 µl of water. To the mixture, 15 µl of an EcoRI methylase (New England Biolabs ; 20 units/µl) was added to make a reaction solution totalling 200 µl in volume. Following incubation at 37°C for 2 hours, treatments with phenol and ether and precipitation with ethanol were conducted to recover the DNA.

(4) Addition of EcoRI linker

To about 1.2 µg of the methylated double-stranded DNA, 1.5 µl of a ligase buffer [250 mM Tris-HCl (pH 7.5) and 100 mM MgCl$_2$], 0.5 µl of a preliminarily phosphorylated EcoRI linker (10mer ; Takara Shuzo Co., Ltd.), 1.5 µl of 10 mM ATP, 1.5 µl of 100 mM dithiothreitol, and 2 µl of H$_2$O were added to make a reaction solution totalling 15 µl in volume. After 0.7 µl of T$_4$ DNA ligase (3.4 units/µl ; Takara Shuzo Co., Ltd.) had been added, reaction was carried out overnight at 4°C. Thereafter, the ligase was inactivated by heating at 65°C for 10 minutes. The reaction solution was worked up to a total volume of 50 µl by addition of 100 mM Tris-HCl (pH 7.5), 5 mM MgCl$_2$, 50 mM NaCl and 100 µg/ml of gelatin. Following addition of EcoRI (3.5 µl ; 10 units/µl), reaction was carried out at 37°C for 2 hours. Subsequently, 2.5 µl of 0.5 M EDTA and 0.5 µl of 20% SDS were added, followed by treatment with phenol/chloroform and precipitation with ethanol so as to recover the DNA. Thereafter, the unreacted EcoRI linker was removed by gel filtration on Ultrogel AcA34 (LKB) or agarose-gel electrophoresis, so as to recover about 0.5 - 0.7 µg of the linker-added double-stranded cDNA.

(5) Joining double-stranded cDNA to λgt10 vector

The linker-added double-stranded cDNA was mixed with 2.4 µg of preliminarily EcoRI-treated λgt10 vector (Vector Cloning system), 1.4 µl of a ligase buffer (250 mM Tris-HCl and 100 mM MgCl$_2$), and 6.5 µl of distilled water, and the mixture was heated at 42°C for 15 minutes. Thereafter, 1 µl of 10 mM ATP, 1 µl of 0.1 M dithiothreitol and 0.5 µl of T$_4$ DNA ligase were added to make a total volume of 15 µl and reaction was carried out overnight at 12°C.

(6) In vitro packaging

About a third of the recombinant DNAs prepared in 5) was packed with an in vitro packaging kit (Promega Biotech) to obtain phage plaques.

Example 8 : Screening of pBR-Line Library with Probe (IWQ)

Whatman 541 paper was placed on a colony-growing agar medium and left to stand at 37°C for 2 hours. The filter paper was subsequently treated by the following method of Taub and Thompson [Anal. Biochem., 126, 222 (1982)].

The colonies transferred onto the 541 paper was further grown onto an agar medium containing chloramphenicol (250 µg/µl) overnight at 37°C.

The 541 paper was recovered and left at room temperature for 3 minutes on another sheet of filter paper that had been impregnated with a 0.5 N NaOH solution. This procedure was repeated twice. Two similar runs were conducted for 3 minutes using a solution of 0.5 M Tris-HCl (pH 8). At 4°C, treatments were conducted with a solution of 0.05 M Tris-HCl (pH 8) for 3 minutes, and with 1.5 mg/ml of a lysozyme solution [containing 0.05 M Tris-HCl (pH 8) and 25% sucrose] for 10 minutes ; then, at 37°C, treatments were conducted with a solution of 1 x SSC (0.15 M NaCl and 0.015 M sodium citrate) for 2 minutes, and with a 1 x SSC solution containing 200 µg/ml of proteinase K for 30 minutes ; finally, at room temperature, treatments were conducted with a 1 x SSC solution for 2 minutes, and with 95% ethanol solution for 2 minutes. The final step was repeated twice. Thereafter, the 541 paper was dried. The dried 541 paper was immersed in a 25 :24 :1 mixture of phenol/chloroform/isoamylalcohol [equilibrated with 100 mM Tris-HCl (pH 8.5), 100 mM NaCl and 10 mM EDTA] for 30 minutes at room temperature. Subsequently, similar procedures were repeated three times with a 5 x SSC solution for 3 minutes, then twice with a 95% ethanol solution for 3 minutes. Thereafter, the filter paper was dried.

The probe (IWQ) was labelled with $^{32}$P in accordance with routine procedures (see Molecular Cloning) and colony hybridization was performed in accordance with the method of Wallace et al. [Nucleic Acids Res., 9, 879 (1981)]. Prehybridization was conducted at 65°C for 4 hours in a preprehybridization buffer containing 6 x NET [0.9 M NaCl ; 0.09 M Tris-HCl (pH 7.5) ; and 6 mM EDTA], 5 x Denhardt's solution, 0.1% SDS and 0.1 mg/ml of denatured DNA (calf thymus). Thereafter, hybridization was conducted overnight at 56°C in a hybridization buffer (for its formulation, see above) containing 1 x 10⁶ cpm/ml of the radiolabelled probe (IWQ). After completion of the reaction, the 541 paper was washed twice with a 6 x SSC solution (containing 0.1% SDS) for 30 minutes at room temperature, then washed at 56°C for 1.5 minutes. The washed 541 paper was then subjected to autoradiography.

The plasmid was separated from positive clones and subjected to Southern blotting with the probe (IWQ). Hybridization and autoradiography were conducted under the same conditions as described above.

Similarly, Southern blotting was conducted with the probe (A). Using a hybridization buffer having the formulation shown above, hybridization was conducted first at 49°C for 1 hour. After leaving it to 39°C, hybridization was further continued at the same temperature for 1 hour. After completion of the reaction, a nitrocellulose filter was washed twice with 0.1% SDS containing 6 x SSC for 30 minutes at room temperature, then washed at 39°C for 3 minutes. The washed paper was subjected to autoradiography.

As a result, a single clone was found to be positive. Nucleotide sequencing by the dideoxy method revealed that this clone had a DNA composed of 308 base pairs containing the portions of both probe (IWQ) and probe (A). The pBR322-derived plasmid containing this insert was named pHCS-1.

Example 9 : Screening of λPhage Line Library with pHCS-1 Derived DNA Probe

Plaque hybridization was conducted in accordance with the method of Benton and Davis [Science, 196, 180 (1977)]. The pHCS-1 obtained in Example 8 was treated with Sau3A and EcoRI to obtain a DNA fragment of ca. 600 bp. This DNA fragment was radiolabelled by nick translation in accordance with routine procedures. A nitrocellulose filter (S & S) was placed on the phage plaque-growing agar medium to transfer the phages onto the filter. After denaturing the phage DNA with 0.5 M NaOH, the filter paper was treated by the following procedures : treatment with 0.1 M NaOH and 1.5 M NaCl for 20 seconds ; two treatments with 0.5 M Tris-HCl (pH 7.5) and 1.5 M NaCl for 20 seconds ; finally, treatment with 120 mM NaCl, 15 mM sodium citrate, 13 mM KH₂PO₄ and 1 mM EDTA (pH 7.2) for 20 seconds.

The filter was subsequently dried and heated at 80°C for 2 hours to immobilize the DNA. Prehybridization was conducted overnight at 42°C in a prehybridization buffer containing 5 x SSC, 5 x Denhardt's solution, 50 mM phosphate buffer, 50% formamide, 0.25 mg/ml of denatured DNA (salmon sperm DNA) and 0.1% SDS. Thereafter, hybridization was conducted at 42°C for 20 hours in a hybridization buffer containing 4 x 10⁵ cpm/ml of pHCS-1 probe that had been radiolabelled by nick translation. This hybridization buffer was a mixture of 5 x SSC, 5 x Denhardt's solution, 20 mM phosphate buffer (pH 6.0), 50% formamide, 0.1% SDS, 10% dextran sulfate and 0.1 mg/ml of denatured DNA (salmon sperm DNA).

The hybridized nitrocellulose filter was washed for 20 minutes with 2 x SSC containing 0.1 SDS at room temperature, then for 30 minutes with 0.1 x SSC containing 0.1% SDS at 44°C, and finally for 10 minutes with 0.1 x SSC at room temperature. Detection by autoradiography was then conducted.

As a result, five positive clones (G1 - G5) were obtained. The clone contained a "full-length" cDNA was checked for its DNA nucleotide sequence by the dideoxy method and the nucleotide sequence shown in Fig. 3(A) was identified. This cDNA was cut out of the λgt10 vector and joined to pBR327 [Soberon et al., Gene, 9, 287 (1980)] at the EcoRI site to form a plasmid which could be prepared on a large scale. This plasmid is named pBRG4.

Example 10 : Screening of λPhage Line Library with pBRG4-Derived DNA Probe and Probe (LC)

Plaque hybridization was performed in accordance with the method of Benton and Davis (see Science, ibid.) employed in Example 9. A nitrocellulose filter (S & S) was placed on the phage plaque-growing agar medium to transfer the phages onto the filter. After denaturing the phage DNA with 0.5 M NaOH, the filter was treated by the following procedures : treatment with 0.1 M NaOH and 1.5 M NaCl for 20 seconds ; then two treatments with 0.5 M Tris-HCl (pH 7.5) and 1.5 M NaCl for 20 seconds ; finally, treatment with 120 mM NaCl, 15 mM sodium citrate, 13 mM $KH_2PO_4$ and 1 mM EDTA (pH 7.2) for 20 seconds. The filter was subsequently dried, and heated at 80°C for 2 hours to immobilize the DNA. Two sheets of the same filter were prepared in the manner described above and subjected to screening with the pBRG4-derived DNA probe and the probe (LC).

Screening with the pBRG4-derived DNA probe was carried out by the following procedures. The pBRG4 was treated with EcoRI to obtain a DNA fragment of ca. 1500 bp. This DNA fragment was radiolabelled by nick translation in accordance with routine procedures. One of the two nitrocellulose filters was subjected to prehybridization overnight at 42°C in a prehybridization buffer containing 5 x SSC, 5 x Denhardt's solution, 50 mM phosphate buffer, 50% formamide, 0.25 mg/ml of denatured DNA (salmon sperm DNA) and 0.1% SDS. Thereafter, the filter was subjected to hybridization at 42°C for 20 hours in a hybridization buffer containing the radiolabelled DNA probe (ca. $1 \times 10^6$ cpm/ml) of ca. 1500 bp. This hybridization buffer was a mixture of 5 x SSC, 5 x Denhardt's solution, 20 mM phosphate buffer (pH 6.0), 50% formamide, 0.1% SDS, 10% dextran sulfate and 0.1 mg/ml of denatured DNA (salmon sperm DNA). The hybridized nitrocellulose filter was washed for 20 minutes with 2 x SSC containing 0.1% SDS at room temperature, then for 30 minutes with 0.1 x SSC containing 0.1% SDS at 44°C, and finally for 10 minutes with 0.1 x SSC at room temperature. Detection by autoradiography was then conducted.

Screening with the probe (LC) was carried out by the following procedures. The other filter was preliminarily treated with 3 x SSC containing 0.1% SDS at 65°C for 2 hours. Then, prehybridization was conducted at 65°C for 2 hours in a solution containing 6 x NET, 1 x Denhardt's solution, and 100 μg/ml of denatured DNA (salmon sperm DNA). Hybridization was subsequently conducted overnight at 63°C in a hybridization buffer containing the radiolabelled probe (LC) ($2 \times 10^6$ cpm/ml). This hybridization buffer was also a mixture of 6 x NET, 1 x Denhardt's solution and 100 μg/ml of denatured DNA (salmon sperm DNA). The hybridized nitrocellulose filter was washed three times (20 minutes each) with 6 x SSC containing 0.1% SDS at room temperature, then washed with 6 x SSC containing 0.1% SDS at 63°C for 2 minutes.

The filter was dried and detection was conducted by autoradiography.

In the screening described above, clones which were positive to both probes were selected and the clone which contained a "full-length" cDNA was checked for its nucleotide sequence by the dideoxy method. It was found to have the nucleotide sequence shown in Fig. 4(A). This cDNA was cut out of the λgt10 vector and joined to pBR327 at the EcoRI site to prepare a plasmid pBRV2.

Example 11 : Construction of E. coli Recombinant Vector (+VSE) and Transformation (Using tac Promoter-Containing Vector)

(1) Construction of recombinant vector

(i) Vector preparation

Five micrograms of a tac promoter-containing vector pKK223-3 (Pharmacia) was treated with 8 units of EcoRI (Takara Shuzo Co., Ltd.) for 2 hours at 37°C in 30 μl of a reaction solution (40 mM Tris-HCl, 7 mM $MgCl_2$, 100 mM NaCl, and 7 mM 2-mercaptoethanol).

Subsequently, 3 μl of an alkali phosphatase (Takara Shuzo Co., Ltd.) was added and treatment was conducted at 60°C for 30 minutes. A DNA fragment was recovered by three treatments with phenol, one treatment with ether and precipitation with ethanol, all being conducted in accordance with routine procedures.

The recovered DNA fragment was dissolved in a 50-μl mixture composed of 50 mM Tris-HCl, 5 mM $MgCl_2$, 10 mM DTT, and 1 mM each of dATP, dCTP, dGTP and dTTP. After addition of 3 μl of an E. coli DNA polymerase I - Klenow fragment (Takara Shuzo Co., Ltd.), reaction was carried out at 14°C for 2 hours to create blunt ends.

(ii) Preparation of synthetic linker

Three micrograms of oligonucleotides having the sequences of synthetic linkers, CGAAT-GACCCCCCTGGGCC and CAGGGGGGGTCATTCG, was phosphorylated by performing reaction in 40 μl of a reaction solution (composed of 50 mM Tris-HCl, 10 mM $MgCl_2$, 10 mM 2-mercaptoethanol and 1 mM ATP) at 37°C for 60 minutes in the presence of 4 units of $T_4$ polynucleotide kinase.

Each of the phosphorylated oligonucleotides (0.2 μg) was dissolved in 20 μl of a 100 mM NaCl-containing TE solution [10 mM Tris-HCl (pH 8.0) and 1 mM EDTA]. After treatment at 65°C for 10 minutes, the oligonucleotides were annealed by slow cooling to room temperature.

(iii) Preparation of G-CSF cDNA fragment

Sixty micrograms of the pBRG4 prepared in Example 9 which contained the cDNA shown in Fig. 3(A) was treated with 100 units of a restriction enzyme ApaI (New England Biolabs) and 50 units of DraI (Takara Shuzo Co., Ltd.) at 37°C for 3 hours in 200 μl of a reaction solution composed of 6 mM Tris-HCl, 6 mM MgCl$_2$, and 6 mM 2-mercaptoethanol. About 2 μg of an ApaI - DraI fragment (ca. 590 bp) was recovered by 1.2% agarose gel electrophoresis.

(iv) Ligation of fragments

About 0.1 μg each of the fragments prepared in (i) to (iii) was dissolved in 20 μl of a ligation solution (66 mM Tris-HCl, 6.6 mM MgCl$_2$, 10 mM DTT, and 1 mM ATP). After addition of 175 units of T$_4$ DNA ligase, the solution was held overnight at 4°C to obtain a recombinant vector (Fig. 6).

(2) Transformation

Using 20 μl of a reaction solution containing the recombinant vector prepared in (iv), E. coli strain JM105 was transformed by the rubidium chloride procedure [see T. Maniatis et al., Molecular Cloning, p. 252 (1982)]. The plasmid was separated from an ampicillin-resistant colony culture of the transformants and treated with restriction enzymes, BamHI, AccII and ApaI to confirm that the transformants were the intended ones.

Example 12 : Construction of E. coli Recombinant Vector (+VSE) and Transformation (Using PL promoter-Containing Vector)

(1) Construction of recombinant vector

(i) Vector preparation

A hundred micrograms of a PL promoter-containing vector pPL-lambda (Pharmacia) was treated overnight at 37°C with 50 units of a restriction enzyme BamHI in 100 μl of a reaction solution [10 mM Tris-HCl (pH 7.6), 7 mM MgCl$_2$, 100 mM NaCl, and 10 mM DTT].

By subjecting the reaction solution to 1% agarose gel electrophoresis, about 49 μg of an approximately 4-kb fragment and about 11 μg of an approximately 1.2-kb fragment were recovered.

The 4-kb fragment was dissolved in 100 μl-of a TE buffer (for its composition, see above) and dephosphorylated by reaction with an alkali phosphatase (Takara Shuzo Co., Ltd.) at 60°C for 60 minutes.

The other fragment of about 1.2 kb in length was dissolved in 20 μl of a buffer (10 mM Tris-HCl, 10 mM MgCl$_2$, 6 mM KCl, and 1 mM DTT) and treated overnight with 20 units of a restriction enzyme MboII (New England Biolabs) at 37°C.

By 4% polyacrylamide gel electrophoresis, about 0.9 μg of a BamHI-MboII fragment (ca. 200 bp) and about 1.9 μg of an MboII-BamHI fragment (ca. 310 bp) were recovered.

(ii) Preparation of synthetic linker

Oligonucleotides having the sequences of synthetic linkers, TAAGGAGAATTCATCGAT and TCGATGAATTCTCCTTAG, were phosphorylated and annealed as in (ii) in Example 11, so as to prepare a synthetic S/D linker.

(iii) Preparation of expression vector

One tenth of a microgram of the ca. 4-kb fragment, 0.05 μg each of the BamHI-MboII fragment having the O$_L$P$_L$ region and the MboII-BamHI fragment having the tL$_1$ region [the three fragments being prepared in (i)], and 0.1 μg of the annealed synthetic S/D linker prepared in (ii) were subjected to reaction overnight at 12°C in 40 μl of a reaction solution (66 mM Tris-HCl, 6.6 mM MgCl$_2$, 10 mM DTT, and 1 mM ATP) in the presence of 175 units of T$_4$ DNA ligase (Takara Shuzo Co., Ltd.) Twenty microliters of the reaction solution was used to transform E. coli strain N99CI$^+$ (Pharmacia) by the calcium chloride procedure (see Molecular Cloning, ibid.)

The transformants were cultured and the plasmid was recovered from the culture of their ampicillin-resistant colonies. Treatment of the plasmid with restriction enzymes, EcoRI, BamHI and SmaI, showed that it was the intended plasmid.

Two micrograms of this plasmid was reacted with a restriction enzyme ClaI (New England Biolabs) at 37°C for 2 hours in 20 µl of a buffer (10 mM Tris-HCl, 6 mM $MgCl_2$ and 50 mM NaCl). Thereafter, the enzyme was inactivated by heating at 65°C for 10 minutes.

One microliter of the reaction solution was reacted overnight at 12°C with 175 units of $T_4$ DNA ligase (Takara Shuzo Co., Ltd.) in a ligation solution having the composition described above. The reaction solution was then used to transform E. coli strain N99cI+ (Pharmacia). The plasmid was recovered from the culture of ampicillin-resistant colonies of the transformants and treated with EcoRI and BamHI to confirm that said plasmid was the intended one.

### (iv) Preparation of G-CSF expressing recombinant vector and transformants

The expression plasmid prepared in (iii) was treated with a restriction enzyme ClaI. After creating blunt ends, the plasmid was then worked up as in Example II to prepare a recombinant vector inserted a cDNA fragment of G-CSF. This vector was used to transform E. coli strain N4830 (Pharmacia) by the calcium chloride procedure described in Molecular Cloning (ibid.) Identification of the desired transformants was achieved as in Example 11 (Fig. 7).

### Example 13 : Construction of E. coli Recombinant Vector (+VSE) and Transformation (Using trp Promoter-Containing Vector)

#### (1) Construction of recombinant vector

#### (i) Vector preparation

A plasmid, pOYI, was prepared by inserting a tryptophan promoter containing HpaII-TaqI fragment (ca. 330 bp) into pBR322 at the ClaI site. Ten micrograms of this plasmid was treated with 7 units of a restriction enzyme ClaI and 8 units of PvuII at 37°C for 3 hours in 30 µl of a reaction solution composed of 10 mM Tris-HCl, 6 mM $MgCl_2$ and 50 mM NaCl.

Subsequently, 2 µl of an alkali phosphatase (Takara Shuzo Co., Ltd.) was added and reaction was carried out at 60°C for 1 hour.

A DNA fragment (ca. 2.5 µg) of about 2.6 kb in length was recovered from the reaction solution by 1% agarose gel electrophoresis.

#### (ii) Preparation of Synthetic linker

Oligonucleotides having the sequences of synthetic linkers, CGCGAATGACCCCCCTGGGCC and CAGGGGGGTCATTCG, were phosphorylated and annealed as in (ii) in Example 11, so as to prepare a synthetic linker.

#### (iii) Preparation of recombinant vector

About 1 µg of the vector fragment prepared in (i), about 1 µg of the synthetic linker prepared in (ii) and about 1 µg of the G-CSF cDNA fragment prepared in (iii) in Example 11 were reacted with 175 units of $T_4$ DNA ligase overnight at 12°C in 20 µl of a ligation solution having the formulation described in Example 11, 1)(iv), so as to obtain a recombinant vector (Fig. 8).

#### (2) Transformation

Twenty microliters of the reaction solution prepared in (iii) was used to transform E. coli DHI by the rubidium chloride procedure described in Molecular Cloning, ibid.

As in Example 11, the plasmid was recovered from amplicillin-resistant colonies of the transformants, and treatment of this plasmid with restriction enzymes, Apal, DraI, NruI and PstI, showed that the desired transformants had been obtained.

Example 14 : Cultivation of Transformants

(1) Cultivation of the transformants (with tac) obtained in Example 11

The transformants were cultured overnight at 37°C, and 1 ml of the culture was added to 100 ml of a Luria medium containing 25 μg/ml or 50 μg/ml of amplicillin. Cultivation was conducted for 2 - 3 hours at 37°C.

The cultivation was continued at 37°C for 2 - 4 hours after addition of isopropyl-β-D-thiogalactoside to make final concentration to 2 mM.

(2) Cultivation of the transformants (with $P_L$) obtained in Example 12

The transformants were cultured overnight at 28°C, and 1 ml of the culture was added to 100 ml of a Luria medium containing 25 or 50 μg/ml of ampicillin. Cultivation was conducted for about 4 hours at 28°C.

The cultivation was continued for 2 - 4 hours at 42°C.

(3) Cultivation of the transformants (with trp) obtained in Example 13

The transformants were cultured overnight at 37°C, and 1 ml of the culture was added to 100 ml of M9 medium containing 0.5% glucose, 0.5% Casamino acids (Difco) and 25 or 50 μg/ml of ampicillin. Cultivation was conducted for 4 - 6 hours at 37°C. After addition of 50 μg/ml of 3-β-indolacrylic acid (IAA), the cultivation was continued for 4 - 8 hours at 37°C.

Example 15 : Recovery and Purification of G-CSF Polypeptide from E. coli

(1) Recovery

The three species of transformants cultured in Example 14 were subjected to the following recovery procedures.

The culture (100 ml) was centrifuged to obtain a cell pellet, which was suspended in 5 ml of a mixture of 20 mM Tris-HCl (pH 7.5) and 30 mM NaCl.

Then, 0.2 M phenylmethylsulfonyl fluoride, 0.2 M EDTA and a lysozyme were added in respective concentrations of 1 mM, 10 mM and 0.2 mg/ml, and the suspension was left for 30 minutes at 0°C.

The cells were lyzed by three cycles of freezing/ thawing, followed by optional sonication. The lysate was centrifuged to obtain the supernatant. Alternatively, the lysate was treated with 8 M guanidine hydrochloride such that its final concentration was 6 M guanidine hydrochloride, followed by centrifugation at 30,000 rpm for 5 hours, and recovery of the supernatant.

(2) Purification

(i) The supernatant obtained in 1) was subjected to gel filtration on an Ultrogel AcA54 column (4.6 cm$^\emptyset$ x 90 cm$^L$ ; LKB) at a flow rate of ca. 50 ml/hr with 0.01 M Tris-HCl buffer (pH 7.4) containing 0.15 M NaCl and 0.01% Tween 20 (Nakai Kagaku Co., Ltd.)

The fractions which showed activity upon analysis by the method of CSA assay (b) (described earlier in this specification) were selected and concentrated to a volume of ca. 5 ml with an ultrafiltration apparatus, pM-10 (Amicon).

(ii) To the concentrated fractions were added n-propanol (of the grade suitable for amino acid sequencing; Tokyo Kasei Co., Ltd.) and trifluoroacetic acid, and the mixture was worked up such that the final concentrations of n-propanol and trifluoroacetic acid were 30% and 0.1%, respectively. The worked up mixture was left in ice for about 15 minutes and centrifuged at 15,000 rpm for 10 minutes to remove the precipitate. The supernatant was adsorbed on a μ-Bondapak CI8 column (of semipreparatory grade ; Waters ; 8 mm x 30 cm) that had been equilibrated with an aqueous solution containing n-propanol (see above) and trifluoroacetic acid. The column was continuously eluted with an aqueous solution of 0.1% trifluoroacetic acid containing n-propanol with a linear density gradient of 30 - 60%. With Hitachi Model 685-50 (high performance liquid chromatographic apparatus of Hitachi, Ltd.) and Hitachi Model 638-41 (detector of Hitachi, Ltd.) being used, the adsorptions at 220 nm and 280 nm were measured simultaneously. After eluting, a 10-μl aliquot of each fraction was diluted 100-fold and the dilutions were screened for active fractions by the method of CSA assay (b). Activity was observed in the peaks that were eluted at 40% n-propanol. These peaks were combined and rechromatographed under the same conditions as used above and the fractions were checked for their activity by the method (b). Again, activity was found in the peaks for 40% n-propanol. These active peaks were collected (four fractions = 4 ml) and freeze-dried.

(iii) The freeze-dried powder was dissolved in 200 µl of an aqueous solution of 0.1% trifluoroacetic acid containing 40% n-propanol, and the solution was subjected to high performance liquid chromatography on TSK-G3000SW column (7.5 mm x 60 cm ; Toyo Soda Manufacturing Co., Ltd.). Elution was conducted at a flow rate of 0.4 ml/min with an aqueous solution of 0.1% trifluoroacetic acid containing 40%-propanol, and 0.4-ml fractions were taken with a fraction collector, FRAC-100 (Pharmacia Fine Chemicals). The fractions were checked for their CSA as described above and the active fractions were recovered. They were further purified on analytical µ-Bondapak Cl8 column (4.6 mm x 30 cm), and the main peak was recovered and freeze-dried.

The protein so obtained was treated with 2-mercaptoethanol and subjected to SDS-polyacrylamide gel (15.0%) electrophoresis (15 mV, 6 hours). Upon staining with Coomassie Blue, the desired G-CSF polypeptide could be identified as a single band.

Example 16 : Assay of G-CSF Activity (+VSE)

The CSF sample obtained in Example 15 was assayed in accordance with the method of CSF assay (a) described earlier in this specification. The results are shown in Table 1.

## Table 1

|  | Human neutrophilic colonies (colonies/dish) |
|---|---|
| Purified human G-CSF (20 ng) | 73 |
| CSF sample obtained in Example 15 (50 ng) | 68 |
| Blank | 0 |

Example 17 : Amino Acid Analysis (+VSE)

(1) Analysis of amino acid composition

The CSF sample purified in Example 15 was hydrolyzed by routine procedures, and the amino acid composition of the protein portion of the hydrolyzate was analyzed by a method of amino acid analysis with an automatic amino acid analyzer, Hitachi 835 (Hitachi Ltd.) The results are shown in Table 2. Hydrolysis was conducted under the following conditions :

(i) 6 N HCl, 110°C, 24 hours, in vacuum

(ii) 4 N methanesulfonic acid + 0.2% 3-(2-aminoethyl)indole, 110°C, 24 hours, 48 hours, 72 hours, in vacuum

The sample was dissolved in a solution (1.5 ml) containing 40% n-propanol and 0.1% trifluoroacetic acid. Aliquots each weighing 0.1 ml were dried with a dry nitrogen gas and, after addition of the reagents listed in (i) or (ii), the containers were sealed in vacuum, followed by hydrolysis of the contents.

Each of the values shown in Table 2 was the average of four measurements, 24 hour value for (i) and 24, 48 and 72 hour values for (ii), except that the contents of Thr, Ser, 1/2 Cys, Met, Val, Ile and Trp were calculated by the following methods (see "Tampaku Kagaku (Protein Chemistry) II", A Course in Biochemical Experiments, Tokyo Kagaku Dohjin) :

-- For Thr, Ser, 1/2 Cys and Met, the time-dependent profile of the 24, 48 and 72 hour values for (ii) was extrapolated by zero hours.

-- For Val and Ile, the 72 hour value for (ii) was used.

-- For Trp, the average of 24, 48 and 72 hour values for (ii) was used.

## Table 2

### (Amino Acid Analysis Data)

| Amino acids | Mole% |
|---|---|
| Asp (Asp + Asn) | 2.3 |
| Thr | 4.0 |
| Ser | 8.5 |
| Glu (Glu + Gln) | 15.2 |
| Pro | 7.3 |
| Gly | 7.9 |
| Ala | 10.7 |
| 1/2 Cys | 2.8 |
| Val | 4.5 |
| Met | 2.0 |
| Ile | 2.3 |
| Leu | 18.3 |
| Tyr | 1.7 |
| Phe | 3.4 |
| Lys | 2.3 |
| His | 2.8 |
| Trp | 1.1 |
| Arg | 2.9 |

(2) Analysis of N-terminal amino acids

The sample was subjected to Edman decomposition with a gas-phase sequencer (Applied Biosystems) and the PTH amino acid obtained was analyzed by routine procedures with a high performance liquid chromatographic apparatus (Beckman Instruments) and Ultrasphere-ODS column (Beckman Instruments). After the column (5 μm ; 4.6 mm⌀ x 250 mm) was equilibrated with a starting buffer [an aqueous solution containing 15 mM sodium acetate buffer (pH 4.5) and 40% acetonitrile], the sample (as dissolved in 20 μl of the starting buffer) was injected and separation was achieved by isocratic elution with the starting buffer. During these operations, the flow rate was held at 1.4 ml/min and the column temperature at 40°C. Detection of the PTH amino acid was accomplished using the absorptions in the ultraviolet range at 269 nm and 320 nm. Standard samples (each weighing 2 nmol) of PTH amino acid (Sigma) had been separated on the same line to determine their retention times, which were compared with those of the sample for the purpose of identification of the N-terminal amino acids. As a result, PTH-methionine and PTH-threonine were detected.

Example 18 : Construction of E. coli Recombinant Vector (-VSE) and Transformation

(1) Using tac promoter-containing vector

The procedures of Example 11 were repeated except that the "pBRG4 prepared in Example 9 which contained the cDNA shown in Fig. 3(A)" [see (iii) in Example 11] was replaced by the "pBRV2 prepared in Example 10 which contained the cDNA shown in Fig. 4(A)". As in Example 11, the transformants obtained were verified as the desired ones (Fig. 9).

(2) Using PL promoter-containing vector

The procedures of Example 12 were repeated using cDNA (-VSE) and the transformants obtained were verified as the desired ones (Fig. 10).

(3) Using trp promoter-containing vector

The procedures of Example 13 were repeated using cDNA (-VSE) and the transformants were verified as the desired ones (Fig. 11).

Example 19 : Assay of G-CSF Activity (-VSE)

The three species of transformants obtained in Example 18 were cultured by the method described in Example 14. From the cultured E. coli cells, G-CSF polypeptides were recovered and purified by the method described in Example 15, with the result that human G-CSF polypeptide was obtained as a single band.

The so obtained CSF sample was assayed by the method of CSF activity assay (a) described earlier in this specification. The results are shown in Table 3.

## Table 3

|  | Human neutrophilic colonies (colonies/dish) |
|---|---|
| Purified human G-CSF (20 ng) | 73 |
| CSF sample obtained in Example 19 (50 ng) | 73 |
| Blank | 0 |

Example 20 : Amino Acid Analysis (-VSE)

(1) Analysis of amino acid composition

The amino acid composition of the CSF sample purified in Example 19 was analyzed by the method described in 1) in Example 17. The results are shown in Table 4.

## Table 4

### (Amino Acid Analysis Data)

| Amino acids | Mole% |
|---|---|
| Asp (Asp + Asn) | 2.3 |
| Thr | 4.0 |
| Ser | 8.1 |
| Glu (Glu + Gln) | 15.0 |
| Pro | 7.5 |
| Gly | 8.1 |
| Ala | 11.0 |
| 1/2 Cys | 2.9 |
| Val | 4.1 |
| Met | 2.0 |
| Ile | 2.2 |
| Leu | 18.8 |
| Tyr | 1.7 |
| Phe | 3.4 |
| Lys | 2.3 |
| His | 2.7 |
| Trp | 1.1 |
| Arg | 2.8 |

(2) Analysis of N-terminal amino acids

The sample was subjected to analysis of the N-terminal amino acids in accordance with the method described in 2) in Example 17. As a result, PTH-methionine and PTH-threonine were detected.

Example 21 : Preparation of pHGA410 Vector (for Use with Animal Cells, +VSE Line)

The EcoRI fragment prepared in Example 9 which had the cDNA shown in Fig. 3(A) was treated with a restriction enzyme, Dral, at 37°C for 2 hours, followed by treatment with the Klenow fragment of DNA polymerase I (Takara Shuzo Co., Ltd.) to create blunt ends. One microgram of BglII linker (8mer, Takara Shuzo Co., Ltd.) was phosphorylated with ATP and joined to about 1 μg of the separately obtained mixture of DNA fragments. The joined fragments were treated with a restriction enzyme, BglII, and subjected to agarose gel electrophoresis. Subsequently, only the largest DNA fragment was recovered.

This DNA fragment was equivalent to about 710 base pairs containing a human G-CSF polypeptide coding portion (see Fig. 5). A vector pdKCR [Fukunaga et al., Proc. Natl. Acad. Sci., USA, 81, 5086 (1984)] was treated with a restriction enzyme, BamHI, and subsequently dephosphorylated with an alkali phosphatase (Takara Shuzo Co., Ltd.) The vector DNA obtained was joined to the 710-bp cDNA fragment in the presence of $T_4$ DNA ligase (Takara Shuzo Co., Ltd.), so as to produce pHGA410 (Fig. 12). As shown in Fig. 12, this plasmid contained the promoter of SV40 early gene, the replication origin of SV40, part of the rabbit β-globin gene, the replication origin of pBR322 and the pBR322-derived β-lactamase gene (Amp$^r$), with the human G-CSF gene being connected downstream of the promoter of the SV40 early gene.

Example 22 : Construction of Recombinant Vector (+VSE) for Use in Transformation of Cl27 Cells

(1) Construction of pHGA410 (H)

Twenty micrograms of the plasmid pHGA410 (Fig. 12) prepared in Example 21 was dissolved in a reaction solution composed of 50 mM Tris-HCl (pH 7.5), 7 mM $MgCl_2$, 100 mM NaCl, 7 mM 2-mercaptoethanol and 0.01% bovine serum alubmin (BSA). A restriction enzyme, EcoRI (10 - 15 units ; Takara Shuzo Co., Ltd.) was added and the reaction solution was held at 37°C for about 30 minutes to cause partial digestion with EcoRI. Subsequently, the DNA fragment was subjected to two treatments with a 1 :1 mixture of phenol/chloroform, one treatment with ether, and precipitation with ethanol.

The DNA fragment obtained was dissolved in 50 μl of a solution composed of 50 mM Tris-HCl, 5 mM $MgCl_2$, 10 mM DTT, and 1 mM each of dATP, dCTP, dGTP and dTTP. After 5 μl of the Klenow fragment of E. coli DNA polymerase (Takara Shuzo Co., Ltd.) was added, the solution was incubated at 14°C for 2 hours to produce blunt ends.

By subsequent 0.8% agarose gel electrophoresis, 6 μg of a DNA fragment of about 5.8 kb in length was recovered.

Five micrograms of the recovered DNA fragment was re-dissolved in 50 μl of a reaction solution composed of 50 mM Tris-HCl (pH 7.6), 10 mM $MgCl_2$, 10 mM DTT and 1 mM ATP. After 2 μg of HindIII linker (Takara Shuzo Co., Ltd.) and 100 units of T4 DNA ligase (Takara Shuzo Co., Ltd.) were added, reaction was carried out overnight at 4°C.

Subsequently, treatments with phenol and ether and precipitation with ethanol were conducted. The precipitate was dissolved in 30 μl of a solution composed of 10 mM Tris-HCl (pH 7.5), 7 mM $MgCl_2$ and 60 mM NaCl, and the solution was incubated at 37°C for 3 hours in the presence of 10 units of HindIII. After re-treatment with $T_4$ DNA ligase, the resulting DNA was used to transform E. coli strain DHl by the rubidium chloride procedure (see Molecular Cloning, ibid.) From ampicillin-resistant (Amp$^r$) colonies of the transformants, cells were selected which harbored a plasmid which was identical to pHGA410 except that HindIII was inserted at the EcoRI site. The so obtained plasmid was named pHGA410 (H) (Fig. 13).

(2) Construction of expression recombinant vector pTN-G4

Twenty micrograms of the pHGA410 (H) thus obtained was dissolved in 50 μl of a reaction solution composed of 10 mM Tris-HCl (pH 7.5), 7 mM $MgCl_2$, 175 mM NaCl, 0.2 mM EDTA, 7 mM 2-mercaptoethanol and 0.01% bovine serum albumin. After 20 units of SalI (Takara Shuzo Co., Ltd.) were added, the reaction solution was incubated at 37°C for 5 hours. Following treatment with phenol and precipitation with ethanol, incubation was conducted as in 1) for about 2 hours at 14°C in the presence of the Klenow fragment of DNA polymerase (Takara Shuzo Co., Ltd.), so as to create blunt ends. Without being subjected to DNA recovery by agarose gel electrophoresis, the reaction solution was immediately subjected to precipitation with ethanol. The resulting DNA fragment was treated with HindIII and 5 μg of a HindIII-SalI fragment (ca. 2.7 kb) was recovered by 1% agarose gel electrophoresis. In a separate step, a plasmid pdBPV-I having a bovine papilloma virus (BPV) [this plasmid was obtained by courtesy of Dr. Howley and is described in Sarver, N, Sbyrne, J.C. & Howley, P.M., Proc. Natl. Acad. Sci., USA, 79, 7147-7151 (1982)] was treated with HindIII and PvuII, as described by Nagata et al. [Fukunaga, Sokawa and Nagata, Proc. Natl. Acad. Sci., USA, 81, 5086-5090 (1984)], to obtain an 8.4-kb DNA fragment. This 8.4-kb DNA fragment and the separately obtained HindIII-SalI DNA fragment (ca. 2.7 kb) were ligated by $T_4$ DNA ligase. The ligation product was used to transform E. coli strain DHI by the rubidium chloride procedure described in Molecular Cloning, ibid. E. coli colonies harboring a plasmid having the pHGA410-derived G-CSF cDNA were selected. This plasmid was named pTN-G4 (Fig. 13).

Example 23 : Transformation of Cl27 Cells and G-CSF Expression Therein (+VSE)

Before it was used to transform mouse Cl27 cells, the pTN-G4 obtained in Example 22 was treated with a restriction enzyme, BamHI. Twenty micrograms of the plasmid pTN-G4 was dissolved in 100 µl of a reaction solution [10 mM Tris-HCl (pH 8.0), 7 mM MgCl₂, 100 mM NaCl, 2 mM 2-mercaptoethanol and 0.01% BSA] and treated with 20 units of BamHI (Takara Shuzo Co., Ltd.), followed by treatments with phenol and ether, and precipitation with ethanol.

Mouse Cl27I cells were grown in a Dulbecco's minimal essential medium containing 10% bovine fetal serum (Gibco). The Cl27I cells growing on plates (5 cm²) were transformed with 10 µg, per plate, of the separately prepared DNA by the calcium phosphate procedure [see Haynes, J. & Weissmann, C., Nucleic Acids Res., 11, 687-706 (1983)]. After treatment with glycerol, the cells were incubated at 37°C for 12 hours.

The incubated cells were transferred onto three fresh plates (5 cm²) and the media were changed twice a week. At day 16, the foci were transferred onto fresh plates and subjected to serial cultivation on a Dulbecco's minimal essential medium containing 10% bovine fetal serum (Gibco), so as to select clones having high G-CSF production rate. These clones produced G-CSF at a level of approximately 1 mg/L. In addition to the Cl27I cells, NIH3T3 cells could also be used as host cells.

## Example 24 : Expression of G-CSF in CHO Cells (+VSE)

### (1) Construction of pHGG4-dhfr

Twenty micrograms of the plasmid pHGA410 obtained in Example 21 was dissolved in 100 µl of a reaction solution containing 10 mM Tris-HCl (pH 7.5), 7 mM MgCl₂, 175 mM NaCl, 0.2 mM EDTA, 0.7 mM 2-mercaptoethanol and 0.01% BSA. Reaction was carried out overnight at 37°C in the present of 20 units of a restriction enzyme Sall (Takara Shuzo Co., Ltd.), followed by treatments with phenol and ether and precipitation with ethanol.

The precipitate of DNA was dissolved in 100 µl of a reaction solution composed of 50 mM Tris-HCl, 5 mM MgCl₂, 10 mM DTT, and 1 mM each of dATP, dCTP, dGTP and dTTP, and reaction was carried out at 14°C for 2 hours in the presence of the Klenow fragment of E. coli DNA polymerase (10 µl ; Takara Shuzo Co., Ltd.), followed by treatments with phenol and ether, and precipitation with ethanol.

An EcoRI linker was attached to the DNA in the precipitate by the following procedures : the DNA was dissolved in 50 µl of a reaction solution composed of 50 mM Tris-HCl (pH 7.4), 10 mM DTT, 0.5 mM spermidine, 2 mM ATP, 2 mM hexamine-cobalt chloride and 20 µg/ml of BSA. Reaction was carried out at 4°C for 12 - 16 hours in the presence of EcoRI linker (Takara Shuzo Co., Ltd.) and 200 units of T₄ DNA ligase (Takara Shuzo Co., Ltd.) After treatment with phenol, washing with ether and precipitation with ethanol, all being conducted in accordance with routine procedures, the DNA precipitate was partially digested with EcoRI and 3 µg of a DNA fragment of about 2.7 kb in length was recovered by 1% agarose gel electrophoresis.

The plasmid pAdD26SVpA [Kaufman, R.G. & Sharp, P.A., Mol. Cell Biol., 2, 1304-1319 (1982)] was treated with EcoRI and dephosphorylated by treatment with a bacterial alkaline phosphatase (BAP). More specifically, 20 µg of pAdD26SVpA and 20 units of EcoRI were added to a reaction solution [50 mM Tris-HCl (pH 7.5), 7 mM MgCl₂, 100 mM NaCl, 7 mM 2-mercaptoethanol and 0.01% BSA] and reaction was carried out at 37°C for 10 hours. Subsequently, 5 units of BAP was added to the reaction solution, and reaction was carried out at 68°C for 30 minutes. Following treatment with phenol, the EcoRI fragment of pAdD26SVpA was recovered by electrophoresis in a yield of approximately 5 µg.

The fragment of about 2.7 kb in length and the pAdD26SVpA, each weighing 0.5 µg, were annealed. The resulting plasmid was used to transform E. coli strain DHI by the rubidium chloride procedure, and the colonies harboring the plasmid of pHGG4-dhfr were selected. The obtained plasmid was named pHGG4-dhfr (Fig. 14a).

The alternative procedure was as follows : the plasmid pHGG4 was treated with Sall and partially digested with EcoRI without any EcoRI linker being attached. A DNA fragment of about 2.7 kb in length was recovered and treated with the Rlenow fragment of E. coli DNA polymerase to create blunt ends. An EcoRI fragment having blunt ends was prepared from pAdD26SVpA as described above. This EcoRI fragment and the separately prepared fragment (ca. 2.7 kb) were treated with T₄ DNA ligase to prepare pHGG4-dhfr.

The pHGA410 (H) prepared in Example 22 was treated with restriction enzymes, HindIII and Sall, as described in 2) in Example 22, and the HindIII-Sall fragment was joined to the blunt-ended EcoRI fragment of pAdD26SVpA described above. This method could also be employed to prepare pHGG4-dhfr (Fig. 14b).

### (2) Transformation and expression

CHO cells (dhfr⁻ strain ; courtesy of Dr. L. Chasin of Columbia University) were cultivated for growth in alpha-minimal essential medium containing 10% calf serum (α-MEN supplemented with adenosine, deoxyadenosine and thymidine) in plates (9 cm², Nunc). The cultured cells were transformed by the calcium phosphate procedure [Wigler et al., Cell, 14, 725 (1978)] in the following manner.

A carrier DNA (calf thymus DNA) was added in an appropriate amount to 1 μg of the plasmid pHGG4-dhfr prepared in 1), and the mixture was dissolved in 375 μl of a TE solution, followed by addition of 125 μl of 1 M CaCl₂. After the solution was cooled on ice for 3 - 5 minutes, 500 μl of 2 x HBS (50 mM Hepes, 280 mM NaCl, and 1.5 mM phosphate buffer) was added to the solution. After recooling on ice, the solution was mixed with 1 ml of the culture of CHO cells, transferred onto plates, and incubated for 9 hours in a CO₂ incubator.

Following washing, addition of 20% glycerol-containing TBS (Tris-buffered saline), and re-washing, a non-selective medium (the α-MEN medium described above except that it was supplemented with nucleotides) was added. After 2-day incubation, a 10-fold dilution of the culture was transferred onto a selective medium (not supplemented with nucleotides). The cultivation was continued, with the medium being replaced by a fresh selective medium every 2 days, and the resulting foci were selected and transferred onto fresh plates, where the cells grew in the presence of 0.02 μM methotrexate (MTX), followed by cloning through growth in the presence of 0.1 μM MTX.

The transformation of CHO cells may also be accomplished by cotransformation with pHGG4 and pAdD26SVpA [see Scahill et al., Proc. Natl. Acad. Sci., USA, 80, 4654-4658 (1983)].

A recombinant vector that is constructed by a method that uses a "polycistronic gene" may also be used to transform CHO cells. An example of this alternative method is as follows : pAdD26SVpA was treated with PstI and the recovered two fragments were joined to a pBRG4-derived CSF cDNA fragment so as to construct a recombinant vector wherein the adeno virus promoter, CSF cDNA, DHFR and the poly(A) site of SV40 were inserted in the order written. This recombinant vector was used to transform CHO cells.

Example 25 : Assay of G-CSF Activity (+VSE)

The supernatants of cultures of Cl27 cells and CHO cells which were obtained in Examples 23 and 24, respectively, were adjusted to a pH of 4 with 1 N acetic acid. After addition of an equal volume of n-propanol, the resulting precipitate was removed by centrifugation. The supernatant was passed through an open column (1∅ x 2 cmᴸ) filled with a C8 reverse-phase carrier (Yamamura Kagaku K.K.) and elution was conducted with 50% n-propanol. The elute was diluted two-fold with water and subjected to reverse-phase high performance liquid chromatography on YMC-C8 column (Yamamura Kagaku K.K.), followed by elution with n-propanol (30 - 60% linear density gradient) containing 0.1% TFA. The fractions which were eluted at n-propanol concentrations of about 40% were recovered, freeze-dried and dissolved in 0.1 M glycine buffer (pH 9). As a result of these procedures, the human G-CSF in the Cl27 and CHO cells was concentrated about 20-fold.

As controls, cells were transformed with human G-CSF cDNA-free plasmids and the supernatants of their cultures were concentrated in accordance with the procedures described above. The human G-CSF activities of the samples were assayed by the method of human G-CSF activity assay (a) described earlier in this specification. If the efficiency of expression is adequately high, the supernatants of cultures may be directly assayed without being concentrated. The results are summarized in Table 5, wherein the data are based on concentrated samples.

## Table 5

### Assay of Human G-CSF Activity

| | | Human neutrophilic colonies (colonies/dish) |
|---|---|---|
| Purified human G-CSF (20 ng) | | 96 |
| BPV | Culture of C127 cells transformed with pdBPV-1 (concentrated 20-fold) | 0 |
| | Culture of 3T3 cells transformed with pdBPV-1 (concentrated 20-fold) | 0 |
| | Culture of C127 cells transformed with pTNG4 (concentrated 20-fold) | 82 |
| | Culture of 3T3 cells transformed with pTNG4 (concentrated 20-fold) | 85 |
| dhfr | Culture of CHO cells transformed with pAdD26SVpA (concentrated 20-fold) | 0 |
| | Culture of CHO cells transformed with pHGG4-dhfr (concentrated 20-fold) | 110 |

Example 26 : Amino Acid Analysis and Sugar Analysis (+VSE)

(1) Analysis of amino acid composition

The crude CSF sample prepared in Example 25 was purified in accordance with the procedures described in Example 2(iii). The purified CSF sample was hydrolyzed by routine procedures, and the protein portion of the hydrolyzate was checked for its amino acid composition by a special method of amino acid analysis with Hitachi 835 automatic amino acid analyzer (Hitachi, Ltd.) The results are shown in Table 6. Hydrolysis was conducted under the following conditions :
(i) 6 N HCl, 110°C, 24 hours, in vacuum
(ii) 4 N methanesulfonic acid + 0.2% 3-(2-aminoethyl)indole, 110°C, 24 hours, 48 hours, 72 hours, in vacuum.

The sample was dissolved in a solution (1.5 ml) containing 40% n-propanol and 0.1% trifluoroacetic acid. Aliquots each weighing 0.1 ml were dried with a dry nitrogen gas and, after addition of the reagents listed in (i) or (ii), the containers were sealed in vacuum, followed by hydrolysis of the contents.

Each of the values shown in Table 6 was the average of four measurements, 24 hour value for (i) and 24, 48 and 72 hour values for (ii), except that the contents of Thr, Ser, 1/2 Cys, Met, Val, Ile and Trp were calculated by the following methods (see "Tampaku Kagaku (Protein Chemistry) II", A Course in Biochemical Experiments, Tokyo Kagaku Dohjin) :
-- For Thr, Ser, 1/2 Cys and Met, the time-dependent profile of the 24, 48 and 72 hour values for (ii) were extrapolated for zero hours.
-- For Val and Ile, the 72 hour value for (ii) was used.
-- For Trp, the average of 24, 48 and 72 hour values for (ii) was used.

## Table 6

### Amino Acid Analysis Data

| Amino acids | Mole% |
| --- | --- |
| Asp (Asp + Asn) | 2.3 |
| Thr | 3.9 |
| Ser | 8.5 |
| Glu (Glu + Gln) | 15.3 |
| Pro | 7.4 |
| Gly | 7.8 |
| Ala | 10.8 |
| 1/2 Cys | 2.8 |
| Val | 4.5 |
| Met | 1.7 |
| Ile | 2.3 |
| Leu | 18.6 |
| Tyr | 1.7 |
| Phe | 3.4 |
| Lys | 2.3 |
| His | 2.8 |
| Trp | 1.1 |
| Arg | 2.8 |

(2) Sugar composition analysis

An internal standard (25 nmol of inositol) was added to 200 ng of the purified CSF sample used in the analysis of amino acid composition 1). After addition of a methanol solution (500 µl) containing 1.5 N HCl, reaction was carried out at 90°C for 4 hours in a $N_2$ purged, closed tube. After the tube was opened, silver carbonate ($Ag_2CO_3$) was added to neutralize the contents. Thereafter, 50 µl of acetic anhydride was added and the tube was shaken for an adequate period. Subsequently, the tube was left overnight in the dark at room temperature. The upper layer was put into a sample tube and dried with a nitrogen gas. Methanol was added to the precipitate and the mixture was washed and lightly centrifuged. The upper layer was put into the same sample tube and dried. After addition of 50 µl of a TMS reagent (5 :1 :1 mixture of pyridine, hexamethyl disilazane and trimethylchlorosilane), reaction was carried out at 40°C for 20 minutes and the reaction product was stored in a deep freezer. A standard was prepared by combining 25 nmol of inositol with 50 nmol each of galactose (Gal), N-acetyl galactosamine (Gal NAc), sialic acid and any other appropriate reagents.

The samples thus prepared were subjected to gas chromatographic analysis under the following condi-

tions :

Conditions of analysis

```
Column :         2% OV - 17 VINport HP, 60 - 80 mesh, 3 m,
                 glass
Temperature : elevated from 110 to 250°C at 4°C/min.
Carrier gas (N₂) pressure : initially 1.2 - 1.6 kg/cm²
                                   finally 2 - 2.5 kg/cm²
Sensitivity : 10³ MΩ range, 0.1 - 0.4 volts
Pressure :       H₂, 0.8 kg/cm²
                 air, 0.8 kg/cm²
Sample feed : 2.5 - 3.0 μl.
         As a result of the analysis, galactose, N-acetyl
galactosamine and sialic acid were identified in the CSF
sample of the present invention.
```

As a result of the analysis, galactose, N-acetyl galactosamine and sialic acid were identified in the CSF sample of the present invention.

Example 27 : Preparation of pHGV2 Vector (for Use with Animal Cells, -VSE line)

The EcoRI fragment prepared in Example 10 which had the cDNA shown in Fig. 4(A) was treated with a restriction enzyme, DraI, at 37°C for 2 hours, followed by treatment with the Klenow fragment of DNA polymerase I (Takara Shuzo Co., Ltd.) to create blunt ends. One microgram of BglII linker (8mer, Takara Shuzo Co., Ltd.) was phosphorylated with ATP and joined to about 1 μg of the separately obtained mixture of DNA fragments. The joined fragments were treated with a restriction enzyme, BglII, and subjected to agarose gel electrophoresis. Subsequently, only the largest DNA fragment was recovered.

This DNA fragment was equivalent to about 710 base pairs containing a human G-CSF polypeptide coding portion (see Fig. 5). A vector pdKCR [Fukunaga et al., Proc. Natl. Acad. Sci., USA, 81, 5086 (1984)] was treated with a restriction enzyme, BamHI, and subsequently dephosphorylated with an alkali phosphatase (Takara Shuzo Co,, Ltd.). The vector DNA obtained was joined to the 710-bp cDNA fragment in the presence of $T_4$ DNA ligase (Takara Shuzo Co., Ltd.), so as to produce pHGV2 (Fig. 15). As shown in Fig. 15, this plasmid contained the promoter of SV40 early gene, the replication origin of SV40, part of the rabbit β-globin gene, the replication origin of pBR322 and the pBR322-derived β-lactamase gene (Ampʳ), with the human G-CSF gene being connected downstream of the promoter of the SV40 early gene.

Example 28 : Construction of Recombinant Vector (-VSE) for Use in Transformation of Cl27 Cells

(1) Construction of pHGV2(H)

Twenty micrograms of the plasmid pHGV2 (Fig. 15) prepared in Example 27 was treated by the procedures described in 1) in Example 22, so as to prepare a plasmid named pHGV2(H).

(2) Construction of expression recombinant vector pTN-V2

With 20 μg of the pHGV2(H) being used, the procedures described in 2) in Example 22 were repeated to select E. coli harboring a plasmid having the pHGV2-derived G-CSF cDNA. This plasmid was named pTN-V2 (Fig. 16).

Example 29 : Transformation of Cl27 Cells and G-CSF Expression Therein (-VSE)

The pTN-V2 obtained in Example 28 was treated with a restriction enzyme, BamHI, before it was used to transform mouse Cl27 cells.

Mouse Cl27I cells were transformed with the so prepared DNA to express G-CSF (see Example 23) and clones having high G-CSF production rate were selected. These clones produced G-CSF at a level of approximately 1 mg/L. In addition to the Cl27I cells, NIH3T3 cells could also be used as host cells.

Example 30 : Expression of G-CSF in CHO Cells (-VSE)

(1) Construction of pHGV2-dhfr

A DNA fragment of about 2.7 kb in length was prepared from 20 μg of the plasmid pHGV2 (Example 27) by the procedures described in 1) in Example 24. This fragment (0.5 μg) and the EcoRI fragment of pAdD26SVpA (0.5 μg) were annealed. The resulting plasmid was used to transform E. coli strain DHI by the rubidium chloride procedure, and the colonies harboring the plasmid of pHGV2-dhfr were selected. The obtained plasmid was named pHGV2-dhfr (Fig. 17a).

The alternative procedure was as follows : the plasmid pHGV2 was treated with SalI and partially digested with EcoRI without any EcoRI linker being attached. A DNA fragment of about 2.7 kb in length was recovered and treated with the Klenow fragment of E. coli DNA polymerase to create blunt ends. A blunt-ended EcoRI fragment was prepared from pAdD26SVpA as described above. This EcoRI fragment and the separately prepared fragment (ca. 2.7 kb) were treated with $T_4$ DNA ligase to prepare pHGV2-dhfr.

The pHGV2 (H) prepared in Example 28 was treated with restriction enzymes, HindIII and SalI, as described in 2) in Example 28, and the HindIII-SalI fragment was joined to the blunt-ended EcoRI fragment of pAdD26SVpA described above. This method could also be employed to prepare pHGG4-dhfr (Fig. 17b).

(2) Transformation and expression

CHO cells were transformed with the plasmid pHGV2-dhfr for G-CSF expression in accordance with the procedures described in 2) in Example 24.

The transformation of CHO cells may also be accomplished by cotransformation with pHGV2 and pAdD26SVpA [see Scahill et al., Proc. Natl. Acad. Sci., USA, 80, 4654-4658 (1983)].

A recombinant vector that is constructed by a method that uses a "polycistronic gene" may also be used to transform CHO cells. An example of this alternative method is as follows : pAdD26SVpA was treated with PstI and the recovered two fragments were joined to a pBRV2-derived CSF cDNA fragment so as to construct a recombinant vector wherein the adeno virus promoter, CSF cDNA, DHFR and the poly(A) site of SV40 were inserted in the order written. This recombinant vector was used to transform CHO cells.

Example 31 : Assay of G-CSF Activity (-VSE)

By the procedures described in Example 25, human G-CSF was obtained from the supernatants of cultures of Cl27 cells and CHO cells which were obtained in Examples 29 and 30, respectively. The human G-CSF activity of each of the recovered samples was assayed as in Example 25. The results are shown in Table 7.

## Table 7

### Assay of Human G-CSF Activity

| | | | Human neutrophilic colonies (colonies/dish) |
|---|---|---|---|
| Purified human G-CSF (20 ng) | | | 96 |
| BPV | Culture of C127 cells transformed with pdBPV-1 (concentrated 20-fold) | | 0 |
| | Culture of 3T3 cells transformed with pdBPV-1 (concentrated 20-fold) | | 0 |
| | Culture of C127 cells transformed with pTN-V2 (concentrated 20-fold) | | 107 |
| | Culture of 3T3 cells transformed with pTN-V2 (concentrated 20-fold) | | 103 |
| dhfr | Culture of CHO cells transformed with pAdD26SVpA (concentrated 20-fold) | | 0 |
| | Culture of CHO cells transformed with pHGV2-dhfr (concentrated 20-fold) | | 111 |

Example 32 : Amino Acid Analysis and Sugar Analysis (-VSE)

(1) Analysis of amino acid composition

The crude CSF sample prepared in Example 31 was purified in accordance with the procedures described in Example 2(iii). The purified CSF sample was subjected to analysis of amino acid composition by the procedures described in 1) in Example 26. The results are shown in Table 8.

## Table 8
### Amino Acid Analysis Data

| Amino acids | Mole% |
|---|---|
| Asp (Asp + Asn) | 2.3 |
| Thr | 4.0 |
| Ser | 8.1 |
| Glu (Glu + Gln) | 15.1 |
| Pro | 7.5 |
| Gly | 8.0 |
| Ala | 10.9 |
| 1/2 Cys | 2.8 |
| Val | 3.9 |
| Met | 1.7 |
| Ile | 2.3 |
| Leu | 18.9 |
| Tyr | 1.7 |
| Phe | 3.5 |
| Lys | 2.3 |
| His | 2.9 |
| Trp | 1.2 |
| Arg | 2.9 |

(2) Analysis of sugar composition

The purified CSF sample used in the analysis of amino acid composition in 1) was also subjected to analysis of its sugar composition by the same procedures and under the same conditions as those described in 2) in Example 26. As a result of this analysis, the presence of galactose, N-acetyl galactosamine and sialic acid in the CSF sample of the present invention was verified.

Example 33 : Protective Effect of Human G-CSF against Microbial Infection

Test Method

1. Protection against infection with Pseudomonas aeruginosa

Endoxan (trade name of Shionogi & Co., Ltd.) was administered intraperioneally into 8-9-wk-old ICR mice (male ; 35.3 ± 1.38 g in body weight) in a dose of 200 mg/kg. The mice were then divided into three groups ; two groups were given four subcutaneous injections (each 0.1-ml dose), at 24-hr intervals, of a solvent [1% propanol and 0.5% (w/v) mouse serum albumin in physiological saline] containing human G-CSF (25,000 or 50,000 units/mouse), whereas the other group was given only the solvent in accordance with the same schedule. Three hours after the last injection, the mice in each group were infected with <u>Pseudomonas</u> <u>aeruginosa</u> GNB-139 by subcutaneous injection (3.9 x $10^5$ CFU/mouse). Twenty-one hours after the infection, the first two groups were given another subcutaneous injection of the solvent containing human G-CSF (25,000 or 50,000 units/mouse) and the other group given the solvent only.

The protective effect of human G-CSF was checked by counting the number of mice which were alive ten days after the infection.

### Preparation of cell suspension

<u>Pseudomonas</u> <u>aeruginosa</u> GNB-139 was cultured overnight with shaking at 37°C in a Heart Infusion liquid medium (trade name of Difco). The culture was suspended in a physiological saline solution.

### 2. Protection against infection with Candida

Endoxan (trade name of Shionogi & Co., Ltd.) was administered intraperitoneally into 8-wk-old ICR mice (male ; 40.5 ± 1.60 g in body weight) in a dose of 200 mg/kg. The mice were then divided into two groups ; one group was given four subcutaneous injections (each 0.1-ml dose), at 24-hr intervals, of a solvent [1% propanol and 10% (w/v) ICR mouse serum in physiological saline] containing human G-CSF (50,000 units/mouse), whereas the other group was given only the solvent in accordance with the same schedule. Four hours after the last injection, the mice in each group were infected with <u>Candida</u> <u>albicans</u> U-50-1 (strain isolated from urine of leukemic patients ; courtesy by Bacteriological Laboratory, Tohoku University, School of Medicine) by intravenous injection (5.6 x $10^5$ CFU/mouse). The protective effect of human G-CSF was checked by counting the number of mice which were alive ten days after the infection.

### Preparation of cell suspension

<u>Candida</u> <u>albicans</u> U-50-1 was cultured overnight with shaking at 37°C in a yeast extract-containing Sabouraud liquid medium (2% dextrose from Junsei Pure Chemicals Co., Ltd. ; 10% Tryptocase Peptone, trade name of BBL ; 5% yeast extract from Difco ; pH, 5.6). The culture was washed twice with physiological saline and suspended in physiological saline.

### 3. Protection against infection with intracellular parasitic Listeria

Endotoxan (trade name of Shionogi & Co., Ltd.) was administered intraperitoneally to 7-wk-old ICR mice (male ; 34.7 ± 1.24 g in body weight) in a dose of 200 mg/kg. The mice were then divided into two groups ; one group was given four subcutaneous injections (each 0.1-ml dose), at 24-hr intervals, of a solvent [1% n-propanol and 10% (w/v) ICR mouse serum in physiological saline] containing human G-CSF (50,000 units/mouse) while the other group was given only the solvent in accordance with the same schedule. Four hours after the last injection, the mice in each group were infected with <u>Listeria</u> <u>monocytogenes</u> 46 (by courtesy of Microbiological Laboratory, Tohoku University, School of Medicine) by intravenous injection of 1.0 x $10^7$ CFU/mouse. The protective effect of human G-CSF was checked by counting the number of mice which were alive 12 days after the infection.

### Preparation of cell suspension

<u>Listeria</u> <u>monocytogenes</u> 46 was cultured overnight with shaking at 37°C in a Brain-Heart Infusion liquid medium (trade name of Difco). The culture was suspended in physiological saline.

### Results

(1) Protective effect of native human G-CSF

Tests 1, 2 and 3 were conducted with the human G-CSF that was obtained from CHU-1 and which was described in Referential Example 2. The results are shown in Tables 9, 10 and 11.

## Table 9
### Effect against Pseudomonas aeruginosa

| Group | CSF concentration (units/mouse/day) | Live mice/ mice tested |
|---|---|---|
| Solvent | 0 | 0/11 |
| CSF-containing solvent | 25,000 | 6/10 |
| CSF-containing solvent | 50,000 | 8/11 |

## Table 10
### Effect against Candida albicans

| Group | CSF concentration (units/mouse/day) | Live mice/ mice tested |
|---|---|---|
| Solvent | 0 | 0/10 |
| CSF-containing solvent | 50,000 | 10/10 |

## Table 11
### Effect against Listeria monocytogenes

| Group | CSF concentration (units/mouse/day) | Live mice/ mice tested |
|---|---|---|
| Solvent | 0 | 0/10 |
| CSF-containing solvent | 50,000 | 10/10 |

(2) Protective effect of human G-CSF obtained from recombinant transformant

i) Tests 1, 2 and 3 were conducted with the E. coli G-CSF (+VSE) polypeptide obtained in Example 15. The results are shown in Tables 12, 13 and 14.

## Table 12
### Effect against Pseudomonas aeruginosa

| Group | CSF concentration (units/mouse/day) | Live mice/ mice tested |
|---|---|---|
| Solvent | 0 | 0/10 |
| CSF-containing solvent | 25,000 | 6/10 |
| CSF-containing solvent | 50,000 | 8/10 |

## Table 13
### Effect against Candida albicans

| Group | CSF concentration (units/mouse/day) | Live mice/ mice tested |
|---|---|---|
| Solvent | 0 | 0/10 |
| CSF-containing solvent | 50,000 | 10/10 |

## Table 14
### Effect against Listeria monocytogenes

| Group | CSF concentration (units/mouse/day) | Live mice/ mice tested |
|---|---|---|
| Solvent | 0 | 0/10 |
| CSF-containing solvent | 50,000 | 10/10 |

ii) Test 1 was conducted with the E. coli G-CSF (-VSE) polypeptide obtained in Example 19. The results are shown in Table 15.

## Table 15
### Effect against Pseudomonas aeruginosa

| Group | CSF concentration (units/mouse/day) | Live mice/ mice tested |
|---|---|---|
| Solvent | 0 | 0/10 |
| CSF-containing solvent | 25,000 | 6/10 |
| CSF-containing solvent | 50,000 | 8/10 |

iii) Test 1 was conducted with a CHO cell derived, purified human G-CSF sample (+VSE) that was the same as what was used in the analysis of amino acid composition in Example 26. The results are shown in Table 16.

### Table 16
### Effect against Pseudomonas aeruginosa

| Group | CSF concentration (units/mouse/day) | Live mice/ mice tested |
|---|---|---|
| Solvent | 0 | 0/10 |
| CSF-containing solvent | 25,000 | 9/10 |
| CSF-containing solvent | 50,000 | 10/10 |

Substantially the same results were attained when Test 1 was conducted with a Cl27 cell derived, purified human G-CSF sample which was the same as what was used in the analysis of amino acid composition in Example 26.

iv) Test 1 was conducted with a CHO cell derived, purified human G-CSF sample (-VSE) which was the same as what was used in the analysis of amino acid composition in Example 32. The results are shown in Table 17.

### Table 17
### Effect against Pseudomonas aeruginosa

| Group | CSF concentration (units/mouse/day) | Live mice/ mice tested |
|---|---|---|
| Solvent | 0 | 0/10 |
| CSF-containing solvent | 25,000 | 9/10 |
| CSF-containing solvent | 50,000 | 10/10 |

Substantially the same results were attained when Test 1 was conducted with a Cl27 cell derived, purified human G-CSF sample which was the same as what was used in the analysis of amino acid composition in Example 32.

### Example 34

The freeze-dried authentic sample of human G-CSF prepared in Example 2 was dissolved in a solution for injection and divided into injections each containing a desired dosage unit.

### Example 35

The freeze-dried authentic sample of E. coli human G-CSF polypeptide prepared in Example 15 was dissolved in a solution for injection and divided into injections each containing a desired dosage unit.

## Claims

### Claims for the following Contracting States : BE, CH, LI, DE, FR, GB, IT, NL, SE

1. A cDNA sequence coding for the following amino acid sequence :

```
-30
Met  Ala  Gly  Pro  Ala  Thr  Gln  Ser  Pro  Met  Lys
Leu  Met  Ala  Leu  Gln  Leu  Leu  Leu  Trp  His  Ser
                                        -1   +1
Ala  Leu  Trp  Thr  Val  Gln  Glu  Ala  Thr  Pro  Leu
Gly  Pro  Ala  Ser  Ser  Leu  Pro  Gln  Ser  Phe  Leu
Leu  Lys  Cys  Leu  Glu  Gln  Val  Arg  Lys  Ile  Gln
Gly  Asp  Gly  Ala  Ala  Leu  Gln  Glu  Lys  Leu  (Val
Ser  Glu)  Cys  Ala  Thr  Tyr  Lys  Leu  Cys  His  Pro
        m
Glu  Glu  Leu  Val  Leu  Leu  Gly  His  Ser  Leu  Gly
Ile  Pro  Trp  Ala  Pro  Leu  Ser  Ser  Cys  Pro  Ser
Gln  Ala  Leu  Gln  Leu  Ala  Gly  Cys  Leu  Ser  Gln
Leu  His  Ser  Gly  Leu  Phe  Leu  Tyr  Gln  Gly  Leu
Leu  Gln  Ala  Leu  Glu  Gly  Ile  Ser  Pro  Glu  Leu
Gly  Pro  Thr  Leu  Asp  Thr  Leu  Gln  Leu  Asp  Val
Ala  Asp  Phe  Ala  Thr  Thr  Ile  Trp  Gln  Gln  Met
Glu  Glu  Leu  Gly  Met  Ala  Pro  Ala  Leu  Gln  Pro
Thr  Gln  Gly  Ala  Met  Pro  Ala  Phe  Ala  Ser  Ala
Phe  Gln  Arg  Arg  Ala  Gly  Gly  Val  Leu  Val  Ala
Ser  His  Leu  Gln  Ser  Phe  Leu  Glu  Val  Ser  Tyr
                                        +174
Arg  Val  Leu  Arg  His  Leu  Ala  Gln  Pro
```

(where m is 0 or 1).

2. The cDNA sequence according to claim 1 having the following nucleotide sequence :

```
ATG  GCT  GGA  CCT  GCC  ACC  CAG  AGC  CCC  ATG  AAG

CTG  ATG  GCC  CTG  CAG  CTG  CTG  CTG  TGG  CAC  AGT

GCA  CTC  TGG  ACA  GTG  CAG  GAA  GCC  ACC  CCC  CTG

GGC  CCT  GCC  AGC  TCC  CTG  CCC  CAG  AGC  TTC  CTG

CTC  AAG  TGC  TTA  GAG  CAA  GTG  AGG  AAG  ATC  CAG

GGC  GAT  GGC  GCA  GCG  CTC  CAG  GAG  AAG  CTG  (GTG
```

$$\text{AGT} \quad \text{GAG)}_m\text{TGT} \quad \text{GCC} \quad \text{ACC} \quad \text{TAC} \quad \text{AAG} \quad \text{CTG} \quad \text{TGC} \quad \text{CAC} \quad \text{CCC}$$

```
GAG  GAG  CTG  GTG  CTG  CTC  GGA  CAC  TCT  CTG  GGC

ATC  CCC  TGG  GCT  CCC  CTG  AGC  AGC  TGC  CCC  AGC

CAG  GCC  CTG  CAG  CTG  GCA  GGC  TGC  TTG  AGC  CAA

CTC  CAT  AGC  GGC  CTT  TTC  CTC  TAC  CAG  GGG  CTC

CTG  CAG  GCC  CTG  GAA  GGG  ATC  TCC  CCC  GAG  TTG

GGT  CCC  ACC  TTG  GAC  ACA  CTG  CAG  CTG  GAC  GTC

GCC  GAC  TTT  GCC  ACC  ACC  ATC  TGG  CAG  CAG  ATG

GAA  GAA  CTG  GGA  ATG  GCC  CCT  GCC  CTG  CAG  CCC

ACC  CAG  GGT  GCC  ATG  CCG  GCC  TTC  GCC  TCT  GCT

TTC  CAG  CGC  CGG  GCA  GGA  GGG  GTC  CTG  GTT  GCC

TCC  CAT  CTG  CAG  AGC  TTC  CTG  GAG  GTG  TCG  TAC

CGC  GTT  CTA  CGC  CAC  CTT  GCC  CAG  CCC
```

(where m is 0 or 1).

3. A recombinant vector containing a DNA sequence according to claim 1 or 2.

4. A transformant containing a recombinant vector according to claim 3.

5. A process for the production of a protein comprising cultivating a transformant according to claim 4 under suitable conditions and recovering said protein.

6. A protein having the following amino acid sequence :

```
      -30
      Met   Ala   Gly   Pro   Ala   Thr   Gln   Ser   Pro   Met   Lys
      Leu   Met   Ala   Leu   Gln   Leu   Leu   Leu   Trp   His   Ser
                                              -1    +1
      Ala   Leu   Trp   Thr   Val   Gln   Glu   Ala   Thr   Pro   Leu
      Gly   Pro   Ala   Ser   Ser   Leu   Pro   Gln   Ser   Phe   Leu
      Leu   Lys   Cys   Leu   Glu   Gln   Val   Arg   Lys   Ile   Gln
      Gly   Asp   Gly   Ala   Ala   Leu   Gln   Glu   Lys   Leu   (Val
      Ser   Glu)  Cys   Ala   Thr   Tyr   Lys   Leu   Cys   His   Pro
                m
      Glu   Glu   Leu   Val   Leu   Leu   Gly   His   Ser   Leu   Gly
      Ile   Pro   Trp   Ala   Pro   Leu   Ser   Ser   Cys   Pro   Ser
      Gln   Ala   Leu   Gln   Leu   Ala   Gly   Cys   Leu   Ser   Gln
      Leu   His   Ser   Gly   Leu   Phe   Leu   Tyr   Gln   Gly   Leu
      Leu   Gln   Ala   Leu   Glu   Gly   Ile   Ser   Pro   Glu   Leu
      Gly   Pro   Thr   Leu   Asp   Thr   Leu   Gln   Leu   Asp   Val
      Ala   Asp   Phe   Ala   Thr   Thr   Ile   Trp   Gln   Gln   Met
      Glu   Glu   Leu   Gly   Met   Ala   Pro   Ala   Leu   Gln   Pro
      Thr   Gln   Gly   Ala   Met   Pro   Ala   Phe   Ala   Ser   Ala
      Phe   Gln   Arg   Arg   Ala   Gly   Gly   Val   Leu   Val   Ala
      Ser   His   Leu   Gln   Ser   Phe   Leu   Glu   Val   Ser   Tyr
                                                    +174
      Arg   Val   Leu   Arg   His   Leu   Ala   Gln   Pro
```

(where m is 0 or 1).

7. A human granulocyte colony stimulating factor having the following amino acid sequence :

```
+1
Thr Pro Leu Gly Pro Ala Ser Ser Leu Pro Gln
Ser Phe Leu Leu Lys Cys Leu Glu Gln Val Arg
Lys Ile Gln Gly Asp Gly Ala Ala Leu Gln Glu
Lys Leu Val Ser Glu Cys Ala Thr Tyr Lys Leu
Cys His Pro Glu Glu Leu Val Leu Leu Gly His
Ser Leu Gly Ile Pro Trp Ala Pro Leu Ser Ser
Cys Pro Ser Gln Ala Leu Gln Leu Ala Gly Cys
Leu Ser Gln Leu His Ser Gly Leu Phe Leu Tyr
Gln Gly Leu Leu Gln Ala Leu Glu Gly Ile Ser
Pro Glu Leu Gly Pro Thr Leu Asp Thr Leu Gln
Leu Asp Val Ala Asp Phe Ala Thr Thr Ile Trp
Gln Gln Met Glu Glu Leu Gly Met Ala Pro Ala
Leu Gln Pro Thr Gln Gly Ala Met Pro Ala Phe
Ala Ser Ala Phe Gln Arg Arg Ala Gly Gly Val
Leu Val Ala Ser His Leu Gln Ser Phe Leu Glu
Val Ser Tyr Arg Val Leu Arg His Leu Ala Gln
+177
Pro
```

8. An infection protective agent which contains the humans granulocyte colony stimulating factor according to claim 7 as an effective ingredient.

**Claims for the following Contracting State : AT**

1. A recombinant vector containing a cDNA sequence coding for the following amino acid sequence :

-30

| Met | Ala | Gly | Pro | Ala | Thr | Gln | Ser | Pro | Met | Lys |
|-----|-----|-----|-----|-----|-----|-----|-----|-----|-----|-----|
| Leu | Met | Ala | Leu | Gln | Leu | Leu | Leu | Trp | His | Ser |
| Ala | Leu | Trp | Thr | Val | Gln | Glu | Ala<br>-1 | Thr<br>+1 | Pro | Leu |
| Gly | Pro | Ala | Ser | Ser | Leu | Pro | Gln | Ser | Phe | Leu |
| Leu | Lys | Cys | Leu | Glu | Gln | Val | Arg | Lys | Ile | Gln |
| Gly | Asp | Gly | Ala | Ala | Leu | Gln | Glu | Lys | Leu | (Val |
| Ser | Glu)$_m$ | Cys | Ala | Thr | Tyr | Lys | Leu | Cys | His | Pro |
| Glu | Glu | Leu | Val | Leu | Leu | Gly | His | Ser | Leu | Gly |
| Ile | Pro | Trp | Ala | Pro | Leu | Ser | Ser | Cys | Pro | Ser |
| Gln | Ala | Leu | Gln | Leu | Ala | Gly | Cys | Leu | Ser | Gln |
| Leu | His | Ser | Gly | Leu | Phe | Leu | Tyr | Gln | Gly | Leu |
| Leu | Gln | Ala | Leu | Glu | Gly | Ile | Ser | Pro | Glu | Leu |
| Gly | Pro | Thr | Leu | Asp | Thr | Leu | Gln | Leu | Asp | Val |
| Ala | Asp | Phe | Ala | Thr | Thr | Ile | Trp | Gln | Gln | Met |
| Glu | Glu | Leu | Gly | Met | Ala | Pro | Ala | Leu | Gln | Pro |
| Thr | Gln | Gly | Ala | Met | Pro | Ala | Phe | Ala | Ser | Ala |
| Phe | Gln | Arg | Arg | Ala | Gly | Gly | Val | Leu | Val | Ala |
| Ser | His | Leu | Gln | Ser | Phe | Leu | Glu | Val | Ser | Tyr |
| Arg | Val | Leu | Arg | His | Leu | Ala | Gln | Pro<br>+174 | | |

(where m is 0 or 1)

or for the amino acid sequence :

```
  +1
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  Val  Ser  Glu  Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
+177
Pro
```

2. The recombinant vector according to claim 1 wherein the cDNA sequence has the following nucleotide sequence :

| | | | | | | | | | |
|---|---|---|---|---|---|---|---|---|---|
| ATG | GCT | GGA | CCT | GCC | ACC | CAG | AGC | CCC | ATG | AAG |
| CTG | ATG | GCC | CTG | CAG | CTG | CTG | CTG | TGG | CAC | AGT |
| GCA | CTC | TGG | ACA | GTG | CAG | GAA | GCC | ACC | CCC | CTG |
| GGC | CCT | GCC | AGC | TCC | CTG | CCC | CAG | AGC | TTC | CTG |
| CTC | AAG | TGC | TTA | GAG | CAA | GTG | AGG | AAG | ATC | CAG |
| GGC | GAT | GGC | GCA | GCG | CTC | CAG | GAG | AAG | CTG | (GTG |
| AGT | GAG)$_m$ | TGT | GCC | ACC | TAC | AAG | CTG | TGC | CAC | CCC |
| GAG | GAG | CTG | GTG | CTG | CTC | GGA | CAC | TCT | CTG | GGC |
| ATC | CCC | TGG | GCT | CCC | CTG | AGC | AGC | TGC | CCC | AGC |
| CAG | GCC | CTG | CAG | CTG | GCA | GGC | TGC | TTG | AGC | CAA |
| CTC | CAT | AGC | GGC | CTT | TTC | CTC | TAC | CAG | GGG | CTC |
| CTG | CAG | GCC | CTG | GAA | GGG | ATC | TCC | CCC | GAG | TTG |
| GGT | CCC | ACC | TTG | GAC | ACA | CTG | CAG | CTG | GAC | GTC |
| GCC | GAC | TTT | GCC | ACC | ACC | ATC | TGG | CAG | CAG | ATG |
| GAA | GAA | CTG | GGA | ATG | GCC | CCT | GCC | CTG | CAG | CCC |
| ACC | CAG | GGT | GCC | ATG | CCG | GCC | TTC | GCC | TCT | GCT |
| TTC | CAG | CGC | CGG | GCA | GGA | GGG | GTC | CTG | GTT | GCC |
| TCC | CAT | CTG | CAG | AGC | TTC | CTG | GAG | GTG | TCG | TAC |
| CGC | GTT | CTA | CGC | CAC | CTT | GCC | CAG | CCC | | |

(where m is 0 or 1).
or the nucleotide sequence :

|     |     |     |     |     |     |     |     | ACC | CCC | CTG |
|-----|-----|-----|-----|-----|-----|-----|-----|-----|-----|-----|
| GGC | CCT | GCC | AGC | TCC | CTG | CCC | CAG | AGC | TTC | CTG |
| CTC | AAG | TGC | TTA | GAG | CAA | GTG | AGG | AAG | ATC | CAG |
| GGC | GAT | GGC | GCA | GCG | CTC | CAG | GAG | AAG | CTG | (GTG |
| AGT | GAG)$_m$ | TGT | GCC | ACC | TAC | AAG | CTG | TGC | CAC | CCC |
| GAG | GAG | CTG | GTG | CTG | CTC | GGA | CAC | TCT | CTG | GGC |
| ATC | CCC | TGG | GCT | CCC | CTG | AGC | AGC | TGC | CCC | AGC |
| CAG | GCC | CTG | CAG | CTG | GCA | GGC | TGC | TTG | AGC | CAA |
| CTC | CAT | AGC | GGC | CTT | TTC | CTC | TAC | CAG | GGG | CTC |
| CTG | CAG | GCC | CTG | GAA | GGG | ATC | TCC | CCC | GAG | TTG |
| GGT | CCC | ACC | TTG | GAC | ACA | CTG | CAG | CTG | GAC | GTC |
| GCC | GAC | TTT | GCC | ACC | ACC | ATC | TGG | CAG | CAG | ATG |
| GAA | GAA | CTG | GGA | ATG | GCC | CCT | GCC | CTG | CAG | CCC |
| ACC | CAG | GGT | GCC | ATG | CCG | GCC | TTC | GCC | TCT | GCT |
| TTC | CAG | CGC | CGG | GCA | GGA | GGG | GTC | CTG | GTT | GCC |
| TCC | CAT | CTG | CAG | AGC | TTC | CTG | GAG | GTG | TCG | TAC |
| CGC | GTT | CTA | CGC | CAC | CTT | GCC | CAG | CCC |     |     |

3. A transformant containing a recombinant vector according to claim 1 or 2.

4. A process for the production of a protein comprising cultivating a transformant according to claim 3 under suitable conditions and recovering said protein.

5. A process for the production of an infection protective agent which comprises combining a human granulocyte colony stimulator factor having the following amino acid sequence :

```
+1
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  Val  Ser  Glu  Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
+177
Pro
```

with a pharmaceutically acceptable carrier excipient, stabilizer, or adsorption preventing agent.

**Patentansprüche**

**Patentansprüche Für folgenden Vertragsstaaten : BE, CH, LI, DE, FR, GB, IT, NL, SE**

1. cDNA-Sequenz, die für die folgende Aminosäuresequenz codiert :

-30

| Met | Ala | Gly | Pro | Ala | Thr | Gln | Ser | Pro | Met | Lys |
|-----|-----|-----|-----|-----|-----|-----|-----|-----|-----|-----|
| Leu | Met | Ala | Leu | Gln | Leu | Leu | Leu | Trp | His | Ser |
| Ala | Leu | Trp | Thr | Val | Gln | Glu | Ala<sup>-1</sup> | Thr<sup>+1</sup> | Pro | Leu |
| Gly | Pro | Ala | Ser | Ser | Leu | Pro | Gln | Ser | Phe | Leu |
| Leu | Lys | Cys | Leu | Glu | Gln | Val | Arg | Lys | Ile | Gln |
| Gly | Asp | Gly | Ala | Ala | Leu | Gln | Glu | Lys | Leu | (Val |
| Ser | Glu)ₘ | Cys | Ala | Thr | Tyr | Lys | Leu | Cys | His | Pro |
| Glu | Glu | Leu | Val | Leu | Leu | Gly | His | Ser | Leu | Gly |
| Ile | Pro | Trp | Ala | Pro | Leu | Ser | Ser | Cys | Pro | Ser |
| Gln | Ala | Leu | Gln | Leu | Ala | Gly | Cys | Leu | Ser | Gln |
| Leu | His | Ser | Gly | Leu | Phe | Leu | Tyr | Gln | Gly | Leu |
| Leu | Gln | Ala | Leu | Glu | Gly | Ile | Ser | Pro | Glu | Leu |
| Gly | Pro | Thr | Leu | Asp | Thr | Leu | Gln | Leu | Asp | Val |
| Ala | Asp | Phe | Ala | Thr | Thr | Ile | Trp | Gln | Gln | Met |
| Glu | Glu | Leu | Gly | Met | Ala | Pro | Ala | Leu | Gln | Pro |
| Thr | Gln | Gly | Ala | Met | Pro | Ala | Phe | Ala | Ser | Ala |
| Phe | Gln | Arg | Arg | Ala | Gly | Gly | Val | Leu | Val | Ala |
| Ser | His | Leu | Gln | Ser | Phe | Leu | Glu | Val | Ser | Tyr |
| Arg | Val | Leu | Arg | His | Leu | Ala | Gln | Pro<sup>+174</sup> | | |

(in der m 0 oder 1 bedeutet).
2. cDNA-Sequenz nach Anspruch 1, die die folgende Nucleotidsequenz aufweist :

```
ATG   GCT   GGA   CCT   GCC   ACC   CAG   AGC   CCC   ATG   AAG
CTG   ATG   GCC   CTG   CAG   CTG   CTG   CTG   TGG   CAC   AGT
GCA   CTC   TGG   ACA   GTG   CAG   GAA   GCC   ACC   CCC   CTG
GGC   CCT   GCC   AGC   TCC   CTG   CCC   CAG   AGC   TTC   CTG
CTC   AAG   TGC   TTA   GAG   CAA   GTG   AGG   AAG   ATC   CAG
GGC   GAT   GGC   GCA   GCG   CTC   CAG   GAG   AAG   CTG   (GTG
AGT   GAG)ₘ  TGT   GCC   ACC   TAC   AAG   CTG   TGC   CAC   CCC
GAG   GAG   CTG   GTG   CTG   CTC   GGA   CAC   TCT   CTG   GGC
ATC   CCC   TGG   GCT   CCC   CTG   AGC   AGC   TGC   CCC   AGC
CAG   GCC   CTG   CAG   CTG   GCA   GGC   TGC   TTG   AGC   CAA
CTC   CAT   AGC   GGC   CTT   TTC   CTC   TAC   CAG   GGG   CTC
CTG   CAG   GCC   CTG   GAA   GGG   ATC   TCC   CCC   GAG   TTG
GGT   CCC   ACC   TTG   GAC   ACA   CTG   CAG   CTG   GAC   GTC
GCC   GAC   TTT   GCC   ACC   ACC   ATC   TGG   CAG   CAG   ATG
GAA   GAA   CTG   GGA   ATG   GCC   CCT   GCC   CTG   CAG   CCC
ACC   CAG   GGT   GCC   ATG   CCG   GCC   TTC   GCC   TCT   GCT
TTC   CAG   CGC   CGG   GCA   GGA   GGG   GTC   CTG   GTT   GCC
TCC   CAT   CTG   CAG   AGC   TTC   CTG   GAG   GTG   TCG   TAC
CGC   GTT   CTA   CGC   CAC   CTT   GCC   CAG   CCC
```

(in der m 0 oder 1 bedeutet).

3. Rekombinanter Vektor, der eine DNA-Sequenz nach Anspruch 1 oder 2 enthält.

4. Transformante, die einen rekombinanten Vektor nach Anspruch 3 enthält.

5. Verfahren zur Herstellung eines Proteins, bei dem man eine Transformante nach Anspruch 4 unter geeigneten Bedingungen züchtet und das Protein gewinnt.

6. Protein mit der folgenden Aminosäuresequenz :

-30

| Met | Ala | Gly | Pro | Ala | Thr | Gln | Ser | Pro | Met | Lys |
|-----|-----|-----|-----|-----|-----|-----|-----|-----|-----|-----|
| Leu | Met | Ala | Leu | Gln | Leu | Leu | Leu | Trp | His | Ser |
| Ala | Leu | Trp | Thr | Val | Gln | Glu | Ala[-1] | Thr[+1] | Pro | Leu |
| Gly | Pro | Ala | Ser | Ser | Leu | Pro | Gln | Ser | Phe | Leu |
| Leu | Lys | Cys | Leu | Glu | Gln | Val | Arg | Lys | Ile | Gln |
| Gly | Asp | Gly | Ala | Ala | Leu | Gln | Glu | Lys | Leu | (Val |
| Ser | Glu)$_m$ | Cys | Ala | Thr | Tyr | Lys | Leu | Cys | His | Pro |
| Glu | Glu | Leu | Val | Leu | Leu | Gly | His | Ser | Leu | Gly |
| Ile | Pro | Trp | Ala | Pro | Leu | Ser | Ser | Cys | Pro | Ser |
| Gln | Ala | Leu | Gln | Leu | Ala | Gly | Cys | Leu | Ser | Gln |
| Leu | His | Ser | Gly | Leu | Phe | Leu | Tyr | Gln | Gly | Leu |
| Leu | Gln | Ala | Leu | Glu | Gly | Ile | Ser | Pro | Glu | Leu |
| Gly | Pro | Thr | Leu | Asp | Thr | Leu | Gln | Leu | Asp | Val |
| Ala | Asp | Phe | Ala | Thr | Thr | Ile | Trp | Gln | Gln | Met |
| Glu | Glu | Leu | Gly | Met | Ala | Pro | Ala | Leu | Gln | Pro |
| Thr | Gln | Gly | Ala | Met | Pro | Ala | Phe | Ala | Ser | Ala |
| Phe | Gln | Arg | Arg | Ala | Gly | Gly | Val | Leu | Val | Ala |
| Ser | His | Leu | Gln | Ser | Phe | Leu | Glu | Val | Ser | Tyr |
| Arg | Val | Leu | Arg | His | Leu | Ala | Gln | Pro[+174] | | |

(in der m 0 oder 1 bedeutet).

7. Menschlicher Granulocyten-Kolonie-stimulierender Faktor mit der folgenden Aminosäuresequenz :

```
+1
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  Val  Ser  Glu  Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
+177
Pro
```

8. Mittel zum Schutz vor Infektionen, das den menschlichen Granulocyten-Kolonie-stimulierenden Faktor nach Anspruch 7 als Wirkstoff enthält.

**Patentansprüche Für folgenden Vertragsstaat : AT**

1. Rekombinanter Vektor, der eine cDNA-Sequenz enthält, die für die folgende Aminosäuresequenz codiert :

-30

| Met | Ala | Gly | Pro | Ala | Thr | Gln | Ser | Pro | Met | Lys |
| Leu | Met | Ala | Leu | Gln | Leu | Leu | Leu | Trp | His | Ser |
| Ala | Leu | Trp | Thr | Val | Gln | Glu | Ala$^{-1}$ | Thr$^{+1}$ | Pro | Leu |
| Gly | Pro | Ala | Ser | Ser | Leu | Pro | Gln | Ser | Phe | Leu |
| Leu | Lys | Cys | Leu | Glu | Gln | Val | Arg | Lys | Ile | Gln |
| Gly | Asp | Gly | Ala | Ala | Leu | Gln | Glu | Lys | Leu | (Val |
| Ser | Glu)$_m$ | Cys | Ala | Thr | Tyr | Lys | Leu | Cys | His | Pro |
| Glu | Glu | Leu | Val | Leu | Leu | Gly | His | Ser | Leu | Gly |
| Ile | Pro | Trp | Ala | Pro | Leu | Ser | Ser | Cys | Pro | Ser |
| Gln | Ala | Leu | Gln | Leu | Ala | Gly | Cys | Leu | Ser | Gln |
| Leu | His | Ser | Gly | Leu | Phe | Leu | Tyr | Gln | Gly | Leu |
| Leu | Gln | Ala | Leu | Glu | Gly | Ile | Ser | Pro | Glu | Leu |
| Gly | Pro | Thr | Leu | Asp | Thr | Leu | Gln | Leu | Asp | Val |
| Ala | Asp | Phe | Ala | Thr | Thr | Ile | Trp | Gln | Gln | Met. |
| Glu | Glu | Leu | Gly | Met | Ala | Pro | Ala | Leu | Gln | Pro |
| Thr | Gln | Gly | Ala | Met | Pro | Ala | Phe | Ala | Ser | Ala |
| Phe | Gln | Arg | Arg | Ala | Gly | Gly | Val | Leu | Val | Ala |
| Ser | His | Leu | Gln | Ser | Phe | Leu | Glu | Val | Ser | Tyr |
| Arg | Val | Leu | Arg | His | Leu | Ala | Gln | Pro$^{+176}$ | | |

(in der m 0 oder 1 bedeutet)
oder für die Aminosäuresequenz :

+1

| Thr | Pro | Leu | Gly | Pro | Ala | Ser | Ser | Leu | Pro | Gln |
|-----|-----|-----|-----|-----|-----|-----|-----|-----|-----|-----|
| Ser | Phe | Leu | Leu | Lys | Cys | Leu | Glu | Gln | Val | Arg |
| Lys | Ile | Gln | Gly | Asp | Gly | Ala | Ala | Leu | Gln | Glu |
| Lys | Leu | Val | Ser | Glu | Cys | Ala | Thr | Tyr | Lys | Leu |
| Cys | His | Pro | Glu | Glu | Leu | Val | Leu | Leu | Gly | His |
| Ser | Leu | Gly | Ile | Pro | Trp | Ala | Pro | Leu | Ser | Ser |
| Cys | Pro | Ser | Gln | Ala | Leu | Gln | Leu | Ala | Gly | Cys |
| Leu | Ser | Gln | Leu | His | Ser | Gly | Leu | Phe | Leu | Tyr |
| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |

+177
Pro

2. Rekombinanter Vektor nach Anspruch 1, wobei die cDNA-Sequenz die folgende Nucleotidsequenz aufweist :

| ATG | GCT | GGA | CCT | GCC | ACC | CAG | AGC | CCC | ATG | AAG |
|-----|-----|-----|-----|-----|-----|-----|-----|-----|-----|-----|
| CTG | ATG | GCC | CTG | CAG | CTG | CTG | CTG | TGG | CAC | AGT |
| GCA | CTC | TGG | ACA | GTG | CAG | GAA | GCC | ACC | CCC | CTG |
| GGC | CCT | GCC | AGC | TCC | CTG | CCC | CAG | AGC | TTC | CTG |
| CTC | AAG | TGC | TTA | GAG | CAA | GTG | AGG | AAG | ATC | CAG |
| GGC | GAT | GGC | GCA | GCG | CTC | CAG | GAG | AAG | CTG | (GTG |
| AGT | GAG)$_m$TGT | GCC | ACC | TAC | AAG | CTG | TGC | CAC | CCC | |
| GAG | GAG | CTG | GTG | CTG | CTC | GGA | CAC | TCT | CTG | GGC |
| ATC | CCC | TGG | GCT | CCC | CTG | AGC | AGC | TGC | CCC | AGC |
| CAG | GCC | CTG | CAG | CTG | GCA | GGC | TGC | TTG | AGC | CAA |
| CTC | CAT | AGC | GGC | CTT | TTC | CTC | TAC | CAG | GGG | CTC |
| CTG | CAG | GCC | CTG | GAA | GGG | ATC | TCC | CCC | GAG | TTG |
| GGT | CCC | ACC | TTG | GAC | ACA | CTG | CAG | CTG | GAC | GTC |
| GCC | GAC | TTT | GCC | ACC | ACC | ATC | TGG | CAG | CAG | ATG |
| GAA | GAA | CTG | GGA | ATG | GCC | CCT | GCC | CTG | CAG | CCC |

```
ACC   CAG   GGT   GCC   ATG   CCG   GCC   TTC·  GCC   TCT   GCT
TTC   CAG   CGC   CGG   GCA   GGA   GGG   GTC   CTG   GTT   GCC
TCC   CAT   CTG   CAG   AGC   TTC   CTG   GAG   GTG   TCG   TAC
CGC   GTT   CTA   CGC   CAC   CTT   GCC   CAG   CCC
```

(in der m 0 oder 1 bedeutet)
oder die Nucleotidsequenz :

```
                                                      ACC   CCC   CTG
GGC   CCT   GCC   AGC   TCC   CTG   CCC   CAG   AGC   TTC   CTG·
CTC   AAG   TGC   TTA   GAG   CAA   GTG   AGG   AAG   ATC   CAG
GGC   GAT   GGC   GCA   GCG   CTC   CAG.  GAG   AAG   CTG   (GTG
AGT   GAG)ₘTGT   GCC   ACC   TAC   AAG   CTG   TGC   CAC   CCC
GAG   GAG   CTG   GTG   CTG   CTC   GGA   CAC   TCT   CTG   GGC
ATC   CCC   TGG   GCT   CCC   CTG   AGC   AGC   TGC   CCC   AGC
CAG   GCC   CTG   CAG   CTG   GCA   GGC   TGC   TTG   AGC   CAA
CTC   CAT   AGC   GGC   CTT   TTC   CTC   TAC   CAG   GGG   CTC
CTG   CAG   GCC   CTG   GAA   GGG   ATC   TCC   CCC   GAG   TTG
GGT   CCC   ACC   TTG   GAC   ACA   CTG   CAG   CTG   GAC   GTC
GCC   GAC   TTT   GCC   ACC   ACC   ATC   TGG   CAG   CAG   ATG
GAA   GAA   CTG   GGA   ATG   GCC   CCT   GCC   CTG   CAG   CCC
ACC   CAG   GGT   GCC   ATG   CCG   GCC   TTC·  GCC   TCT   GCT
TTC   CAG   CGC   CGG   GCA   GGA   GGG   GTC   CTG   GTT   GCC
TCC   CAT   CTG   CAG   AGC   TTC   CTG   GAG   GTG   TCG   TAC
CGC   GTT   CTA   CGC   CAC   CTT   GCC   CAG   CCC
```

3. Transformante, die einen rekombinanten Vektor nach Anspruch 1 oder 2 enthält.

4. Verfahren zur Herstellung eines Proteins, bei dem man eine Transformante nach Anspruch 3 unter geeigneten Bedingungen züchtet und das Protein gewinnt.

5. Verfahren zur Herstellung eines Mittels zum Schutz vor Infektionen, bei dem man einen menschlichen Granulocyten-Kolonie-stimulierenden Faktor der folgenden Aminosäuresequenz :

```
 +1
Thr   Pro   Leu   Gly   Pro   Ala   Ser   Ser   Leu   Pro   Gln
Ser   Phe   Leu   Leu   Lys   Cys   Leu   Glu   Gln   Val   Arg
Lys   Ile   Gln   Gly   Asp   Gly   Ala   Ala   Leu   Gln   Glu
Lys   Leu   Val   Ser   Glu   Cys   Ala   Thr   Tyr   Lys   Leu
Cys   His   Pro   Glu   Glu   Leu   Val   Leu   Leu   Gly   His
Ser   Leu   Gly   Ile   Pro   Trp   Ala   Pro   Leu   Ser   Ser

Cys   Pro   Ser   Gln   Ala   Leu   Gln   Leu   Ala   Gly   Cys
Leu   Ser   Gln   Leu   His   Ser   Gly   Leu   Phe   Leu   Tyr
Gln   Gly   Leu   Leu   Gln   Ala   Leu   Glu   Gly   Ile   Ser
Pro   Glu   Leu   Gly   Pro   Thr   Leu   Asp   Thr   Leu   Gln
Leu   Asp   Val   Ala   Asp   Phe   Ala   Thr   Thr   Ile   Trp
Gln   Gln   Met   Glu   Glu   Leu   Gly   Met   Ala   Pro   Ala
Leu   Gln   Pro   Thr   Gln   Gly   Ala   Met   Pro   Ala   Phe
Ala   Ser   Ala   Phe   Gln   Arg   Arg   Ala   Gly   Gly   Val
Leu   Val   Ala   Ser   His   Leu   Gln   Ser   Phe   Leu   Glu
Val   Ser   Tyr   Arg   Val   Leu   Arg   His   Leu   Ala   Gln
+177
Pro
```

mit einem pharmazeutisch verträglichen Träger, Excipients, Stabilisator oder Adsorptionsverhinderungsmittel vermischt.

## Revendications

**Revendications pour les Etats contractants suivants : BE, CH, LI, DE, FR, GB, IT, NL, SE**

1. Séquence d'ADNc codant pour la séquence suivante d'amino-acides :

```
        -30
        Met  Ala  Gly  Pro  Ala  Thr  Gln  Ser  Pro  Met  Lys
        Leu  Met  Ala  Leu  Gln  Leu  Leu  Leu  Trp  His  Ser
                                            -1   +1
        Ala  Leu  Trp  Thr  Val  Gln  Glu  Ala  Thr  Pro  Leu
        Gly  Pro  Ala  Ser  Ser  Leu  Pro  Gln  Ser  Phe  Leu
        Leu  Lys  Cys  Leu  Glu  Gln  Val  Arg  Lys  Ile  Gln
        Gly  Asp  Gly  Ala  Ala  Leu  Gln  Glu  Lys  Leu  (Val
        Ser  Glu)_m Cys  Ala  Thr  Tyr  Lys  Leu  Cys  His  Pro
        Glu  Glu  Leu  Val  Leu  Leu  Gly  His  Ser  Leu  Gly
        Ile  Pro  Trp  Ala  Pro  Leu  Ser  Ser  Cys  Pro  Ser
        Gln  Ala  Leu  Gln  Leu  Ala  Gly  Cys  Leu  Ser  Gln
        Leu  His  Ser  Gly  Leu  Phe  Leu  Tyr  Gln  Gly  Leu
        Leu  Gln  Ala  Leu  Glu  Gly  Ile  Ser  Pro  Glu  Leu
        Gly  Pro  Thr  Leu  Asp  Thr  Leu  Gln  Leu  Asp  Val
        Ala  Asp  Phe  Ala  Thr  Thr  Ile  Trp  Gln  Gln  Met
        Glu  Glu  Leu  Gly  Met  Ala  Pro  Ala  Leu  Gln  Pro
        Thr  Gln  Gly  Ala  Met  Pro  Ala  Phe  Ala  Ser  Ala
        Phe  Gln  Arg  Arg  Ala  Gly  Gly  Val  Leu  Val  Ala
        Ser  His  Leu  Gln  Ser  Phe  Leu  Glu  Val  Ser  Tyr
                                                      +174
        Arg  Val  Leu  Arg  His  Leu  Ala  Gln  Pro
```

(dans laquelle m est 0 ou 1).

2. Séquence d'ADNc selon la revendication 1 ayant la séquence suivante de nucléotides :

```
        ATG—  GCT  GGA  CCT  GCC  ACC  CAG  AGC  CCC  ATG  AAG
        CTG   ATG  GCC  CTG  CAG  CTG  CTG  CTG  TGG  CAC  AGT
        GCA   CTC  TGG  ACA  GTG  CAG  GAA  GCC  ACC  CCC  CTG
        GGC   CCT  GCC  AGC  TCC  CTG  CCC  CAG  AGC  TTC  CTG
        CTC   AAG  TGC  TTA  GAG  CAA  GTG  AGG  AAG  ATC  CAG
        GGC   GAT  GGC  GCA  GCG  CTC  CAG  GAG  AAG  CTG  (GTG
        AGT   GAG)_m TGT  GCC  ACC  TAC  AAG  CTG  TGC  CAC  CCC
        GAG   GAG  CTG  GTG  CTG  CTC  GGA  CAC  TCT  CTG  GGC
        ATC   CCC  TGG  GCT  CCC  CTG  AGC  AGC  TGC  CCC  AGC
```

```
CAG   GCC   CTG   CAG   CTG   GCA   GGC   TGC   TTG   AGC   CAA
CTC   CAT   AGC   GGC   CTT   TTC   CTC   TAC   CAG   GGG   CTC
CTG   CAG   GCC   CTG   GAA   GGG   ATC   TCC   CCC   GAG   TTG
GGT   CCC   ACC   TTG   GAC   ACA   CTG   CAG   CTG   GAC   GTC
GCC   GAC   TTT   GCC   ACC   ACC   ATC   TGG   CAG   CAG   ATG
GAA   GAA   CTG   GGA   ATG   GCC   CCT   GCC   CTG   CAG   CCC
ACC   CAG   GGT   GCC   ATG   CCG   GCC   TTC   GCC   TCT   GCT
TTC   CAG   CGC   CGG   GCA   GGA   GGG   GTC   CTG   GTT   GCC
TCC   CAT   CTG   CAG   AGC   TTC   CTG   GAG   GTG   TCG   TAC
CGC   GTT   CTA   CGC   CAC   CTT   GCC   CAG   CCC
```

(dans laquelle m est 0 ou 1).

3. Vecteur recombinant contenant une séquence d'ADN selon la revendication 1 ou 2.

4. Transformant contenant un vecteur recombiné selon la revendication 3.

5. Procédé pour la production d'une protéine comprenant la culture d'un transformant selon la revendication 4 dans des conditions appropriées et la récupération de ladite protéine.

6. Protéine ayant la séquence d'amino-acides suivante :

```
-30
Met   Ala   Gly   Pro   Ala   Thr   Gln   Ser   Pro   Met   Lys
Leu   Met   Ala   Leu   Gln   Leu   Leu   Leu   Trp   His   Ser
                                        -1    +1
Ala   Leu   Trp   Thr   Val   Gln   Glu  Ala   Thr   Pro   Leu
Gly   Pro   Ala   Ser   Ser   Leu   Pro   Gln   Ser   Phe   Leu
Leu   Lys   Cys   Leu   Glu   Gln   Val   Arg   Lys   Ile   Gln
Gly   Asp   Gly   Ala   Ala   Leu   Gln   Glu   Lys   Leu   (Val
Ser   Glu)  Cys   Ala   Thr   Tyr   Lys   Leu   Cys   His   Pro
        m
Glu   Glu   Leu   Val   Leu   Leu   Gly   His   Ser   Leu   Gly
Ile   Pro   Trp   Ala   Pro   Leu   Ser   Ser   Cys   Pro   Ser
Gln   Ala   Leu   Gln   Leu   Ala   Gly   Cys   Leu   Ser   Gln
Leu   His   Ser   Gly   Leu   Phe   Leu   Tyr   Gln   Gly   Leu
Leu   Gln   Ala   Leu   Glu   Gly   Ile   Ser   Pro   Glu   Leu
Gly   Pro   Thr   Leu   Asp   Thr   Leu   Gln   Leu   Asp   Val
Ala   Asp   Phe   Ala   Thr   Thr   Ile   Trp   Gln   Gln   Met
```

```
Glu   Glu   Leu   Gly   Met   Ala   Pro   Ala   Leu   Gln   Pro
Thr   Gln   Gly   Ala   Met   Pro   Ala   Phe   Ala   Ser   Ala
Phe   Gln   Arg   Arg   Ala   Gly   Gly   Val   Leu   Val   Ala
Ser   His   Leu   Gln   Ser   Phe   Leu   Glu   Val   Ser   Tyr
                                              +174
Arg   Val   Leu   Arg   His   Leu   Ala   Gln   Pro
```

60

(dans laquelle m est 0 ou 1).

7. Facteur stimulant la formation de colonies de granulocytes humain ayant la séquence suivante des amino-acides :

```
+1
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  Val  Ser  Glu  Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
+177
Pro
```

8. Agent de protection contre l'infection qui contient le facteur stimulant la formation de colonies de granulocytes humain selon la revendication 7 comme ingrédient actif.

**Revendications pour l'Etat contractant suivant : AT**

1. Vecteur recombinant contenant une séquence d'ADNc codant pour la séquence suivante d'amino-acides :

```
-30
Met  Ala  Gly  Pro  Ala  Thr  Gln  Ser  Pro  Met  Lys

Leu  Met  Ala  Leu  Gln  Leu  Leu  Leu  Trp  His  Ser
                                        -1   +1
Ala  Leu  Trp  Thr  Val  Gln  Glu  Ala  Thr  Pro  Leu

Gly  Pro  Ala  Ser  Ser  Leu  Pro  Gln  Ser  Phe  Leu

Leu  Lys  Cys  Leu  Glu  Gln  Val  Arg  Lys  Ile  Gln

Gly  Asp  Gly  Ala  Ala  Leu  Gln  Glu  Lys  Leu  (Val

Ser  Glu)  Cys  Ala  Thr  Tyr  Lys  Leu  Cys  His  Pro
          m
Glu  Glu  Leu  Val  Leu  Leu  Gly  His  Ser  Leu  Gly

Ile  Pro  Trp  Ala  Pro  Leu  Ser  Ser  Cys  Pro  Ser

Gln  Ala  Leu  Gln  Leu  Ala  Gly  Cys  Leu  Ser  Gln

Leu  His  Ser  Gly  Leu  Phe  Leu  Tyr  Gln  Gly  Leu

Leu  Gln  Ala  Leu  Glu  Gly  Ile  Ser  Pro  Glu  Leu

Gly  Pro  Thr  Leu  Asp  Thr  Leu  Gln  Leu  Asp  Val

Ala  Asp  Phe  Ala  Thr  Thr  Ile  Trp  Gln  Gln  Met

Glu  Glu  Leu  Gly  Met  Ala  Pro  Ala  Leu  Gln  Pro

Thr  Gln  Gly  Ala  Met  Pro  Ala  Phe  Ala  Ser  Ala

Phe  Gln  Arg  Arg  Ala  Gly  Gly  Val  Leu  Val  Ala

Ser  His  Leu  Gln  Ser  Phe  Leu  Glu  Val  Ser  Tyr
                                        +174
Arg  Val  Leu  Arg  His  Leu  Ala  Gln  Pro
```

(dans laquelle m est 0 ou 1)

ou pour la séquence d'amino-acides :

```
+1
Thr  Pro  Leu  Gly  Pro  Ala  Ser  Ser  Leu  Pro  Gln

Ser  Phe  Leu  Leu  Lys  Cys  Leu  Glu  Gln  Val  Arg

Lys  Ile  Gln  Gly  Asp  Gly  Ala  Ala  Leu  Gln  Glu

Lys  Leu  Val  Ser  Glu  Cys  Ala  Thr  Tyr  Lys  Leu

Cys  His  Pro  Glu  Glu  Leu  Val  Leu  Leu  Gly  His

Ser  Leu  Gly  Ile  Pro  Trp  Ala  Pro  Leu  Ser  Ser

Cys  Pro  Ser  Gln  Ala  Leu  Gln  Leu  Ala  Gly  Cys

Leu  Ser  Gln  Leu  His  Ser  Gly  Leu  Phe  Leu  Tyr
```

```
Gln   Gly   Leu   Leu   Gln   Ala   Leu   Glu   Gly   Ile   Ser
Pro   Glu   Leu   Gly   Pro   Thr   Leu   Asp   Thr   Leu   Gln
Leu   Asp   Val   Ala   Asp   Phe   Ala   Thr   Thr   Ile   Trp
Gln   Gln   Met   Glu   Glu   Leu   Gly   Met   Ala   Pro   Ala
Leu   Gln   Pro   Thr   Gln   Gly   Ala   Met   Pro   Ala   Phe
Ala   Ser   Ala   Phe   Gln   Arg   Arg   Ala   Gly   Gly   Val
Leu   Val   Ala   Ser   His   Leu   Gln   Ser   Phe   Leu   Glu
Val   Ser   Tyr   Arg   Val   Leu   Arg   His   Leu   Ala   Gln
+177
Pro
```

2. Vecteur recombinant selon la revendication 1, dans lequel la séquence d'ADNc a la séquence suivante de nucléotides :

```
ATG   GCT   GGA   CCT   GCC   ACC   CAG   AGC   CCC   ATG   AAG
CTG   ATG   GCC   CTG   CAG   CTG   CTG   CTG   TGG   CAC   AGT
GCA   CTC   TGG   ACA   GTG   CAG   GAA   GCC   ACC   CCC   CTG
GGC   CCT   GCC   AGC   TCC   CTG   CCC   CAG   AGC   TTC   CTG
CTC   AAG   TGC   TTA   GAG   CAA   GTG   AGG   AAG   ATC   CAG
GGC   GAT   GGC   GCA   GCG   CTC   CAG   GAG   AAG   CTG   (GTG
AGT   GAG)ₘ  TGT   GCC   ACC   TAC   AAG   CTG   TGC   CAC   CCC
GAG   GAG   CTG   GTG   CTG   CTC   GGA   CAC   TCT   CTG   GGC
ATC   CCC   TGG   GCT   CCC   CTG   AGC   AGC   TGC   CCC   AGC
CAG   GCC   CTG   CAG   CTG   GCA   GGC   TGC   TTG   AGC   CAA
CTC   CAT   AGC   GGC   CTT   TTC   CTC   TAC   CAG   GGG   CTC
CTG   CAG   GCC   CTG   GAA   GGG   ATC   TCC   CCC   GAG   TTG
GGT   CCC   ACC   TTG   GAC   ACA   CTG   CAG   CTG   GAC   GTC
GCC   GAC   TTT   GCC   ACC   ACC   ATC   TGG   CAG   CAG   ATG
GAA   GAA   CTG   GGA   ATG   GCC   CCT   GCC   CTG   CAG   CCC
ACC   CAG   GGT   GCC   ATG   CCG   GCC   TTC   GCC   TCT   GCT
TTC   CAG   CGC   CGG   GCA   GGA   GGG   GTC   CTG   GTT   GCC
TCC   CAT   CTG   CAG   AGC   TTC   CTG   GAG   GTG   TCG   TAC
CGC   GTT   CTA   CGC   CAC   CTT   GCC   CAG   CCC
```

(dans laquelle m est 0 ou 1)

ou la séquence de nucléotides :

```
                                        ACC  CCC  CTG
GGC  CCT  GCC  AGC  TCC  CTG  CCC  CAG  AGC  TTC  CTG
CTC  AAG  TGC  TTA  GAG  CAA  GTG  AGG  AAG  ATC  CAG
GGC  GAT  GGC  GCA  GCG  CTC  CAG  GAG  AAG  CTG  (GTG
AGT  GAG)ₘ TGT  GCC  ACC  TAC  AAG  CTG  TGC  CAC  CCC
GAG  GAG  CTG  GTG  CTG  CTC  GGA  CAC  TCT  CTG  GGC
ATC  CCC  TGG  GCT  CCC  CTG  AGC  AGC  TGC  CCC  AGC
CAG  GCC  CTG  CAG  CTG  GCA  GGC  TGC  TTG  AGC  CAA
CTC  CAT  AGC  GGC  CTT  TTC  CTC  TAC  CAG  GGG  CTC
CTG  CAG  GCC  CTG  GAA  GGG  ATC  TCC  CCC  GAG  TTG
GGT  CCC  ACC  TTG  GAC  ACA  CTG  CAG  CTG  GAC  GTC
GCC  GAC  TTT  GCC  ACC  ACC  ATC  TGG  CAG  CAG  ATG
GAA  GAA  CTG  GGA  ATG  GCC  CCT  GCC  CTG  CAG  CCC
ACC  CAG  GGT  GCC  ATG  CCG  GCC  TTC  GCC  TCT  GCT
TTC  CAG  CGC  CGG  GCA  GGA  GGG  GTC  CTG  GTT  GCC
TCC  CAT  CTG  CAG  AGC  TTC  CTG  GAG  GTG  TCG  TAC
CGC  GTT  CTA  CGC  CAC  CTT  GCC  CAG  CCC
```

3. Transformant contenant un vecteur recombinant selon la revendication 1 ou 2.

4. Procédé pour la production d'une protéine comprenant la culture d'un transformant selon la revendication 3 dans des conditions appropriées et la récupération de ladite protéine.

5. Procédé pour la production d'un agent de protection contre l'infection qui comprend la combinaison d'un facteur stimulant la formation des colonies de granulocytes humain ayant la séquence suivante d'amino-acides:

```
+1
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  Val  Ser  Glu  Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
```

| | | | | | | | | | |
|---|---|---|---|---|---|---|---|---|---|
| Leu | Ser | Gln | Leu | His | Ser | Gly | Leu | Phe | Leu | Tyr |
| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |

+177
Pro

avec un véhicule, excipient, stabilisant ou agent évitant l'adsorption, pharmaceutiquement acceptables.

(Fig. 1)

(Probe)  ( I W Q )

Ile Trp Gln Gln Met Glu Glu Leu Gly Met

5'---- ATI TGG CAA CAA ATG GAA GAA CTI GGI ATG ----3'
             G   G           G   G T

(Probe)  ( A )

Met Pro Ala Phe Ala

5'---- ATG CCA GCA TTT GC ----3'
            T   T   C
            G   G
            C   C

3'---- TAC GGA CGA AAA CG ----5'
            T   T   G
            G   G
            C   C            ⎤ (A)
                             ⎦(Probe)

(Probe)  ( L C )

Gln Glu Lys Leu Cys Ala Thr Tyr

5'---- CAG GAG AAG CTG TGT GCC ACC TAC ---3'

3'---- GTC CTC TTC GAC ACA CGG TGG ATG ----5'   (LC)
                                              (Probe)

(Fig. 2)

```
CC  CTG  GAA  GGG  ATC  TCC  CCC  GAG
TTG  GGT  CCC  ACC  TTG  GAC  ACA  CTG
CAG  CTG  GAC  GTC  GCC  GAC  TTT  GCC
ACC  ACC  ATC  TGG  CAG  CAG  ATG  GAA
GAA  CTG  GGA  ATG  GCC  CCT  GCC  CTG
CAG  CCC  ACC  CAG  GGT  GCC  ATG  CCG
GCC  TTC  GCC  TCT  GCT  TTC  CAG  CGC
CGG  GCA  GGA  GGG  GTC  CTA  GTT  GCC
TCC  CAT  CTG  CAG  AGC  TTC  CTG  GAG
GTG  TCG  TAC  CGC  GTT  CTA  CGC  CAC
CTT  GCC  CAG  CCC  TGA  GCC  AAG  CCC
TCC  CCA  TCC  CAT  GTA  TTT  ATC  TCT
ATT  TAA  TAT  TTA  TGT  CTA  TTT
```

(Fig. 3(A)-1)

```
                                           10                        30
                       CGGAGCCTGCAGCCCAGCCCCACCCAGACCC


                50                        70                       90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                          -20


                110                       130                      150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
-10                                      -1    1                              10


                170                       190                      210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                                  20                                         30


                230                       250                      270
CTC CAG GAG AAG CTG GTG AGT GAG TGT GCC ACC TAC AAG CTG TGC CAC CCC GAG GAG CTG
Leu Gln Glu Lys Leu Val Ser Glu Cys Ala Thr Tyr Lys Leu Cys His Pro Glu Glu Leu
                                  40                                         50


                290                       310                      330
GTG CTG CTC GGA CAC TCT CTG GGC ATC CCC TGG GCT CCC CTG AGC AGC TGC CCC AGC CAG
Val Leu Leu Gly His Ser Leu Gly Ile Pro Trp Ala Pro Leu Ser Ser Cys Pro Ser Gln
                                  60                                         70


                350                       370                      390
GCC CTG CAG CTG GCA GGC TGC TTG AGC CAA CTC CAT AGC GGC CTT TTC CTC TAC CAG GGG
Ala Leu Gln Leu Ala Gly Cys Leu Ser Gln Leu His Ser Gly Leu Phe Leu Tyr Gln Gly
                                  80                                         90
```

(Fig. 3(A)-2)

```
                    410                              430                              450
CTC CTG CAG GCC CTG GAA GGG ATC TCC CCC GAG TTG GGT CCC ACC TTG GAC ACA CTG CAG
Leu Leu Gln Ala Leu Glu Gly Ile Ser Pro Glu Leu Gly Pro Thr Leu Asp Thr Leu Gln
                                        100                                          110


                    470                              490                              510
CTG GAC GTC GCC GAC TTT GCC ACC ACC ATC TGG CAG CAG ATG GAA GAA CTG GGA ATG GCC
Leu Asp Val Ala Asp Phe Ala Thr Thr Ile Trp Gln Gln Met Glu Glu Leu Gly Met Ala
                                        120                                          130


                    530                              550                              570
CCT GCC CTG CAG CCC ACC CAG GGT GCC ATG CCG GCC TTC GCC TCT GCT TTC CAG CGC CGG
Pro Ala Leu Gln Pro Thr Gln Gly Ala Met Pro Ala Phe Ala Ser Ala Phe Gln Arg Arg
                                        140                                          150


                    590                              610                              630
GCA GGA GGG GTC CTG GTT GCC TCC CAT CTG CAG AGC TTC CTG GAG GTG TCG TAC CGC GTT
Ala Gly Gly Val Leu Val Ala Ser His Leu Gln Ser Phe Leu Glu Val Ser Tyr Arg Val
                                        160                                          170


                    650                              670                              690
CTA CGC CAC CTT GCC CAG CCC TGA GCCAAGCCCTCCCCATCCCATGTATTTATCTCTATTTAATATTTATG
Leu Arg His Leu Ala Gln Pro End


        710                 730                 750                 770
    TCTATTTAAGCCTCATATTTAAAGACAGGGAAGAGCAGAACGGAGCCCCAGGCCTCTGTGTCCTTCCCTGCATTTCTG


        790                 810                 830                 850
    AGTTTCATTCTCCTGCCTGTAGCAGTGAGAAAAAGCTCCTGTCCTCCCATCCCCTGGACTGGGAGGTAGATAGGTAAAT


        870                 890                 910                 930
    ACCAAGTATTTATTACTATGACTGCTCCCCAGCCCTGGCTCTGCAATGGGCACTGGGATGAGCCGCTGTGAGCCCCTGG
```

(Fig. 3(A)-3)

TCCTGAGGGTCCCCACCTGGGACCCTTGAGAGTATCAGGTCTCCCACGTGGGAGACAAGAAATCCCTGTTTAATATTTA

AACAGCAGTGTTCCCCATCTGGGTCCTTGCACCCCTCACTCTGGCCTCAGCCGACTGCACAGCGGCCCCTGCATCCCCTT

GGCTGTGAGGCCCCTGGACAAGCAGAGGTGGCCAGAGCTGGGAGGCATGGCCCTGGGGTCCCACGAATTTGCTGGGGAA

TCTCGTTTTTCTTCTTAAGACTTTTGGGACATGGTTTGACTCCCGAACATCACCGACGCGTCTCCTGTTTTTCTGGGTG

GCCTCGGGACACCTGCCCTGCCCCCACGAGGGTCAGGACTGTGACTCTTTTTAGGGCCAGGCAGGTGCCTGGACATTTG

CCTTGCTGGACGGGGACTGGGGATGTGGGAGGGAGCAGACAGGAGGAATCATGTCAGGCCTGTGTGTGAAAGGAAGCTC

CACTGTCACCCTCCACCTCTTCACCCCCCACTCACCAGTGTCCCCTCCACTGTCACATTGTAACTGAACTTCAGGATA

ATAAAGTGTTTGCCTCCAAAAAAAAAAAAAAAAAAAAAAAA

EP 0 215 126 B1

(Fig. 3(B))

```
Met-Ala-Gly-Pro-Ala-Thr-Gln-Ser-
Pro-Met-Lys-Leu-Met-Ala-Leu-Gln-
Leu-Leu-Leu-Trp-His-Ser-Ala-Leu-
Trp-Thr-Val-Gln-Glu-Ala-Thr-Pro-
Leu-Gly-Pro-Ala-Ser-Ser-Leu-Pro-
Gln-Ser-Phe-Leu-Leu-Lys-Cys-Leu-
Glu-Gln-Val-Arg-Lys-Ile-Gln-Gly-
Asp-Gly-Ala-Ala-Leu-Gln-Glu-Lys-
Leu-Val-Ser-Glu-Cys-Ala-Thr-Tyr-
Lys-Leu-Cys-His-Pro-Glu-Glu-Leu-
Val-Leu-Leu-Gly-His-Ser-Leu-Gly-
Ile-Pro-Trp-Ala-Pro-Leu-Ser-Ser-
Cys-Pro-Ser-Gln-Ala-Leu-Gln-Leu-
Ala-Gly-Cys-Leu-Ser-Gln-Leu-His-
Ser-Gly-Leu-Phe-Leu-Tyr-Gln-Gly-
Leu-Leu-Gln-Ala-Leu-Glu-Gly-Ile-
Ser-Pro-Glu-Leu-Gly-Pro-Thr-Leu-
Asp-Thr-Leu-Gln-Leu-Asp-Val-Ala-
Asp-Phe-Ala-Thr-Thr-Ile-Trp-Gln-
Gln-Met-Glu-Glu-Leu-Gly-Met-Ala-
Pro-Ala-Leu-Gln-Pro-Thr-Gln-Gly-
Ala-Met-Pro-Ala-Phe-Ala-Ser-Ala-
Phe-Gln-Arg-Arg-Ala-Gly-Gly-Val-
Leu-Val-Ala-Ser-His-Leu-Gln-Ser-
Phe-Leu-Glu-Val-Ser-Tyr-Arg-Val-
Leu-Arg-His-Leu-Ala-Gln-Pro
```

( I )

(Fig. 3(B))

Thr-Pro-Leu-Gly-Pro-Ala-Ser-Ser-
Leu-Pro-Gln-Ser-Phe-Leu-Leu-Lys-
Cys-Leu-Glu-Gln-Val-Arg-Lys-Ile-
Gln-Gly-Asp-Gly-Ala-Ala-Leu-Gln-
Glu-Lys-Leu-Val-Ser-Glu-Cys-Ala-
Thr-Tyr-Lys-Leu-Cys-His-Pro-Glu-
Glu-Leu-Val-Leu-Leu-Gly-His-Ser-
Leu-Gly-Ile-Pro-Trp-Ala-Pro-Leu-
Ser-Ser-Cys-Pro-Ser-Gln-Ala-Leu-
Gln-Leu-Ala-Gly-Cys-Leu-Ser-Gln-
Leu-His-Ser-Gly-Leu-Phe-Leu-Tyr-
Gln-Gly-Leu-Leu-Gln-Ala-Leu-Glu-
Gly-Ile-Ser-Pro-Glu-Leu-Gly-Pro-
Thr-Leu-Asp-Thr-Leu-Gln-Leu-Asp-
Val-Ala-Asp-Phe-Ala-Thr-Thr-Ile-
Trp-Gln-Gln-Met-Glu-Glu-Leu-Gly-
Met-Ala-Pro-Ala-Leu-Gln-Pro-Thr-
Gln-Gly-Ala-Met-Pro-Ala-Phe-Ala-
Ser-Ala-Phe-Gln-Arg-Arg-Ala-Gly-
Gly-Val-Leu-Val-Ala-Ser-His-Leu-
Gln-Ser-Phe-Leu-Glu-Val-Ser-Tyr-
Arg-Val-Leu-Arg-His-Leu-Ala-Gln-
Pro

( II )

(Fig. 4(A)-1)

```
                                 10                           30
                    GGAGCCTGCAGCCCAGCCCCACCCAGACCC
```

```
                    50                        70                      90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                      -20
```

```
                    110                       130                     150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
 !0                                       -1  1                          10
```

```
                    170                       190                     210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                              20                                        30
```

```
                    230                       250                     270
CTC CAG GAG AAG CTG TGT GCC ACC TAC AAG CTG TGC CAC CCC GAG GAG CTG GTG CTG CTC
Leu Gln Glu Lys Leu Cys Ala Thr Tyr Lys Leu Cys His Pro Glu Glu Leu Val Leu Leu
                              40                                        50
```

```
                    290                       310                     330
GGA CAC TCT CTG GGC ATC CCC TGG GCT CCC CTG AGC AGC TGC CCC AGC CAG GCC CTG CAG
Gly His Ser Leu Gly Ile Pro Trp Ala Pro Leu Ser Ser Cys Pro Ser Gln Ala Leu Gln
                              60                                        70
```

```
                    350                       370                     390
CTG GCA GGC TGC TTG AGC CAA CTC CAT AGC GGC CTT TTC CTC TAC CAG GGG CTC CTG CAG
Leu Ala Gly Cys Leu Ser Gln Leu His Ser Gly Leu Phe Leu Tyr Gln Gly Leu Leu Gln
                              80                                        90
```

EP 0 215 126 B1

(Fig. 4(A)-2)

GCC CTG GAA GGG ATC TCC CCC GAG TTG GGT CCC ACC TTG GAC ACA CTG CAG CTG GAC GTC
Ala Leu Glu Gly Ile Ser Pro Glu Leu Gly Pro Thr Leu Asp Thr Leu Gln Leu Asp Val

GCC GAC TTT GCC ACC ACC ATC TGG CAG CAG ATG GAA GAA CTG GGA ATG GCC CCT GCC CTG
Ala Asp Phe Ala Thr Thr Ile Trp Gln Gln Met Glu Glu Leu Gly Met Ala Pro Ala Leu

CAG CCC ACC CAG GGT GCC ATG CCG GCC TTC GCC TCT GCT TTC CAG CGC CGG GCA GGA GGG
Gln Pro Thr Gln Gly Ala Met Pro Ala Phe Ala Ser Ala Phe Gln Arg Arg Ala Gly Gly

GTC CTA GTT GCC TCC CAT CTG CAG AGC TTC CTG GAG GTG TCG TAC CGC GTT CTA CGC CAC
Val Leu Val Ala Ser His Leu Gln Ser Phe Leu Glu Val Ser Tyr Arg Val Leu Arg His

CTT GCC CAG CCC TGA GCCAAGCCCTCCCCATCCCATGTATTTATCTCTATTTAATATTTATGTCTATTTAAGCC
Leu Ala Gln Pro End

TCATATTTAAAGACAGGGAAGAGCAGAACGGAGCCCCAGGCCTCTGTGTCCTTCCCTGCATTTCTGAGTTTCATTCTCC

TGCCTGTAGCAGTGAGAAAAAGCTCCTGTCCTCCCATCCCCTGGACTGGGAGGTAGATAGGTAAATACCAAGTATTTAT

(Fig. 4(A)-3)

870          890               910                 930
TACTATGACTGCTCCCCAGCCCTGGCTCTGCAATGGGCACTGGGATGAGCCGCTGTGAGCCCCTGGTCCTGAGGGTCCC

950            970                990              1010
CACCTGGGACCCTTGAGAGTATCAGGTCTCCCACGTGGGAGACAAGAAATCCCTGTTTAATATTTAAACAGCAGTGTTC

1030           1050             1070             1090
CCCATCTGGGTCCTTGCACCCCTCACTCTGGCCTCAGCCGACTGCACAGCGGCCCCTGCATCCCCTTGGCTGTGAGGCC

1110            1130              1150              1170
CCTGGACAAGCAGAGGTGGCCAGAGCTGGGAGGCATGGCCCTGGGGTCCCACGAATTTGCTGGGGAATCTCGTTTTTCT

1190             1210              1230              1250
TCTTAAGACTTTTGGGACATGGTTTGACTCCCGAACATCACCGACGCGTCTCCTGTTTTTCTGGGTGGCCTCGGGACA

1270              1290              1310              1330
CCTGCCCTGCCCCCACGAGGGTCAGGACTGTGACTCTTTTTAGGGCCAGGCAGGTGCCTGGACATTTGCCTTGCTGGAC

1350              1370              1390              1410
GGGGACTGGGGATGTGGGAGGGAGCAGACAGGAGGAATCATGTCAGGCCTGTGTGTGAAAGGAAGCTCCACTGTCACCC

1430              1450              1470              1490
TCCACCTCTTCACCCCCCACTCACCAGTGTCCCCTCCACTGTCACATTGTAACTGAACTTCAGGATAATAAAGTGCTTG

1510
CCTCCAAAAAAAAAAAAAAAAAAAAAAAAAA

EP 0 215 126 B1

(Fig. 4(B))

Met-Ala-Gly-Pro-Ala-Thr-Gln-Ser-
Pro-Met-Lys-Leu-Met-Ala-Leu-Gln-
Leu-Leu-Leu-Trp-His-Ser-Ala-Leu-
Trp-Thr-Val-Gln-Glu-Ala-Thr-Pro-
Leu-Gly-Pro-Ala-Ser-Ser-Leu-Pro-
Gln-Ser-Phe-Leu-Leu-Lys-Cys-Leu-
Glu-Gln-Val-Arg-Lys-Ile-Gln-Gly-
Asp-Gly-Ala-Ala-Leu-Gln-Glu-Lys-
Leu-Cys-Ala-Thr-Tyr-Lys-Leu-Cys-
His-Pro-Glu-Glu-Leu-Val-Leu-Leu-
Gly-His-Ser-Leu-Gly-Ile-Pro-Trp-
Ala-Pro-Leu-Ser-Ser-Cys-Pro-Ser-
Gln-Ala-Leu-Gln-Leu-Ala-Gly-Cys-
Leu-Ser-Gln-Leu-His-Ser-Gly-Leu-
Phe-Leu-Tyr-Gln-Gly-Leu-Leu-Gln-
Ala-Leu-Glu-Gly-Ile-Ser-Pro-Glu-
Leu-Gly-Pro-Thr-Leu-Asp-Thr-Leu-
Gln-Leu-Asp-Val-Ala-Asp-Phe-Ala-
Thr-Thr-Ile-Trp-Gln-Gln-Met-Glu-
Glu-Leu-Gly-Met-Ala-Pro-Ala-Leu-
Gln-Pro-Thr-Gln-Gly-Ala-Met-Pro-
Ala-Phe-Ala-Ser-Ala-Phe-Gln-Arg-
Arg-Ala-Gly-Gly-Val-Leu-Val-Ala-
Ser-His-Leu-Gln-Ser-Phe-Leu-Glu-
Val-Ser-Tyr-Arg-Val-Leu-Arg-His-
Leu-Ala-Gln-Pro

( I )

EP 0 215 126 B1

(Fig. 4(B))

```
Thr-Pro-Leu-Gly-Pro-Ala-Ser-Ser-
Leu-Pro-Gln-Ser-Phe-Leu-Leu-Lys-
Cys-Leu-Glu-Gln-Val-Arg-Lys-Ile-
Gln-Gly-Asp-Gly-Ala-Ala-Leu-Gln-
Glu-Lys-Leu-Cys-Ala-Thr-Tyr-Lys-
Leu-Cys-His-Pro-Glu-Glu-Leu-Val-
Leu-Leu-Gly-His-Ser-Leu-Gly-Ile-
Pro-Trp-Ala-Pro-Leu-Ser-Ser-Cys-
Pro-Ser-Gln-Ala-Leu-Gln-Leu-Ala-
Gly-Cys-Leu-Ser-Gln-Leu-His-Ser-
Gly-Leu-Phe-Leu-Tyr-Gln-Gly-Leu-
Leu-Gln-Ala-Leu-Glu-Gly-Ile-Ser-
Pro-Glu-Leu-Gly-Pro-Thr-Leu-Asp-
Thr-Leu-Gln-Leu-Asp-Val-Ala-Asp-
Phe-Ala-Thr-Thr-Ile-Trp-Gln-Gln-
Met-Glu-Glu-Leu-Gly-Met-Ala-Pro-
Ala-Leu-Gln-Pro-Thr-Gln-Gly-Ala-
Met-Pro-Ala-Phe-Ala-Ser-Ala-Phe-
Gln-Arg-Arg-Ala-Gly-Gly-Val-Leu-
Val-Ala-Ser-His-Leu-Gln-Ser-Phe-
Leu-Glu-Val-Ser-Tyr-Arg-Val-Leu-
Arg-His-Leu-Ala-Gln-Pro
```

( II )

77

EP 0 215 126 B1

(Fig. 5)

(Fig. 6)

(synthetic Linker)

```
CGAATGACCCCCCTGGGCC
GCTTACTGGGGGGAC
```

EcoRI
Ptac
pKK 223-3

EcoRI  ApaI
Amp$^r$
pBRG4
CSFc DNA
DraI
DraI
EcoRI

(digest with EcoRI)
(treat with BAP)
(treat with Klenow)

(digest with ApaI-DraI)

CSF
EcoRI
Ptac
ptac G4

(Fig. 7(A))

(Fig. 7)

(Fig. 7(A))

(Fig. 7(B))

EcoRI

Amp$^r$

BamHI
MboⅡ

MboⅡ
ClaI

BamHI
ClaI

Ori

pPL-λ(lambda)

(digest with BamHI)
(1% agarose gel)

EcoRI        BamHI

Amp$^r$

BamHI

(BAP)

Amp$^r$

BamHI
OH

OH
BamHI

ClaI  ClaI

| O.P.L. | N | t.L1 |

BamHI   MboⅡ   MboⅡ   BamHI

~200bp   ~690 bp   ~310bp

MboⅡ
4% PAGE

(alkali
phosphatase)

| O.P.L. |

BamHI   MboⅡ

ClaI

| t.L1 |

MboⅡ   BamHI

synthetic S/D linker

S/D

MboⅡ

TAAGGAGAATTCATCGAT
GATTCCTCTTAAGTAGCT

MboⅡ                EcoRI   ClaI

(Fig. 7(B))

synthetic linker

```
CGAATGACCCCCCTGGGCC
GCTTACTGGGGGGAC
```

digest with ApaI-DraI

EP 0 215 126 B1

(Fig. 8)

EcoRI

ClaI

Trp

pOY-1

PvuII

(synthetic linker)

CGCGAATGACCCCCCTGGGCC
GCTTACTGGGGGGAC

EcoRI  ApaI

CSFcDNA

Amp

pBRG4

DraI

DraI

EcoRI

(digest with ClaI-PvuII)

(digest with ApaI-DraI)

(T4 DNA ligase)

CSF

Trp

ptrpG4

EP 0 215 126 B1

## (Fig. 9)

EP 0 215 126 B1

(Fig. 10(A))

(Fig. 10)

(Fig. 10(A))

(Fig. 10(B))

pPL-λ (lambda)

(digest with BamHI)
(1% agarose gel)

(synthetic S/D linker)

~200bp  ~690bp  ~310bp

MboII
4% PAGE

(alkali phosphatase)

(BAP)

(Fig. 10(B))

EcoRI

BamHI

tL₁

ClaI

ClaI

MboⅡ

EcoRI

(S/D linker)

MboⅡ

PₗOₗ

BamHI

Ampʳ

EcoRI

ApaI

CSFcDNA

AmP

DraI

DraI

pBRV2

EcoRI

(synthetic linker)

CGAATGACCCCCCTGGGCC
GCTTACTGGGGGGAC

ApaI-DraI

EcoRI

BamHI

tL₁

ClaI

EcoRI

(S/D linker)

MboⅡ

PₗOₗ

BamHI

Ampʳ

(digest with ClaI)
(Sl nuclease)

(ClaI)

CSF

PₗOₗ

(ClaI)

tL₁

pPₗV2

Ampʳ

EP 0 215 126 B1

85

(Fig. 11)

synthetic linker

CGCGAATGACCCCCCTGGGCC
GCTTACTGGGGGGAC

pOY-1

EcoRI  ClaI

Trp

PvuII

(digest with CalI-PvuII)

pBRV2

EcoRI  ApaI

AmP

CSFc DNA

DraI
DraI

EcoRI

(digest with ApaI-DraI)

(T4 DNA legase)

ptrpV2

CSF  Trp

EP 0 215 126 B1

(Fig. 12)

(Fig. 13)

(Fig. 14a)

(Fig. 14b)

(Fig. 15)

(Fig. 16)

(Fig. 17a)

(Fig. 17b)